# EUROPÄISCHE PATENTSCHRIFT

(11) **EP 1 006 196 B1**
(45) Veröffentlichungstag und Bekanntmachung des Hinweises auf die Patenterteilung: **13.04.2005**
(21) Anmeldenummer: 99250415.9
(22) Anmeldetag: 25.11.1999
(51) Int. Cl.: C12N 15/867, C12N 5/10, C12N 7/01, A61K 48/00, C07K 14/08, C12N 15/40

(54) **Retrovirale, mit LCMV-Glykoprotein pseudotypisierte Hybrid-Vektoren**
Retroviral hybrid vectors pseudotyped with LCMV glycoprotein
Vecteurs hybrides retroviraux pseudotypés avec une glycoprotéine de LCMV

(30) Priorität: 26.11.1998 DE 19856463
(43) Veröffentlichungstag der Anmeldung: 07.06.2000
(73) Patentinhaber: Heinrich-Pette-Institut, 20251 Hamburg (DE)
(72) Erfinder: Laer, Meike-Dorothee von, 60596 Frankfurt am Main (DE); Beyer Winfried, 21035 Hamburg (DE)
(74) Vertreter: UEXKÜLL & STOLBERG

(56) Entgegenhaltungen:
- TENG M. N. ET AL.: "A single amino acid change in the glycoprotein of lymphocytic choriomenengitis virus is associated with the ability to cause growth hormone deficiency syndrome." JOURNAL OF VIROLOGY, Bd. 70, Nr. 12, Dezember 1996 (1996-12), Seiten 8438-8443, XP002135672
- VILLARETE L. ET AL.: " Tissue-mediated selection of viral variants: Correlation between glycoprotein mutation and growth in neuronal cells." JOURNAL OF VIROLOGY, Bd. 68, Nr. 11, 1994, Seiten 7490-7496, XP002135673
- MILLER N. ET AL.: "TARGETED VECTORS FOR GENE THERAPY" FASEB JOURNAL,US,FED. OF AMERICAN SOC. FOR EXPERIMENTAL BIOLOGY, BETHESDA, MD, Bd. 9, Nr. 2, 1. Februar 1995 (1995-02-01), Seiten 190-199, XP000616414 ISSN: 0892-6638
- BRUNS M. ET AL.: "Lymphocytic choriomeningitis virus." VIROLOGY, Bd. 137, 1984, Seiten 49-57, XP000891881
- MILETIC H. ET AL.: "Retroviral vectors pseudotyped with lymphocytic choriomenengitis virus." JOURNAL OF VIROLOGY, Bd. 73, Nr. 7, Juli 1999 (1999-07), Seiten 6114-6116, XP002135674

## Beschreibung

Die vorliegende Erfindung betrifft allgemein die Pseudotypisierung von Retroviren mit lymphozytärem Choriomeningitisvirus (LCMV). Insbesondere betrifft die Erfindung die Pseudotypisierung in MLV-Verpackungszellen, die gegebenenfalls *env*-deletiert sind, oder in Verpackungszellen, die von Lentiviren abgeleitet sind. vorzugsweise erfolgt die Pseudotypisierung durch Infektion mit LCMV oder durch Transfektion mit einem das gp-Gen des LCMV oder einen Teil desselben und gegebenenfalls zusätzlich das *np*-, das *l*- und/oder das *z*-Gen des LCMV enthaltenden Expressionsplasmid. Die Erfindung betrifft ferner die Verwendung derartiger Pseudotypen zur Infektion von Zellen, insbesondere die Verwendung in der Gentherapie.

Retrovirale Vektoren finden im Stand der Technik zunehmend Verwendung, beispielsweise für den Gentransfer in der gentechnischen bzw. medizinischen Forschung oder bei gentherapeutischen Ansätzen (vgl. z.B. C. Baum et al. in *Seminars in Oncology: Gene Therapy of Cancer: Translational approaches from preclinical studies to clinical implementations*., eds. Gerson, & Lattime, Academic Press, 1998). Die retroviralen Vektoren sind meist von murinen Leukämieviren (MLV) abgeleitet und enthalten alle für die Integration notwendigen Sequenzen der LTR-Regionen und das für die Verpackung verantwortliche ψ-Element. Die für die Virusproteine codierenden Bereiche sind durch Fremdgene und Kontrollsequenzen ersetzt, die man in menschliche Zellen einbringen möchte. Die Vektoren werden in sogenannten Helferzellinien (Verpackungszellinien) hergestellt, die eine Kopie eines kompletten Retrovirusgenoms enthalten. Es synthetisiert alle für die Replikation und Infektion notwendigen Proteine, kann jedoch seine genomische Virus-RNA nicht in Partikel verpacken, da es einen Defekt in den ψ-Sequenzen aufweist. Werden die retroviralen Vektoren in diese Helferzellen eingebracht und transkribiert, kann die gebildete transgene mRNA durch die ihr eigene ψ-Region mit den Strukturproteinen des Helfervirus interagieren und zu Partikeln verpackt werden. Die rekombinanten virionen, die keinerlei Erbinformation für Viruskomponenten besitzen, adsorbieren über ihre Oberflächenproteine an Zellen, die Capside werden in das Cytoplasma aufgenommen, und die transgene RNA wird in doppelsträngige DNA überschrieben und in das Wirtszellgenom integriert. Der Vorteil dieses Systems ist die stabile Integration der Fremdgene, die bei Teilungen auf die Tochterzellen weitergegeben werden. Nachteilig ist die Retroviren eigene unspezifische Integration an willkürlichen Stellen des Zellgenoms.

Retrovirale Vektoren vermitteln eine stabile, kolineare Integration (d.h. ohne Rekombinationen und Rearrangierung der kodierenden sequenzen im Vektorgenom) und dadurch eine langfristige Expression des Transgens. Eine langfristige Genexpression ist sonst bislang nur noch durch die episomalen Herpesvirusvektoren oder die Adeno-associated virus-Vektoren (AAV-Vektoren) möglich. Für die letztgenannten Vektorsysteme sind die Verpackungssysteme (Verpackungszellinien) jedoch noch nicht optimiert. AAV-Vektoren weisen ferner eine geringe Verpackungskapazität (ca. 5 kb für AAV gegenüber ca. 10-12 kb für retrovirale Vektoren) auf.

In Verpackungslinien wird das Vektor-Genom, das retrovirale cis-Elemente enthält, durch Transkription gebildet. Dieses genomische Vektortranskript kodiert für das zu transferierende Gen, aber nicht für retrovirale Proteine. Es wird jedoch in den Verpackungslinien mit Hilfe der *gag-, pol*- und *env*-Genprodukte in ein infektiöses, aber nicht replikationsfähiges Virion eingebaut. Dieses Virion kann dann als retroviraler Vektor zum Transfer des in das Vektorgenom integrierten Transgens in die gewünschten Zielzellen eingesetzt werden, ohne daß es dort zur weiteren Vermehrung des Vektors kommt. Mit anderen Worten, der virale Vektor kann die Zielzellen infizieren, sich dort aber nicht weiter vermehren.

Die Entwicklung retroviraler Verpackungssysteme ist bereits weit fortgeschritten, und Vektorüberstände, die frei von replikationskompetenten Viren sind, können in großen Mengen unter GMP-Bedingungen (Good manufacturing practice; Richtlinie der Kommission zur Festlegung der Grundsätze und Leitlinien der guten Herstellungspraxis (GMP) für bestimmte Arzneimittel zur Anwendung beim Menschen (91/356/EWG) vom 13-6.91) hergestellt werden. Vektoren auf Basis des murinen Leukämievirus (MLV-Vektoren) wurden bereits mehrfach in klinischen Studien eingesetzt (P. Chu et al., J. Mol. Med. 76 (1998) 184-192).

Im Stand der Technik sind zwei grundsätzliche Typen retroviraler Verpackungssysteme bekannt (J.M. Wilson, Clin. Exp. Immunol 107 Suppl. 1 (1997) 31-32; C. Baum et al. (1998), loc. cit.).

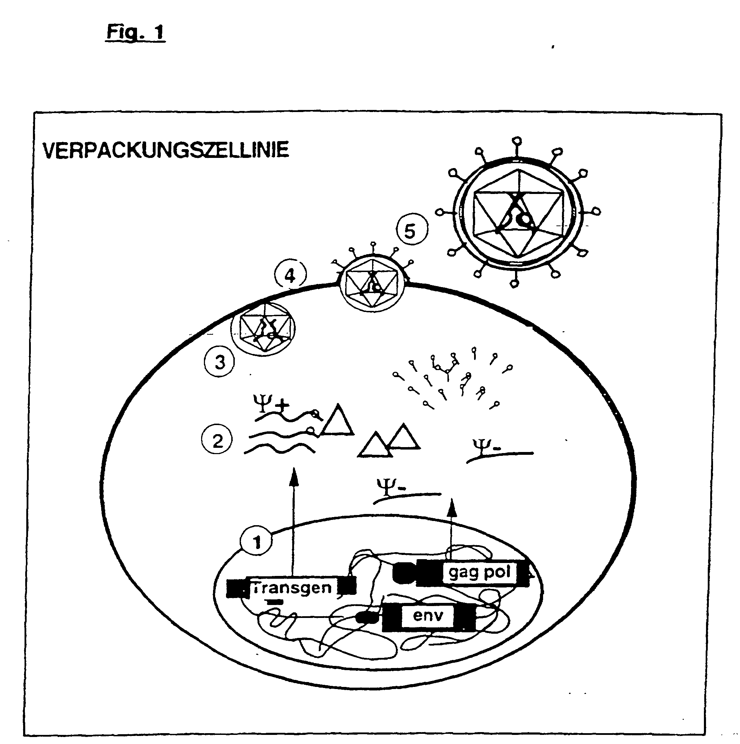

MLV-Verpackungszellinien enthalten die retroviralen Gene gag, pol und env (Abb. 1), und die für die Verpackung der retroviralen RNA erforderliche Sequenzen sind deletiert (C. Baum et al. (1998), loc. cit.).

Der zweite Typ der bekannten Verpackungssysteme leitet sich vor den Lentiviren ab (R. Carroll et al., J. Virol. 68 (1994) 6047-6051; P. Corbeau et al., Proc. Natl. Acad. Sci. USA 93 (1996) 14070-14075; L. Naldini et al., Science 272 (1996) 263-267; C. Parolin et al., J. Virol. 68 (1994) 3888-3895; J. Reiser et al., Proc. Natl. Acad. Sci. USA 93 (1996) 15266-15271; J.H. Richardson et al., J. Gen. Virol. 76 (1995) 691-696; T. Shimada et al., J. Clin. Invest. 88 (1991) 1043-1047). Lentiviren sind komplexe Retroviren, die zusätzlich zu den *gag-, pol-* und *env*-Genprodukten noch eine Reihe regulatorischer Gene exprimieren. Beispiele für Lentiviren, aus denen Verpackungssysteme abgeleitet wurden, sind das "Humane Immundefizienzvirus" (HIV), das "Simian Immundefizienzvirus" (SIV) und das "Feline Immundefizienzvirus" (FIV). Der Aufbau der lentiviralen Verpackungssysteme ähnelt prinzipiell demjenigen der MLV-Vektoren.

vorteil der lentiviralen Vektoren ist, daß sie auch ruhende zellen infizieren können. Bei MLV-Vektoren hingegen kann das Vektorgenom nur während der Zellteilung in den Zellkern transportiert werden, d.h. wenn die Kernmembran aufgelöst ist. Allerdings weisen von Lentiviren abgeleitete Verpackungssysteme aufgrund des komplexen Aufbaus des lentiviralen Genoms Nachteile auf, die sich in einem vergleichsweise geringen Titer und einer geringeren Sicherheit äußern. Durch den komplexen Genomaufbau lassen sich cis- und trans-Elemente im Genom nicht klar voneinander trennen. In den Verpackungskonstrukten, die lentivirale *gag-, pol-* und *env*-Gene exprimieren, befinden sich daher auch wichtige cis-regulatorische Sequenzen (z.B. Teile des Verpackungs-Signals), die auch im Vektor-Genom enthalten sein müssen. Durch diese Homologien kann es zu Rekombinationen zwischen Vektorgenom und den Verpackungskonstrukten und damit zur Freisetzung replikationskompetenter Retroviren (z.B. einem HIV-Wildvirus, was hochgradig unerwünscht wäre) kommen, so daß diese Systeme von der Sicherheit her nicht mit MLV-Verpackungslinien vergleichbar sind.

Alle bislang im Stand der Technik bekannten Vektorsysteme weisen ferner einige entscheidende Mängel auf, die einen erfolgreichen Einsatz in der Gentherapie verhindern: 1. Retrovirale Vektoren werden meist nur in unzureichenden Titern produziert und können durch die Instabilität ihrer Hüllproteine nicht weiter aufkonzentriert werden. 2. Vektorpartikel können aufgrund der Instabilität ihrer Hüllproteine nicht ohne Verlust der Infektiosität aufgereinigt werden. Eine solche Aufreinigung ist aber essentiell, da die Zellkulturüberstände, aus denen Vektoren geerntet werden, durch zelluläre Bestandteile verunreinigt sind. 3. Durch ihre Hüllproteine werden retrovirale Vektoren von humanem Serum-Komplement inaktiviert. 4. Der Rezeptor für das Hüllprotein der klassischen, amphotropen Vektoren wird auf fast allen daraufhin untersuchten Zellinien exprimiert. Allerdings weisen viele primäre humane Zellen, wie Hepatozyten und hämatopoetische Stammzellen, die attraktive Ziele der Gentherapie sind, einen Mangel an funktionellen amphotropen Rezeptoren auf, wodurch eine Transduktion erschwert oder verhindert wird.

Aufgabe der vorliegenden Erfindung ist es daher, retrovirale Verpackungssysteme zur Verfügung zustellen, die die Nachteile der im Stand der Technik bekannten Verpackungszellinien nicht aufweisen.

Insbesondere ist es Aufgabe der vorliegenden Erfindung, weitere Verpackungssysteme zur Verfügung zu stellen, die einen stabilen, retroviralen Transfer von Transgenen in die Zielzellen ermöglichen, d.h., zu einer stabilen Integration des Transgens in das Genom der Ziel- oder Wirtszellen und einer anschließenden stabilen Expression dieses Gens führen.
Die Aufgabe wird erfindungsgemäß dadurch gelöst, daß man Retroviren mit Lymphozytärem Choriomeningitisvirus (LCMV) pseudotypisiert.

Die vorliegende Erfindung betrifft daher ein rekombinantes virion, das vorzugsweise den Gentransfer von einem oder mehreren Fremdgenen vermittelt und das durch Pseudotypisierung mit LCMV entstanden ist.

Der Tropismus und auch die Stabilität eines Virus wird in erster Linie vom Hüllprotein bestimmt. Murine Retroviren können, neben den MLV-env kodierten Glykoproteinen, auch Hüllproteine anderer Virusarten in ihre Virushülle einbauen. Da-durch entstehen sogenannte Pseudotypen. Retrovirale Pseudotypvektoren entstehen durch Expression fremder viraler Hüllproteine in MLV-Verpackungslinien. Herkömmliche MLV-Verpackungszellinien enthalten die retroviralen Gene gag, pol und env. Sequenzen, die für die Verpackung retroviraler genomischer RNA notwendig sind, wurden deletiert. In solche Verpackungslinien wird ein Vektor eingebracht, der neben dem Gen, das transferiert werden soll, auch die retrovirale Verpackungssequenz und weitere retrovirale cis Elemente enthält (LTR, leader). Das retrovirale RNA-Genom wird mit Hilfe der Gag-, Pol- und Env-Genprodukte in ein infektiöses, aber nicht replikationsfähiges Virion eingebaut. Dieses virion kann dann als retroviraler Vektor zur Transduktion von Zellen eingesetzt werden. Pseudotyp-Verpackungslinien enthalten zusätzlich das Hüllproteingen eines fremden Virus. Die erfindungsgemäßen Pseudotyp-Verpackungslinien enthalten das Hüllproteingen des LCMV, und es erfolgt eine Expression der LCMV-Glykoproteine.

Mit der vorliegenden Erfindung werden erstmals Vektorsysteme zur verfügung gestellt, die in hohen Titern produziert werden und aufkonzentriert werden können. Die erfindungsgemäßen vektorpartikel lassen sich ferner ohne oder ohne wesentlichen Verlust der Infektiosität aufreinigen. Es hat sich im Rahmen der vorliegenden Erfindung überraschenderweise gezeigt, daß die erfindungsgemäße Pseudotypisierung für die Verpackungszellen nicht zelltoxisch ist. Es werden somit neue, stabile Verpackungszellinien (Verpackungssysteme) zur Verfügung gestellt, die auf der Verwendung von LCMV Glykoproteinen basieren und die zu einer größeren Stabilität von retroviralen Gentransferpartikeln führen.
Die erfindungsgemäßen retroviralen Pseudotypvektoren zeichnen sich ferner durch ein breites, speziesübergreifendes Wirtszellspektrum (Zelltropismus) aus. Ein entscheidender Vorteil der vorliegenden Erfindung ist die Tatsache, daß einzelne Mutationen im Hüllprotein des LCMV zu einer Änderung des Tropismus von LCMV führen können. D.h., durch einzelne Punktmutationen in gp werden Viren, die eher Nervenzellen infizieren, zu Viren, die eher Lymphozyten infizieren oder zu solchen, die eher Monozyten infizieren.

Im Rahmen der vorliegenden Erfindung wird zur Pseudotypisierung LCMV eingesetzt. Dabei ist es möglich oder kann sogar bevorzugt sein, statt des LCMV-Wildtyps andere LCMV-Stämme einzusetzen. So können leichte variationen in der *gp*-Nukleinsäuresequenz bzw. in der Aminosäure-Sequenz des exprimierten Hüllproteins in verschiedenen Stämmen von LCMV den Zelltropismus (Wirtszellspektrum) von LCMV erheblich verändern (M. Matloubian et al., J. Virol. 67 (1993) 7340-7349; M.N. Teng, J. Virol. 70 (1996) 8438-8443; King et al., J.Virol 64 (1990) 5611-5616). solche Tropismusvarianten im Glykoprotein findet man bei keinem der bisher bekannten retroviralen vektorsysteme, und es wird erfindungsgemäß erstmals eine gezieltere Transduktion des gewünschten Zelltyps ermöglicht. Gemäß einer bevorzugten Ausführungsform der Erfindung kann es daher von Vorteil sein, Verpackungssysteme mit verschiedenen Glykoprotein-Varianten (GP-Varianten) für unterschiedliche Anwendungen anzulegen.

Im Rahmen der vorliegenden Erfindung wird vorzugsweise von den *gp*-Genen des eher neurotropen LCMV-Stammes Armstrong, L(ARM) (L. Villarete et al., J. Virol. 68 (1994) 7490-7496) (für SEQ ID NO: 4 kodierender Bereich; vgl. Anlage zum Sequenzprotokoll, ZU SEQ ID NO: 3), und des eher hämatotropen Stammes WE (V. Romanowski et al., Virus Res. 3, (1985) 101-114) (SEQ ID NO: 1) ausgegangen. Erfindungsgemäß eingeschlossen sind auch Varianten (Tropismusvarianten) dieser beiden Stämme, bei denen im gp-Genprodukt einzelne Aminosäuren ausgetauscht sind, da dadurch der Tropismus des Virus verändert werden kann.

Da LCMV ein RNA-Virus ist, das im Vermehrungszyklus keine Kernphase hat, erscheint es sehr wahrscheinlich, daß sich "kryptische Spleiß-Regionen" in der RNA Sequenz befinden. Die Entfernung (Bereinigung) solcher Regionen kann ausgenutzt werden, um eine verbesserte Expression zu erzielen. Solche "spleißbereinigte" Varianten sind daher erfindungsgemäß ebenfalls eingeschlossen.
Durch eine solche Optimierung der GP-Expression kann die Pseudotypisierung verbessert werden, wobei auf eine zusätzliche Unterstützung durch mindestens ein weiteres LCMV-Protein verzichtet werden kann.

Eine bevorzugte Variante ist das im Rahmen der vorliegenden Erfindung erstmals beschriebene LCMV Glykoprotein WB-HPI. Die kodierende Nukleinsäuresequenz gp (Open reading frame (ORF) in SEQ ID NO: 25 dargestellt) enthält gegenüber dem LCMV-Stamm WE Mutationen an den Positionen 281, 329, 385, 397, 463, 521, 543, 631, 793, 1039, 1363 und 1370 und besitzt somit gegenüber dem Glykoprotein vom LCMV-Stamm WE Aminosäureaustäusche an den Positionen 94, 110, 129, 133, 155, 174, 181, 211, 265, 347, 455 und 457. Diese GP-Variante mit der in SEQ ID NO: 26 gezeigten Aminosäuresequenz weist den Vorteil auf, daß sie auch ohne zusätzliche LCMV-Hilfsproteine stabil ist und gegenüber dem Stamm WE eine verbesserte Pseudotypisierung bewirkt.

Die Erfindung betrifft daher ferner eine Variante des Lymphozytären Chroriomeningitis Virus, die das Gen gp enthält, das für die in SEQ ID NO: 26 dargestellte Sequenz oder einen Teil derselben kodiert, wobei das *gp*-Gen vorzugsweise die in SEQ ID NO: 25 dargestellte Sequenz oder einen Teil derselben aufweist. Erfindungsgemäß wird ein Protein mit der in SEQ ID NO: 26 gezeigten Aminosäuresequenz oder ein Teil derselben exprimiert, wobei eine für dieses Protein kodierende Nukleinsäuresequenz vorzugsweise die in SEQ ID NO: 25 dargestellte Sequenz oder ein Teil derselben ist. Diese Virusvariante sowie die letztgenannten Nuklein- und Aminosäuresequenzen sind, z.B. ausgehend von der LCMV-Variante WE, durch dem Fachmann allgemein bekannte Verfahren (z.B. durch Einführung von Punktmutationen) erhältlich.

Zur Expression von LCMV Glykoprotein eignen sich allgemein Expressionsvektoren, die eine hohe stabile Genexpression in eukaryontischen Zellen ermöglichen. Die Wahl des Expressionsvektors ist für die Verpackung der retroviralen LCMV--Pseudotypen jedoch nur insoweit entscheidend, als er ein hohes und stabiles Expressionsniveau gewährleisten muß, d.h. ein Expressionsniveau, das hoch genug ist, um die Bildung von Pseudotypen zu ermöglichen, und das dauerhaft (stabil) ist, ohne daß es zum Abschalten des Promoters kommt.

Erfindungsgemäß besonders bevorzugt sind die folgenden beiden Expressionskassetten:
(CMV-Promoter)--(β-Globin-Intron-2)--(*gp*)--(SV40 poly A-Signal) und
(EF-lalpha-Promoter)--(*gp*)--(poly-A-Signal des G-CSF-Genes).
(S. Mizushima, Nucleic Acids Res. 18 (1990) 5322, T. Uetsuki, J. Biol. Chem. 264 (1989) 5791-5798).

Die Sequenzen für die Bestandteile der Expressionskassetten sind im Sequenzprotokoll dargestellt oder allgemein bekannt:
Cytomegalovirus-Promoter (CMV-Promoter):
   (M. Boshart et al., Cell 41 (1958) 521-530; F. Langle-Rouault et al., Virol. 72(7) 6181-5 (1998))
betaglobin-Intron-2:
   (Jeffreys, A.J. et al., Cell 12 (1977) 1097-1108)
SV40 poly A-Signal:
   (M. Boshart et al., Cell 41 (1958) 521-530; F. Langle-Rouault et al., Virol. 72(7) 6181-5 (1998))
EF-lalpha-Promoter:SEQ ID NO: 9
   (S. Mizushima, Nucleic Acids Res. 18 (1990) 5322, T. Uetsuki, J. Biol. Chem. 264 (1989) 5791-5798).
G-CSF poly A-Signal:
   (S. Mizushima, Nucleic Acids Res. 18 (1990) 5322, T. Uetsuki, J. Biol. Chem. 264 (1989) 5791-5798).
gp (LCMV): vgl. SEQ ID NO: 1, 3, für SEQ ID NO: 4 kodierender Bereich (siehe auch Anlage zum Sequenzprotokoll).

Im Rahmen der vorliegenden Erfindung sind somit auch die o.g. Expressionskassetten eingeschlossen, wobei Änderungen in den jeweiligen Nukleinsäuresequenzen möglich sind, solange die Funktionalität der Expressionskassetten erhalten bleibt, d.h. deren erfindungsgemäße Verwendung die Pseudotypisierung der Verpackungszellen ermöglicht und auch die Transfektion der Zielzellen und die stabile Integration der Transgene in das Wirtsgenom nicht behindert.

Ferner zeigt auch ein episomaler EBV-Expressionsvektor (Epstein-Barr-Virus; vgl. F. Langle-Rouault et al., Virol. 72(7) 6181-5 (1998)) (pCep4) der Firma Invitrogen hohe Expression und kommt daher im Rahmen der vorliegenden Brfindung bevorzugt in Betracht.
Gegenstand der vorliegenden Erfindung ist somit ferner eine Verpackungszelle, die die retroviralen Gene gag (für SEQ ID NO: 12 kodierender Bereich; vgl. Anlage zum Sequenzprotokoll, ZU SEQ ID NO: 11), *pol* (für SEQ ID NO: 13 kodierender Bereich; vgl. Anlage zum Sequenzprotokoll, ZU SEQ ID NO: 11) und gegebenenfalls das retrovirale Gen *env* (für SEQ ID NO: 14 kodierender Bereich; vgl. Anlage zum Sequenzprotokoll, ZU SEQ ID NO: 11) und/oder regulatorische retrovirale Gene (im Falle lentiviraler Verpackungssysteme, s.u., z.B. das für das lentivirale Rev-Protein kodierende Gen, das ein Spleißen der retroviralen genomischen RNA verhindert) umfaßt und ferner das für die Glykoproteine GP-1 und GP-2 des LCMV kodierende Gen gp (für SEQ ID NO: 4 kodierender Bereich; vgl. Anlage zum Sequenzprotokoll, ZU SEQ ID NO: 3) oder einen Teil desselben umfaßt. Ferner eingeschlossen sind Nukleinsäuresequenzen, die Abweichungen (Punktmutationen, Deletionen) in den Sequenzen aufweisen (Derivate), solange bei der erfindungsgemäßen Verwendung die Pseudotypisierung von retroviralen Gentransferpartikeln gewährleistet bleibt und auch die Transduktion der Zielzellen sowie die stabile Integration der Transgene in das Wirtsgenom nicht behindert wird. Im folgenden sollen diese Derivate stets mitumfaßt sein, wenn ein beliebiges Gen als solches erwähnt wird.

Im Rahmen der vorliegenden Erfindung bezeichnet "GP" oder "GP-Protein" das GP-C-Vorläuferprotein von LCMV, aus dem durch proteolytische Spaltung dann GP-1 und GP-2 entstehen, die nachfolgend auch einfach als "LCMV-Glykoprotein" bezeichnet werden.

Erfindungsgemäß werden ferner Pseudotyp-Verpackungssysteme bereitgestellt, in denen neben dem gp-Genprodukt (SEQ ID NO: 4) ein oder mehrere weitere Gene von LCMV exprimiert werden, wie zum Beispiel das für das Nukleoprotein kodierende Gen *np* (für SEQ ID NO: 5 kodierender Bereich; vgl. Anlage zum Sequenzprotokoll, ZU SEQ ID NO: 3), das für ein Protein mit unbekannter Funktion kodierenden Gen *z* (für SEQ ID NO: 8 kodierender Bereich; vgl. Anlage zum Sequenzprotokoll, ZU SEQ ID NO: 6) und das für die RNA-Polymerase kodierende Gen *l* (für SEQ ID NO: 7 kodierender Bereich; vgl. Anlage zum Sequenzprotokoll, ZU SEQ ID NO: 6). Diese Gene können gemäß einer besonderen Ausführungsform der Erfindung entweder vom WEoder Armstrong-Stamm des LCMV stammen. In diesem Zusammenhang können entweder die vollständigen Sequenzen der Gene *np, z* und/oder *l* eingesetzt werden (SEQ ID NOs: s.o.) oder Teile derselben verwendet werden. Erfindungsgemäß eingeschlossen sind Nukleinsäuresequenzen, die Abweichungen (Punktmutationen, Deletionen etc.) in den Sequenzen aufweisen (Derivate), solange die Pseudotypisierungvon retroviralen Gentransferpartikeln gewährleistet ist und auch die Transfektion der Zielzellen und die stabile Integration der Transgene in das Wirtsgenom nicht behindert wird. Im folgenden sollen diese Derivate stets mitumfaßt sein, wenn ein beliebiges Gen als solches erwähnt wird.

Gegenstand der vorliegenden Erfindung ist daher ferner eine Verpackungszelle, die zusätzlich zu dem gp-Gen des LCMV mindestens ein Gen aus der Gruppe bestehend aus dem für das Nukleoprotein kodierenden Gen np, dem für die RNA-Polymerase kodierenden Gen 1 und dem für ein Protein mit unbekannter Funktion kodierenden Gen z des LCMV enthält.

Von den erfindungsgemäßen Verpackungszellen werden die MLV/LCMV-Pseudotypen produziert, d.h. rekombinante retrovirale Virionen, die in ihrer Hülle das LCMV-Glykoprotein eingebaut enthalten.

Für die Herstellung der rekombinanten Virionen geht man im Rahmen der vorliegenden Erfindung für die viralen Verpackungszellinien vorzugsweise von allen Zellinien aus, die hohe Titer retroviraler Vektoren produzieren. Bevorzugt eingesetzte Zellinien sind NIH3T3, Te671, 293T, HT1080 (P.L. Cosset et al., J. Virol. 69 (1995) 7430-7436; D. Markowitz et al., Virology 167 (1988) 400-406; W.S.Pear et al., PNAS 90 (1993) 8392-8396). Die Wahl der Zellinie ist für die spezifischen Vorteile der Erfindung jedoch unerheblich, da es sich gezeigt hat, daß GP in keiner bislang untersuchten Zellinie toxisch wirkt. Sollten daher zukünftig Linien gefunden werden, die eine effizientere Vektorproduktion erlauben (d.h. stabiler Titer, der mindestens so hoch ist wie in den zuvor genannten Linien, > 10⁶/ml), können auch diese eingesetzt werden.

In den zur erfindungsgemäßen Pseudotypisierung vorzugsweise verwendeten Verpackungssystemen werden die *gag*- und *pol*-Gene des Moloney Stammes muriner Leukämieviren (MoMLV) exprimiert (*gag:* für SEQ ID NO: 12 kodierender Bereich; *pol:* SEQ ID NO: 11, Nukleotide 1970-5573; vgl. Anlage zum Sequenzprotokoll, ZU SEQ ID NO: 11). Erfindungsgemäß sind aber auch andere *gag-* und *pol*-varianten von MLV eingeschlossen, solange sie die o.g. Vorteile für die Vektorproduktion aufweisen. Insbesondere sind erfindungsgemäß die o.g. Gen-Derivate eingeschlossen.

Im Rahmen der vorliegenden Erfindung wurde festgestellt, daß LCMV-GP auch lentivirale Nucleocapside pseudotypisiert (s. Beispiele). Gemäß einer besonderen Ausführungsform können die Verpackungssysteme daher auch die *gag-* und *pol*-Genprodukte von Lentiviren enthalten, d.h., es können lentivirale Verpackungssysteme (Verpackungszellinien) eingesetzt werden. Dabei ist es unerheblich, von welchem Lentivirus sich das Verpackungssystem ableitet. Als lentivirale Verpackungszellen kommen im Rahmen der vorliegenden Erfindung beispielsweise Zellinien in Betracht, deren Verpackungssystemen vom Humanem Immundefizienzvirus (HIV), Affen-Immundefizienzvirus (SIV) oder Felinem Immundefizienzvirus (PIV) abgeleitet wurden. In diesem Zusammenhang könnte es für eine effiziente Produktion infektiöser Lentivirusvektoren erforderlich sein, zusätzlich akzessorische lentivirale Gene wie *rev* (für SEQ ID NO: 21 kodierender Bereich: vgl. Anlage zum Sequenzprotokoll, ZU SEQ ID NO: 15) oder *tat* (für SEQ ID NO: 20 kodierender Bereich; vgl. Anlage zum Sequenzprotokoll, ZU SEQ ID NO: 15) bei HIV-Vektoren zu exprimieren. Im Rahmen der vorliegenden Erfindung können LCMV-Proteine in allen lentiviralen Verpackungssystemen zur Pseudotypisierung eingesetzt werden.

Gegenstand der vorliegenden Erfindung ist somit ferner eine (virale) Verpackungszelle bzw. ein pseudotypisiertes Virion, bei dem das Virus aus der Familie der Retroviridiae, insbesondere den MLV-related viruses und den Lentiviren, ausgewählt ist. Gemäß einer besonderen Ausführungsform der Erfindung wird die retrovirale Verpackungszelle aus der Gruppe ausgewählt, deren Verpackungssystem sich von MLV, HIV, SIV, oder FIV ableiten.

Erfindungsgemäß wird ferner ein pseudotypisiertes Virion zur Verfügung gestellt, das von retroviralen Verpackungszellen gebildet wird, die kein ENV-Protein exprimieren, wobei zur Pseudotypisierung die Mutante L(ARM) von LCMV verwendet wird. Alternativ können auch andere Varianten von LCMV eingesetzt werden.

Gegenstand der vorliegenden Erfindung ist ferner ein Verfahren zur Herstellung der erfindungsgemäßen Verpackungszellen/Verpackungszellinien, bei dem man eine mit einem oder mehreren Fremdgenen transfizierte retrovirale Verpackungszellinie nach bekannten Methoden (vgl. z.B. Maniatis, Sambrook, Fritsch: Molecular cloning, a laboratory manual; Cold Spring Harbor Laboratory Press, 1982) mit LCMV infiziert. Die Virionen, die LCMV-Glykoprotein in ihrer Hülle eingebaut enthalten, werden von den verpackungslinien gebildet. Gemäß einer besonderen Ausführungsform der Erfindung können die Pseudotypproduzierenden Verpackungszellen hergestellt werden, indem man eine mit einem oder mehreren Fremdgenen transfizierte retrovirale Verpackungszellinie mit einem Expressionsplasmid transfiziert, das das *gp*-Gen des LCMV oder einen Teil desselben und gegebenenfalls zusätzlich ein oder mehrere Gene aus der Gruppe bestehend aus *np, l* und *z* des LCMV enthält.

Die vorliegende Erfindung betrifft somit ferner ein Verfahren zur Herstellung retroviraler Pseudotyp-Vektoren, bei dem man zunächst retrovirale Verpackungszellen herstellt, die mindestens das *gp*-Gen des LCMV oder einen Teil desselben enthalten (s.o.), und diese anschließend unter Bedingungen kultiviert, die zur Produktion von Virionen geeignet sind.

Im Rahmen der vorliegenden Erfindung verwendet man als retrovirale Verpackungszellinie vorzugsweise eine MLV-Verpackungszellinie, die kein funktionelles ENV-Protein exprimiert; und als LCMV zur Pseudotypisierung die Mutante L(ARM). Als ENV-negative Verpackungszellinie kann auch die von von Laer et al. (J. Virol. 72 (1998) 1424-1430) beschriebene Zellinie verwendet werden.

Die erfindungsgemäßen Verpackungszellinien lassen sich in vorteilhafter Weise zur Erzeugung viraler Pseudotypvektoren verwenden, die man in vorteilhafter Weise zur Transduktion von Zellinien aber auch von primären Eukaryonten-Zellen zu Forschungszwecken (in vitro) oder im Rahmen der Gentherapie einsetzen kann.

Im Rahmen der vorliegenden Erfindung kommt jedoch auch die Verwendung der Virionen/Verpackungszellen zur Gentherapie in Betracht. Die Gentherapie kann in diesem Zusammenhang die Therapie von infektiösen Erkrankungen (wie HIV-Infektionen oder AIDS) und Neoplasien (wie dem Mamma-Karzinom) oder Melanomen sowie anderen durch Gentherapie zugänglichen Erkrankungen umfassen. Im Rahmen der vorliegenden Erfindung kommt die Transduktion hämatopoetischer Stammzellen in Betracht.

Das rekombinante Virion bzw. die rekombinante Verpackungszelle (-zellinie) der vorliegenden Erfindung umfaßt ein oder mehrere Transgene. Vorzugsweise sind diese Transgene aus der Gruppe bestehend aus Markergenen, wie z.B. *neo*, *lacZ* oder enhanced green fluorescent protein (EGFP)-Gen und/oder therapeutisch einsetzbaren Genen, wie z.B. die Suizidgene Herpes Simplex Virus Thymidin Kinase (HSV-tk), Cytosin Deaminase (CD), und antiviral wirksame Sequenzen wie Ribozyme, antisense Sequenzen und transdominant-negativ wirkende Gene und in der Tumortherapie entsetzbaren Genen, wie *mdr-1* für den Schutz der hämatopoetischen Zellen bei Chemotherapie, und Zytokin-Genen, ausgewählt. Darüber hinaus können jedoch alle Transgene eingesetzt werden, die im Rahmen eines gezielten Gentransfers und der Expression des Transgens (der Transgene) in Zellen *in vitro* oder im Rahmen der Gentherapie von Interesse sein könnten.

Erfindungsgemäß eingeschlossen ist ferner ein Verfahren zur Herstellung eines pharmazeutischen Präparats zur Gentherapie, bei dem man erfindungsgemäße virale Pseudotypvektoren bzw. retrovirale Verpackungszellen gegebenenfalls mit pharmazeutisch verträglichen Hilfs- und/oder Trägerstoffen formuliert. Gegenstand der vorliegenden Erfindung ist ferner ein pharmazeutisches Präparat zur Gentherapie, das erfindungsgemäße retrovirale Verpackungszellen und gegebenenfalls pharmazeutisch verträgliche Hilfs- und/oder Trägerstoffe umfaßt.

Für eine Gentherapie *in vivo* ist oft wesentlich, daß nur bestimmte Zielzellen genetisch verändert werden und andere nicht. So soll z.B. ein Suizidgen nur in Tumorzellen und nicht in gesunde Zellen gelangen, ein Chemotherapieresistenzgen nur in gesunde Blutstammzellen und nicht in kontaminierende Tumorzellen. Für den gezielten Gentransfer in spezifische Zellen wurde bereits versucht, Liganden in die Hülle von retroviralen Vektoren oder in Liposomen einzubauen. Ein gezielter Gentransfer gelang damit aber bislang nicht, da die Liganden nur die Bindung an die Zielzelle aber keinen Eintritt des Vektorgenoms in das Zytoplasma vermitteln (F.L. Cosset et al., J. Virol. 69 (1995) 6314-6322), d.h. keine Fusion mit der Lysosomenmembran erfolgt. Dieses Problem läßt sich durch den Einsatz viraler Hüllproteine lösen, die Fusionsaktivität aufweisen, d.h., die als Fusionsprotein die Lysosomen-Membran überwinden. Die Verwendung von LCMV GP ist in diesem Zusammenhang von großem Vorteil, da bei diesem viralen Hüllprotein bei niedrigem pH-Wert, wie er im Endosom der Zielzelle vorherrscht, eine Konformationsänderung erfolgt, die - ohne daß eine Rezeptorbindung des LCMV GP erforderlich ist - zur Aktivierung der Fusionsfunktion führt. Da LCMV GP - im Gegensatz zu anderen Fusionsproteinen - keine Fusion von Zellmembranen vermittelt, ist es nicht zytotoxisch und führt nicht zur Riesenzellenbildung (vgl. C. di Simone et al-, Virology 198 (1994) 455-465).

Erfindungsgemäß eingeschlossen sind daher auch retrovirale Verpackungszellen, die die retroviralen Gene *gag, pol* und *env* und/oder regulatorische retrovirale Gene und ferner das für die Glykoproteine GP-1 und GP-2 des LCMV kodierende Gen gp oder einen Teil desselben enthält, wobei env so modifiziert ist, daß es für ein Env-Protein kodiert, das eine spezifische Bindung an die Zielzelle vermittelt (sogen. targeting-env) und wobei gp eine Variante ist, die für ein GP-Protein kodiert, das Fusionsaktivität aufweist (sogen. fusion helper). Ferner sind Verfahren zur Herstellung dieser Verpackungszellen und der von ihnen gebildeten retroviralen Pseudotypvektoren eingeschlossen. In diesem Zusammenhang kann es im Einzelfall erforderlich sein, den für die Rezeptorbindungsstelle des LCMV GP kodierenden Sequenzabschnitt zu mutieren (ohne die Fusionsaktivität zu beeinträchtigen), um eine Bindung von GP an dessen zellulären Rezeptor zu vermeiden oder zurückzudrängen. Alternativ kommt bei diesen Pseudotypvektoren die Verwendung neutralisierender Antikörper gegen GP in Betracht, die in der Lage sind, die Rezeptor-Bindung zu neutralisieren, ohne die pH-Wert-abhängige Fusion durch GP zu hemmen. Durch die im Rahmen der vorliegenden Erfindung erstmals zur Verfügung gestellten stabilen Verpackungszellinien ist es nunmehr möglich die Rezeptorbindungsstelle durch gezielte Mutagenese des GP-1 zu identifizieren. Diese Methode ist dem Fachmann ebenso wohlbekannt wie die Isolierung der erwähnten neutralisierenden Antikörper.

Mit der vorliegenden Erfindung werden erstmals Vektorsysteme zur Verfügung gestellt, die in hohen Titern produziert werden und aufkonzentriert werden können. Die erfindungsgemäßen Vektorpartikel lassen sich ferner ohne oder ohne wesentlichen Verlust der Infektiositat aufreinigen. Die erfindungsgemäßen Zellinien zeichnen sich ferner durch ein breites, speziesübergreifendes Wirtszellspektrum (Zelltropismus) aus. Es hat sich im Rahmen der vorliegenden Erfindung überraschenderweise gezeigt, daß die erfindungsgemäße Pseudotypisierung für die Verpackungszellen nicht zelltoxisch ist.

Es werden somit stabile Verpackungszellinien (Verpackungssysteme) zur Verfügung gestellt, die einen stabilen, retroviralen Transfer von Transgenen in die Zielzellen ermöglichen, d.h., zu einer stabilen Integration des Transgens in das Genom der Ziel- oder Wirtszellen und einer anschließenden stabilen Expression dieses Gens führen.

Die vorliegende Erfindung wird nachfolgend anhand von Beispielen, Figuren und einem Sequenzprotokoll erläutert.

### BEISPIELE

### Materialien und Methoden

### Zellen und viren

Die env-negative verpackungszellinie TELCeB wurde von F.-L. Cosset (F.L. Cosset et al.J Virol. 69 (1995) 7430-7436) zur Verfügung gestellt. Die env-negative Zellinie 293gp2 wurde bereits früher beschrieben (D. von Laer et al., J. Virol. 72 (1997) 1424-1430). Die Mausfibroblastenzellinie Sc-1 wurde in Minimal Essential Medium (Sigma, Deisenhofen, Deutschland), das mit 10% fetalem Kälberserum (PCS, PAN Systems, Aidenbach, Deutschland) angereichert worden war, kultiviert. Die humane Nierenzellinie 293, die humane Hepatomalinie HUH-7, die humane Fibroplastenzellinie Te671 und TELCeB und die Mausfibroblastenzellinie L-929 wurden in Dulbecco's Minimal Essential Medium (DMEM, Gibco, Paisley, Great Britain) kultiviert, das mit 10%igem FCS angereichert worden war. Die humane hämatopoetische Vorläuferzellinie TP-1 wurde in Iscove's Modified Dulbecco's Medium (Gibco, Paisley, Großbritannien) gehalten, das mit 10%igem FCS und IL-3 angereichert worden war. Konditioniertes Medium von NIH3T3-Zellen, die mit einem BPV-Vektor transfektiert worden waren, der das IL-3 Gen trägt, wurden als Quelle für IL-3 bei Konzentrationen verwendet, die für das maximale Wachstum von TF-1 notwendig sind (H. Karasuyama et al., Eur. J. Immunol. 18 (1988) 97-104). Die humane Vorläuferzellinie K562 wurde in RPMI (Gibco) gehalten, das mit 10%-igem FCS angereichert worden war.

LCMV wurde am 10.11.1998 bei der European Collection of Cell Cultures (ECACC), Salisbury, Wiltshire SP4 OJG, Großbritannien, unter der Zugriffsnummer V98111005 nach dem Budapester Vertrag hinterlegt.

Der MESV-artige retrovirale Vektor, der das Neomycin phosphotransferase Gen (MP1N) trägt, wurde früher beschrieben (H.-G. Bckert, Blood 88 (1996) 3407-3415). Der amphotrophe Helfer war ein rekombinantes replikationskompetentes Moloney-MLV, bei dem Teile des *pol-* und der größte Teil des env-Genes mit dem von dem MLV Stamm 4070A (Mo-Ampho-MP, R320 (C. Münk, Proc. Natl. Acad. Sei. U.S.A. 94 (1997) 5837-5842)] als ein SalI- bis ClaI-Fragment ersetzt wurden. Der Virus wurde in Sc-1 propagiert. Der Plaque-gereinigte WE Stamm des LCM Virus wurde in L-929 Zellen propagiert (T.M. Rivers, virology 26 (1965) 270-282).

### Durchflußzytrometische Analyse der LCMV-GP-Expression

Zur Analyse der Expression des LCMV Glykoproteins wurden 3 x 10⁵ bis 10⁶ Zellen geerntet, pelletiert und in 50µl einer 1:40 Verdünnung von Maus-Ascites resuspendiert, die einen murinen monoklonal Antikörper gegen LCMV GP-1 enthielt (M. Bruns et al., Virology 130 (1983) 247-251). Nach 20-minütiger Inkubation auf Eis wurden die Zellen dreimal mit phosphatgepufferter Kochsalzlösung (PBS) gewaschen und dann für weitere 20 Minuten in einer 1:80 Verdünnung eines FITC-markierten Ziege Anti-Maus Antikörpers inkubiert (Dako, Glostrup, Dänemark). Nach drei abschließenden Waschschritten in PBS wurden die Zellen mittels eines FACScalibur Geräts analysiert (Becton Dickinson, Heidelberg).

### Titration der Viren

Zur Bestimmung des Vektortiters wurden 5 x 10⁶Sc-l Zellen mit einer fünffachen Verdünnung der Überstände in 24-Vertiefungen Gewebekulturplatten inokuliert. Für den retroviralen Neovektor wurde die Selektion nach 24 Stunden mit 400µg G418 pro ml (Trokkengewicht GIBCO) initiiert. Das Medium wurde alle vier Tage ersetzt. Die Kolonien wurden nach zehn Tagen ausgewertet. Der Titer wurde ausgedrückt als G418 Resistenztransfereinheiten pro ml (GTU/ml). Für den retroviralen MFGnlsLacZ Vektor wurde X-Gal (5-Bromo-4-chloro-3-indolyl-β-D-galaktopyranosid) Färbung zwei Tage nach der Inokulation wie früher beschrieben (G.R. McGregor, Methods Mol. Biol. 7 (1989) 1-19) durchgeführt. Der Titer wurde ausgedrückt in LacZ Transfereinheiten (LTU) pro ml. Plaque-bildende Einheiten von LCMV wurden an L-929 Zellen wie früher beschrieben getestet (F. Lehmann-Grube, J. Gen. Virol. 37 (1977) 85-92).

MLV (LCMV) Pseudotypen wurden durch Preinkubation eines gleichen Volumens eines Virus-positiven Überstandes mit einem Anti-LCMV gp44 neutralisierendem monoklonalen Antikörper neutralisiert, der 1: 100 verdünnt wurde (M. Bruns et al., Virology 130 (1983) 247-251). Die Titer wurden dann in LTU pro ml wie beschrieben bestimmt.

### DNA Analyse

Herstellung von DNA und Southern Analyse wurden durchgeführt wie früher beschrieben (C. Stocking et al., Cell 53 (1988) 869-879). Die genomische DNA wurde mit HindIII verdaut, das einmal an dem 3' Ende des neo-Gens in dem MPIN-Vektor schneidet. Ein Fragment, das das vollständige neo-Gen enthielt, wurde als Sonde verwendet.

### Herstellung und Reinigung des Virus

Die Virionen wurden durch Gradienten Ultrazentrifugation wie im Detail früher beschrieben gereinigt (L. Martinez-Peralta et al., J. Gen. Virol. 55 (1981) 475-479). Kurz gesagt wurden infektiöse Zellkulturüberstände durch Zentrifugation bei niedrigen und hohen Geschwindigkeiten gereinigt. Das Virus wurde durch Ultrazentrifugation pelletiert und dann in einem 0-40 %-igen Urografin Gradienten (Schering AG, Berlin, Deutschland) gereinigt.

### Beispiel 1

### Infektion von TeLCeb mit LCMV-LacZ-Gentransfer auf Zielzellen mit Neutralisation des Vektors durch anti-GP mAb und Konzentrierung des Vektors im Gradienten

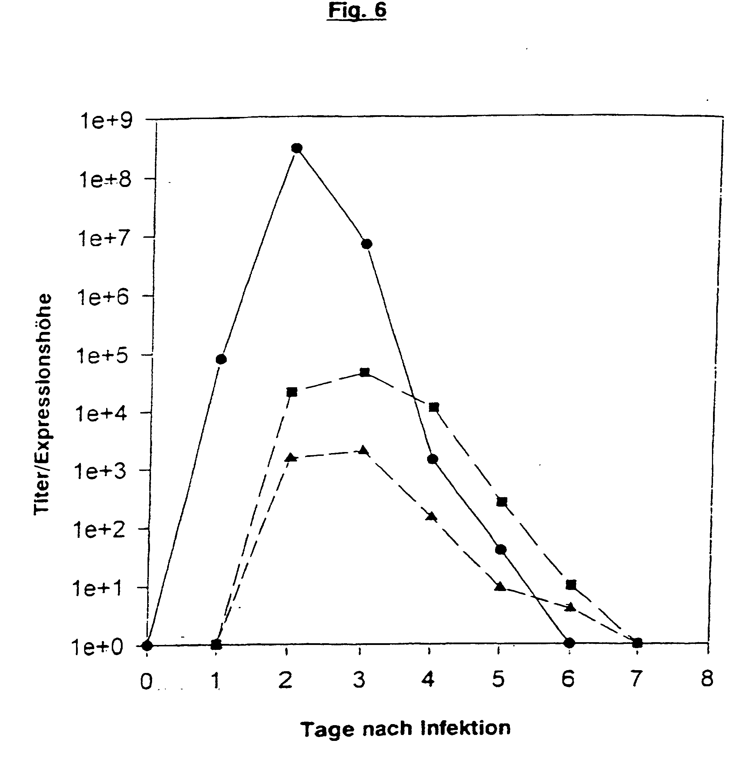

Wiederherstellung (rescue) eines Hüllprotein-negativen Murine Leukemia Virus Vector mit Lymphocytic Choriomeningitis Virus: Um zu testen, ob LCMV einen Hüllprotein-negativen retroviralen Vektor wiederherstellen (mobilisieren, komplettieren) kann, wurde die env-negative Verpackungszellinie TELCeB mit dem LCMV WE Stamm bei einer m.o.i. von 0.01 infiziert (m.o.i.: Infektionsmultiplizität; bezeichnet die Anzahl von Viren-Partikeln, mit denen eine Zelle infiziert wird). TELCeB stammen von der Humanfibroblastenzellinie Te671 ab und enthalten *gag-* und *pol*-Gene sowie den retrovialen Vektor MFGnlsLacZ (G.M. Crooks et al., Blood 82 (1993) 3290-3297). Nach Infektion mit LCMV wurde der Titer von LacZ transferring units (LTU) und von LCMV Wildtypvirus durch X-Gal-Färben der Mausfibroblasten-Zielzellen (Sc-1) und durch einen Plaqueversuch (in plaquebildenden Einheiten, PFU) gemessen. Zusätzlich wurde die Expression von LCMV-Glykoproteinen in den infizierten TELCeB durch durchflußzytometrische Analyse gemessen. Die Resultate sind in Fig. 6 gezeigt. LTU wurden sechs Tage lang hergestellt, mit einem Maximum von 5 x 10⁴ LTU pro ml an Tag 3. Der höchste Titer für LCMV Wildtypvirus betrug 3 x 10⁸ an Tag 2. Die Produktion von PFU hatte bereits an Tag 3 abgenommen, als die maximale Produktion von LTU zusammen mit der Höchstexpression von LCMV Glykoprotein zu sehen war. Diese Diskrepanz könnte in der Herstellung von defekten interferierenden LCMV Partikeln begründet sein, die die Replikation von LCMV Wildtyp inhibieren, mit größter Wahrscheinlichkeit aber nicht die Ausschüttung von infektiösen retroviralen Vektorpartikeln. Während der Replikation von LCMV in den Verpackungszellinien war kein offensichtlicher zytopathischer Effekt zu beobachten, obwohl hohe Spiegel an LCMV Glykoproteinen expremiert wurden (Daten nicht gezeigt).

Es wurde dann getestet, ob das infektiöse Virus, das durch die LCMV-infizierten TELCeB hergestellt wurde, den LacZ-Gentransfer spezifisch durch LCMV Glykoproteine vermittelte. Die Überstände wurden mit einem neutralisierenden Anti-LCMV gp44 monoklonalem Antikörper eine Stunde lang inkubiert. Dies führte zu einer mehr als dreifachen Log Reduzierung des LTU-Titers. Der amphotrophe Pseudotyp desselben retroviralen Vektors wurde nicht durch den anti-LCMV Antikörper neutralisiert (Tab. 1). Diese Daten zeigen, daß die MLV/LCMV chimären Virionen tatsächlich LCMV Glykoproteine auf ihrer Oberfläche trugen, die den Gentransfer in Abwesenheit von retroviralen Hüllproteinen durch den LCMV Rezeptor vermitteln können.

**Tab. 1:**

| Infektiöse retrovirale Vektor-Partikel, die von LCMV-infizierten Verpackungszellinien produziert werden, lassen sich durch anti-LCMV-Glykoprotein monoklonale Antikörper (mAb) neutralisieren. | | |
|---|---|---|
| Virus | anti-LCMV mAb | Titer (LTU/ml) |
| Amphotroper Helfer | nein | 2 · 10⁵ |
| Amphotroper Helfer | ja | 2 · 10⁵ |
| LCMV | nein | 7 · 10⁴ |
| LCMV | ja | 3 · 10¹ |

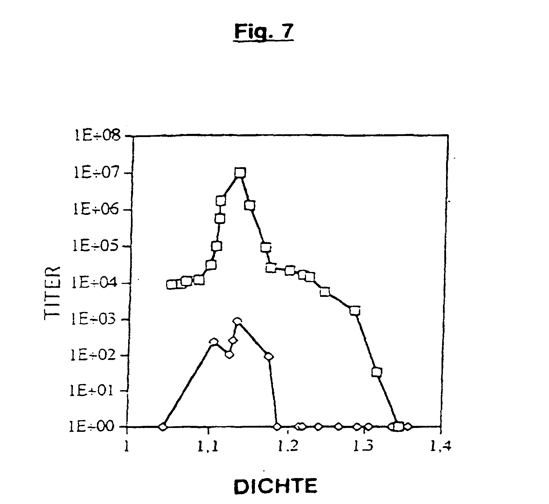

MLV (LCMV) Pseudotypen behalten Infektiosität bei Konzentrierung mittels Ultrazentrifugation durch einen Gradienten: Amphotrophe Retroviren verlieren Infektivität nach Ultrazentrifugation, höchstwahrscheinlich aufgrund der Labilität der retroviralen Hüllglykoproteine (V. Moenning et al., Virology 61 (1974) 100-111). Es wurde untersucht, ob die MLV(LCMV)-Pseudotypen stabiler sind. TELCeB wurden mit LCMV oder mit amphotrophem Helfervirus infiziert. Titer des viralen Vektors im direkten Überstand betrugen 5x 10⁶ LTU pro ml für beide Pseudotypen. Viren wurden aus 60 ml Überstand pelletiert und durch Ultrazentrifugation über einen 0-40%-igen Urografin-Gradienten gereinigt. Die Pseudotypentiter (in LTU pro ml) sind in Fig. 7 gezeigt. Der gesamte erwartete Ertrag von 3 x 10⁶ LTU für den LCMV Pseudotyp wurde vollständig erhalten, im Gegensatz zu 1 x10³ LTU für den amphotrophen Virus. Die reverse Transriptase-Aktivität in den Banden zeigte, daß die Menge an Viruspartikeln, die aus dem Gradienten gewonnen wurde, für beide Pseudotypen ähnlich war (Daten nicht gezeigt). Verglichen mit den amphotrophen Virionen war die Infektivität von MLV(LCMV)-Pseudotypen bei Ultrazentrifugation allerdings mindestens um einen Faktor 1000 stabiler.

LCMV Pseudotypen waren auch bei Lagerung bei 4°C stabil. Innerhalb des Beobachtungszeitraumes von drei Tagen war der Titerverlust zweifach niedriger als (im Vergleich zum Ausgangstiter des MLV(LCMV)). Ein Tieffrierzyklus (-80°C) und Auftauen führte zu einem Verlust an Pseudotyptiter, der zweifach niedriger lag.

### Beispiel 2

### Gag- und pol-Genprodukte sind erforderlich für die Verpackung von retroviralen RNA in die LCMV Glykoprotein Pseudotypen

Es wurde untersucht, ob die retrovirale RNA alleine in den LCMV verpackt werden könnte oder ob gag und pol Genprodukte erforderlich waren. 293-Zellen und 293gp2-Zellen, die letzteren enthielten *gag* und *pol* von MLV, wurden mit einem auf MLV-basierenden retroviralen Vektor transfiziert, der das *neo*-Gen (MP1N) enthielt, und Zellinien, die den stabil integrierten Vektor enthielten, wurden durch G418-Selektion hergestellt (293MP1N und 293gp2MP1N; ein als SF23 bezeichneter Klon der Zellinie 293gp2MP1N wurde am 05.11.1998 bei der Deutschen Sammlung von Mikroorganismen und Zellkulturen (DSMZ), Mascheroder Weg 1b, 38124 Braunschweig, Deutschland, unter der Zugriffsnummer DSM ACC2374 nach dem Budapester Vertrag hinterlegt). Diese Zellen (Massenkulturen) wurden dann entweder mit einem replikationskompetenten amphotropen Helfer oder mit dem LCMV-Wildtypvirus infiziert. Die Resultate sind in Tab. 2 gezeigt. Infektiöser Vektor, der Neomycin-Resistenz transferierte, wurde von beiden Zellinien nach Infektion mit dem amphotrophen Helfer gewonnen. Nach Infektion mit LCMV allerdings produzierte nur 293gpMPlN infektiöse retrovirale Partikel, 293MP1N, die kein retrovirales Gag oder Pol exprimierte, hingegen nicht. Retrovirale genomische RNA wurde daher in Abwesenheit von *gag*- und *pol*-Genprodukten durch LCMV nicht in infektiöse Virionen verpackt.

**Tab. 2:**

| *Gag-* und/oder *pol*-Genprodukte sind für die Wiederherstellung (rescue) eines retroviralen Vektors durch LCMV essentiell | | | |
|---|---|---|---|
| Zellinie | freigesetzter Vektortiter* nach Infektion mit | | |
| | LCMV | amphotrop. Helfer | (Kontrolle) |
| 293MP1N | 0 | 1 · 10⁴ | 0 |
| 293gp2MP1N | 2 · 10³ | 6 · 10⁴ | 0 |

| | | | |
|---|---|---|---|
| *Vektortiter werden in G418-resistenten Zellkolonien exprimiert, die man nach Inkulation von Sc-1 mit den viralen Überständen erhielt (G418 Transfereinheiten/ml) | | | |

### Beispiel 3

### Infektion von 293gpMP1N mit LCMV und stabiler Gentransfer auf L929 - Nachweis durch Southern-Blot

MLV (LCMV) Pseudotypen vermitteln Transfer und stabile Integration des retroviralen Vektor Genoms: Der Transfer von G418-Resistenz durch den retroviralen LCMV-Pseudotyp zeigte, daß das Markergen in das Wirtsgenom stabil integriert worden war. Um zu verifizieren, daß MLV(LCMV)-Pseudotypen stabile Transduktion mit Integration des Transgens in das Zielzellengenom vermitteln können, wurde ein retroviraler Vektor, der das Neomycin-Resistenzgen (*neo*) enthielt, durch LCMV-Infektion der *env*-negativen Verpackungszellinie 293gp2MP1N wiederhergestellt. Die Titer wurden durch Transfer von G418-Resistenz auf Sc-1 Zellen gemessen und lagen zwischen 1 x 10³ und 1 x 10⁴ G418-Transfereinheiten (GTU) pro ml. Resistente Zellklone tauchten nach acht Tagen Selektion auf und wurden für weitere drei Wochen kultiviert. Die DNA von 12 G418-resistenten Klonen wurde einer Southern Blot Analyse nach Restriktion mit HindIII unterworfen, einem Enzym, daß nur einmal schneidet, unter Verwendung einer Neo-Sonde. In 10 Klonen wurde eine Kopie des integrierten retroviralen Vektor-Genoms pro Zelle detektiert, und zwei Kopien in den übrigen zwei Klonen (Daten nicht gezeigt). Transduktion mit dem MLV(LCMV) Pseudotyp führte daher zu stabiler Integration des Transgens.

### Beispiel 4

### Expression von LCMV-Glykoprotein (LCMV-GP) in TeLCeB-L(Arm)

### Material und Methoden

Die Anlage der env-negativen Verpackungslinie TeLCeb, die *gag* und *pol* von MLV sowie ein retrovirales Vektorgenom mit LacZ als Transgen enthält, wurde bereits ausführlich beschrieben (F.L. Cosset et al., J. Virol. 69 (1995) 7430-7436). Die Titration der Vektorüberstände wurde wie bereits beschrieben auf 293-Zellen durch X-Gal Färbung vorgenommen (G.R. McGregor, Methods Mol. Biol. 7 (1989) 1-19). Die Zellen wurden in DMEM mit 10 % FCS kultiviert. Der L(Arm)-Stamm von LCMV entsteht nach mehreren Passagen von LCMV in L929-Zellen (M. Bruns et al., Virology 177 (1990) 615-624). LCMV-Nukleoprotein (LCMV-NP) von L(Arm) wurde durch Immunfluoreszenzfärbung der Zellen auf Objektträgern mit einem polyklonalen anti-LCMV Kaninchenserum nachgewiesen. Diese Standardmethode wurde bereits zuvor detailliert beschrieben (M. Bruns et al., Virology 177 (1990) 615-624). Zur Expression von LCMV-GP, wurde das *gp*-Gen in den episomalen EBV-Vektor pCep4 (Invitrogen), der ein Hygromycin-Resistenzgen trägt, kloniert.

### Ergebnisse

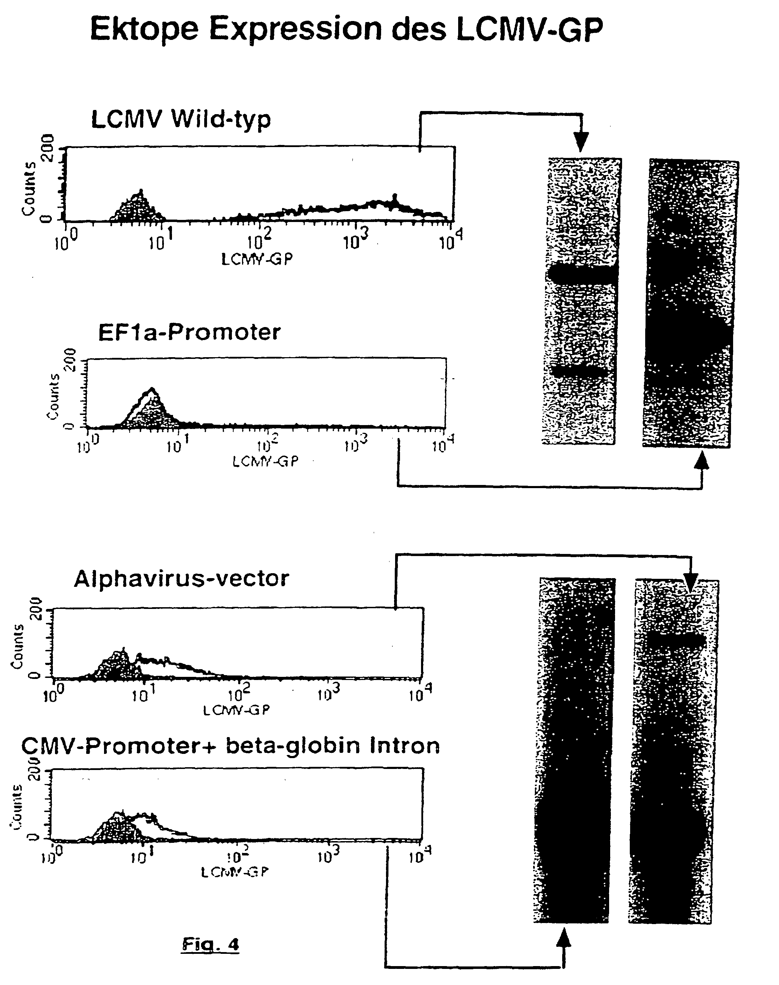

Bei den Experimenten zur Pseudotypisierung durch die alleinige Expression von LCMV-GP in env-negativen retroviralen Verpackungslinien wurde mit den Expressionsplasmiden eine höhere GP-mRNA--Expression erzielt als bei LCM-Wildvirus-Infektion (Fig. 4). Dieses Ergebnis zeigt, daß das gleichzeitige Vorliegen noch mindestens eines weiteren LCMV-Genprodukts neben dem LCMV-Glykoprotein eine Steigerung der Glykoprotein-Produktion bewirkt bzw. die Bildung von Pseudotypen fördert. Um diese Schlußfolgerung direkt zu untermauern, wurde das ektop (von einem Plasmid) exprimierte LCMV-GP mit den LCMV Proteinen des L(ARM) Stammes von LCMV komplementiert. Diesem defekten Stamm fehlt das funktionelle Glykoprotein, und er bildet daher keine Plaques, ist nicht pathogen für Mäuse und breitet sich nur über mehrere Wochen innerhalb einer Zellkultur aus (LCM-Wildvirus dagegen innerhalb von 24 Stunden). Alle weiteren Genprodukte von L(ARM) (NP,L und Z) weisen keine nachweisbaren Defekte auf.

TeLCeb wurden mit L(Arm)-haltigem Zellkulturüberstand infiziert und anschließend 5 Wochen passagiert. Dieses ist erfahrungsgemäß der Zeitraum, den das defekte Virus benötigt, um alle Zellen einer Kultur zu infizieren. Die vollständige Infektion aller Zellen wurde durch Immunfluoreszenz-Färbung mit einem anti-LCMV Serum nachgewiesen. TeLCeb-L(Arm) wurden mit pCep-GP durch Elektroporation (Elektroporator der Firma Dr. Fischer, Heidelberg) transfiziert und 2 Wochen mit Hygromycin selektioniert. Als Kontrolle wurden Zellen mit pCep4 (ohne GP-Gen) transfiziert. In TeLCeb-L(Arm), die mit pCep-GP transfiziert waren, kam es nach der Selektion zur Produktion von Pseudotypen, die lacZ auf 293-Zellen übertrugen. Der Titer lag zwischen 10² und 10³/ml. Dieses Ergebnis zeigt eindeutig, daß das LCMV-GP im beschriebenen Expressionsplasmid funktionell war und retrovirale Vektoren pseudotypisieren kann.

### Beispiel 5

### Pseudotypisierung eines HIV-Vektors, der das green fluorescent protein (GFP) exprimiert

### Material und Methoden

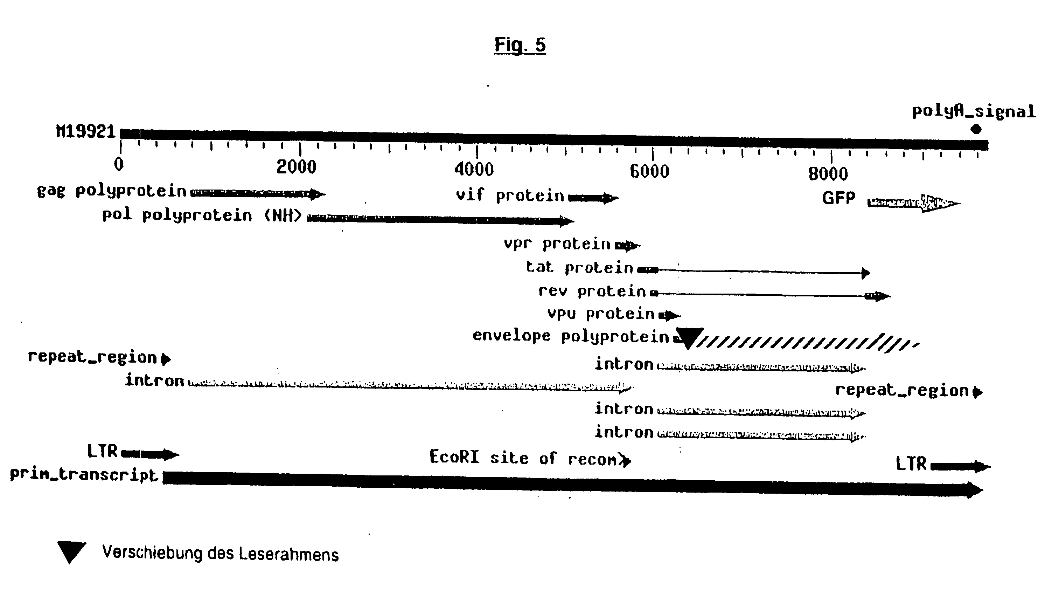

Der Lentivirale Vektor HIV-GFP leitet sich von dem infektiösen DNA-Klon pNL4-3 von HIV ab und ist bereits detailliert beschrieben (Abb. 5) (R.I. Connor et al., Virology 206 (1995) 935-944). Am Anfang von *env* wurde die NdeI-Schnittstelle aufgefüllt und religiert, wodurch sich das Leseraster verschiebt und kein funktionelles Hüllprotein synthetisiert wird. An Stelle von *nev* wurde außerdem das Gen für das green fluorescent protein (GFP) kloniert. Der Titer des verwendeten LCMV-WE-Stammes wurde durch einen Plaque Assay auf L929 bestimmt, der bereits detailliert beschrieben ist (F. Lehmann-Grube et al., J. Gen. Virol. 37 (1977) 85-92). Die Calciumphosphat-Transfektionen wurden auf 293 mit einem Standard-Protokoll durchgeführt (Maniatis, et al.: Molecular cloning, a laboratory manual; Cold Spring Harbor Laboratory Press, 1982).

### Ergebnisse

293 Zellen wurden mit einer m.o.i. von 0.1 des LCMV-WE-Stammes infiziert. Nach einer Stunde erfolgte die Transfektion mit HIV-GFP und nach zwei Tagen wurden die Überstände geerntet und auf 293 Zellen übertragen. Der Titer wurde durch die GFP-Expression in den 293-Zielzellen mittels Immunfluoreszenz ermittelt und lag zwischen 10² und 10³ pro ml. Nach Transfektion mit HIV-GFP allein (ohne vorherige Infektion mit LCMV) kam es wie erwartet nicht zur Produktion von infektiösen Vektorpartikeln.

### Beispiel 6

### Untersuchung des Zelltropismus

MLV(LCMV) Pseudotypen infizieren verschiedene humane Zellinien: Der Tropismus von MLV(LCMV) Pseudotypen wurde analysiert. Verschiedene humane Zellinien, die von Zellen abstammten, die attraktive Ziele für Gentherapie sind, wie hämatopoetische Vorläuferzellen und Hepatozyten, wurden analysiert. Die Transfereffizienz bezogen auf Mausfibroblasten ist in Tab. 3 gezeigt. Alle analysierten Zellinien waren für MLV(LCMV)-Pseudotypen empfänglich. Auch Hamsterzellen, die normalerweise resistent gegenüber einer Transduktion mit von MLV abstammenden Vektoren sind, konnten mit den MLV(LCMV)-Pseudotypen effizient transduziert werden.

**Tab. 3:**

| Wirtsspektrum von MLV(LCMV)-Pseudotypen | | |
|---|---|---|
| Zellinie | Herkunft | Transduktionseffizienz |
| 293 | Epithel, Mensch | + + + |
| K-562 | myeloide Progenitorzellen, Mensch | + + + |
| TF-1 | myeloide Progenitorzellen, Mensch | + |
| HUH-1 | Hepatom, Mensch | + + |
| Jurkat | Lymphozyt, Mensch | + +^{*} |
| Sc-1 | Fibroblast, Maus | + + + |
| CHO | Epithel, Hamster | + + + |
| Cf2Th | Thymus stroma, Hund | + + + |

| | | |
|---|---|---|
| ^{*} Die Pseudotypen wurden mit auf Lymphozyten passagiertem (adaptiertem) LCMV hergestellt. Pseudotypen aus Fibroblasten-passagiertem LCMV transduzieren Lymphozyten nicht. | | |

### FIGUREN:

- Fig. 1:: Retrovirale Verpackungslinien
Die retroviralen Gene gag, pol und env sind stabil in das Genom der retroviralen Verpackungslinien integriert. Env wird in der Regel getrennt von gag und pol exprimiert. Außerdem wird das Vektorgenom mit dem Transgen und regulatorischen cis-Elementen exprimiert (1). Die genomische RNA des Vektor enthält ein Verpackungssignal, das auf den gag, pol und env Genen deletiert wurde. Im Zytoplasma verpacken die retroviralen Proteine daher selektiv das Vektorgenom (2). Ein komplettes retrovirales Nukleokapsid bildet sich (3), und wird durch Sprossung an der Zellmembran freigesetzt (4). Hierbei wird retrovirales Hüllprotein, bzw. bei den Pseudotypen ein fremdes virales Hüllprotein, mitgenommen. Außerhalb der Zelle reift das Virus durch proteolytische Spaltung der retroviralen Vorläuferproteine im Nukleokapsid.

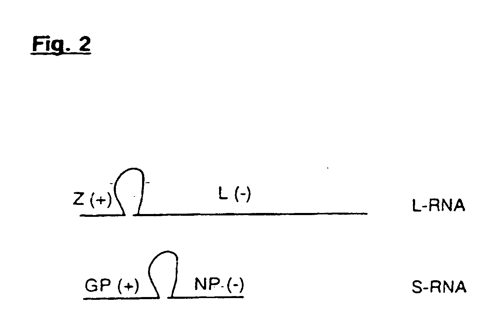

- Fig. 2:: Die genomische RNA des LCM-Virus Das Genom von LCMV besteht aus 2 ambisense RNA Molekülen mit je zwei offenen Leserahmen. Die 4 Gene und ihre Orientierung sind angegeben.

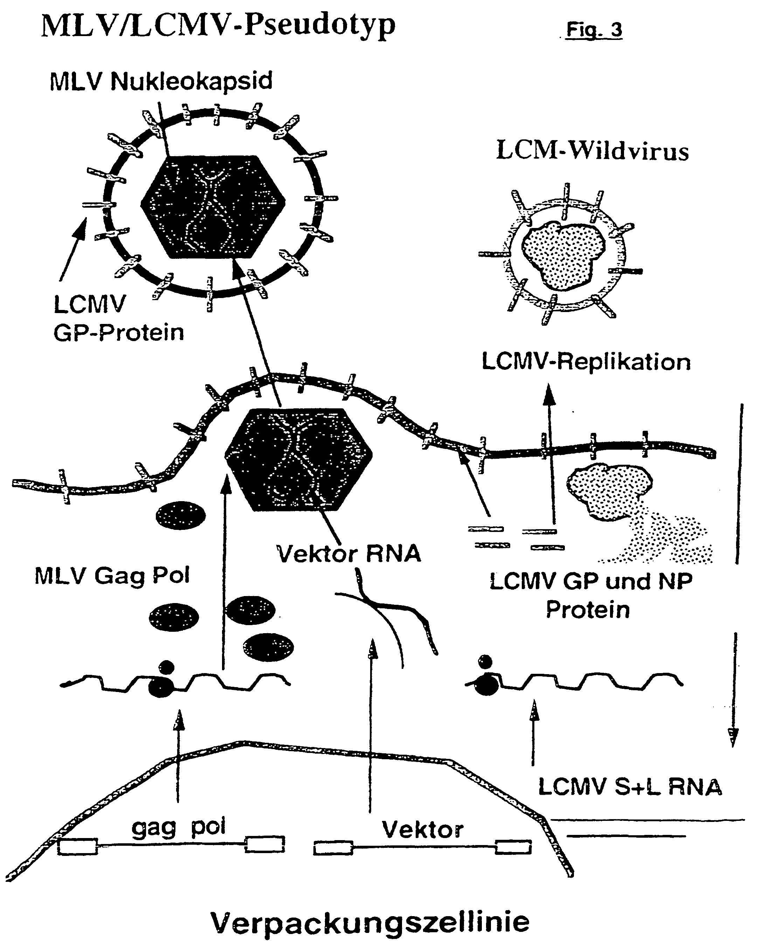

- Fig. 3:: Die Herstellung von MLV(LCMV) Pseudotypen. Verpackungslinien, die keine viralen Hüllproteine produzieren, setzen auch keine infektiösen retroviralen Vektoren frei. Wird eine solche env-negative Verpackungslinie mit LCM-Virus infiziert, so können die Retroviren das LCMV-Glykoprotein in ihre Hülle einbauen. Es entstehen sogenannte Pseudotypen. Außerdem wird während der LCMV-Replikation auch LCM-Wildvirus freigesetzt.
- Fig. 4:: Diskrepanz von hoher mRNA und niedriger Proteinkonzentration bei ektoper GP-C-Expression. 293-Zellen wurden mit unterschiedlichen Expressionskonstrukten für LCMV Glykoprotein transfiziert (Ef-1 alpha Promoter, Alphavirus-Vektor von Invitrogen, CMV-Promoter+Beta-Globin-Intron, s.o.: bevorzugte Expressionskassetten). Nach 2 Tagen wurden parallel die gp-mRNA Konzentration im Northern Blot und die GP-1/GP-2 Protein-Mengen auf den Zellen mittels Durchflußzytometrie gemessen. Als Kontrolle dienten 293-Zellen, die mit LCMV Wild-Typ infiziert waren. Bei der Wildvirusinfektion lag die GP-1/GP-2 Protein-Expression 2-3 Logstufen über der Negativkontrolle, bei einer relativ schwachen mRNA-Bande im Northern Blot (obere Bande LCMV-genomische RNA, untere Bande gp-mRNA). Bei ektoper Expression lag trotz höherer mRNA-Konzentrationen als bei der Wildvirus-Infektion die Proteinkonzentration für GP-1/GP-2 nur maximal eine Log-Stufe über.der Negativkontrolle.
- Fig. 5:: HIV-GFP
Der infektiöse DNA-Klon von HIV pNL4-3 wurde am Anfang des env Genes mit NdeI geschnitten, die Schnittstelle aufgefüllt und religiert (s). Hierdurch verschiebt sich der Leserahmen und es entsteht kein funktionelles env Genprodukt. Zwischen XhoI und BamHI wurde anstelle von nef das Gen für das "green fluorescent protein" kloniert (GFP).
- Fig. 6:: Wiederherstellung (rescue) des retroviralen Vektors MFGlnsLacZ durch LCMV.
Die retrovirale env-negative Verpackungszellinie TELCeB wurde durch LCMV bei einer m.o.i. von 0.01 infiziert. Zwischen Tag 1 und 7 nach Infektion wurden die Überstände täglich ersetzt, und es wurden die Titer des LCMV-Wildtyps und des LacZ-Vektors durch einen Plaque-Assay auf L-929 bzw. durch LacZ-Gentransfer auf Sc-1 ermittelt. Zusätzlich wurde ein Teil der LCMV-infizierten TELCeB-Zellen täglich mit einem gegen das LCMV-Glykoprotein GP-1 gerichteten monoklonalen Antikörper gefärbt und mittels Durchflußzytometrie analysiert. Es ist die mittlere Fluoreszenz gezeigt. O-O LCMV-Wildtyp-Titer; □-□ LacZ-Transfereinheiten; Δ-Δ mittlere Fluoreszenz der LCMV Glykoprotein GP-Expression.
- Fig. 7:: MLV(LCMV)-Pseudotypen bewahren ihre Infektivität nach Ultrazentrifugation.
TELCeB wurden mit LCMV oder amphotropem Helfervirus infiziert. Die Überstände wurden geerntet und eingefroren. Es wurden die MLV(LCMV)- und amphotropen Pseudotyp-Titer bestimmt. Gleiche Mengen des infectiösen Virus wurde mittels Ultrazentrifugation pelletiert und dann einer Reinigung an einem 0 % - 40 % Urografin-Gradienten unterworfen. Vektor-Titer und -dichten wurden in jeder Fraktion bestimmt. O-O amphotroper Pseudotyp; □-□ MLV(LCMV)-Pseudotyp.

## Patentansprüche

1. Retrovirale verpackungszelle, **dadurch gekennzeichnet, daß** sie die retroviralen Gene *gag, pol* und ferner das für die Glykoproteine GP-1 und GP-2 des LCMV kodierende Gen *gp* enthält.

2. Retrovirale Verpackungszelle nach Anspruch 1, **dadurch gekennzeichnet, daß** der LCMV-Stamm LCMV-Wildtyp, LCMV-Armstrong, LCMV-WE oder LCMV WE-HPI ist.

3. Retrovirale Verpackungszelle nach Anspruch 1 und 2, **dadurch gekennzeichnet, daß** die gp-Nukleinsäureseqüenz eine Nukleinsäuresequenz ist, die gegenüber der Nukleinsäuresequenz von LCMV Punktmutationen oder Deletionen aufweist.

4. Retrovirale Verpackungszelle nach den Ansprüchen 1 bis 3, **dadurch gekennzeichnet, daß** sie ferner das retrovirale Gen *env* und/oder regulatorische retrovirale Gene enthält.

5. Retrovirale Verpackungszelle nach den Ansprüchen 1 bis 4, **dadurch gekennzeichnet, daß** sie ferner mindestens ein Gen aus der Gruppe bestehend aus dem für das Nukleoprotein kodierenden Gen np, dem für die RNA-Polymerase kodierenden Gen 1 und dem für ein Protein mit unbekannter Funktion kodierenden Gen z des LCMV enthält.

6. Retrovirale Verpackungszelle nach den Ansprüchen 1 bis 5, **dadurch gekennzeichnet, daß** das Retrovirus aus der Gruppe bestehend aus MLV-related viruses und Lentiviren ausgewählt ist.

7. Retrovirale Verpackungszelle nach Anspruch 6, **dadurch gekennzeichnet, daß** das Retrovirus aus MLV, HIV, SIV oder FIV ausgewählt ist.

8. Retrovirale Verpackungszelle nach den Ansprüchen 1 bis 7, **dadurch gekennzeichnet, daß** sie ein Transgen oder mehrere Transgene enthält, das/die aus der Gruppe bestehend aus Markergenen und/oder therapeutisch einsetzbaren Genen - ausgewählt ist/sind.

9. Retrovirale Verpackungszelle nach Anspruch 8, **dadurch gekennzeichnet, daß** die Markergene aus der Gruppe bestehend aus *neo, lacz* oder *EGFP* ausgewählt sind.

10. Retrovirale Verpackungszelle nach Anspruch 8 oder 9, **dadurch gekennzeichnet, daß** die therapeutisch einsetzbaren Gene aus der Gruppe bestehend aus den Suizidgenen Herpes Simplex Virus Thymidin Kinase (HSV-tk) und Cytosin Deaminase (CD), antiviral wirksamen Sequenzen und in der Tumortherapie entsetzbaren Genen ausgewählt sind.

11. Retrovirale Verpackungszelle nach Anspruch 10, **dadurch gekennzeichnet, daß** die antiviral wirksamen Sequenzen aus der Gruppe bestehend aus Ribozymen, antisense Sequenzen, transdominant-negativ wirkenden Genen und Zytokin-Genen ausgewählt sind.

12. Retrovirale Verpackungszelle nach Anspruch 10, **dadurch gekennzeichnet, daß** das in der Tumortherapie entsetzbare Gen *mdr-1* ist.

13. Retrovirale verpackungszelle nach den Ansprüchen 1 bis 12, die mit Lymphozytärem Choriomeningitisvirus infiziert ist und die Fremdgene enthält.

14. Retrovirale Verpackungszelle nach den Ansprüchen 1 bis 12, **dadurch gekennzeichnet, daß** sie durch Transfektion einer Verpackungszelle mit einem Expressionsplasmid erhältlich ist, das das gp-Gen des LCMV nach Anspruch 1 bis 3 und gegebenenfalls zusätzlich das *np*-Gen, das *l*-Gen und/oder das z-Gen des LCMV oder Teile derselben umfaßt.

15. Retrovirale Verpackungszelle nach den Ansprüchen 1 bis 14, **dadurch gekennzeichnet, daß** sie das retrovirale Gen *env* enthält, das für ein Env-Protein kodiert, das eine spezifische Bindung an die Zielzelle vermittelt, und wobei *gp* von LCMV eine Variante ist, die für ein GP-Protein kodiert, das Fusionsaktivität aufweist.

16. Retrovirale Verpackungszelle nach den Ansprüchen 1 bis 15, **dadurch gekennzeichnet, daß** *gp* für die in SEQ ID NO: 26 dargestellte Sequenz kodiert.

17. Retrovirale Verpackungszelle nach den Ansprüchen 1 bis 16, **dadurch gekennzeichnet, daß** *gp* die in SEQ ID NO: 25 dargestellte Sequenz aufweist.

18. Retrovirale Verpackungszelle nach den Ansprüchen 8 bis 13, **dadurch gekennzeichnet, daß** das Retrovirus MLV ist, das kein Env-Protein exprimiert und LCMV die defekte Mutante L(ARM) ist.

19. Retrovirale Verpackungszelle, die die retroviralen Gene *gag* und *pol* enthält, **dadurch gekennzeichnet, daß** sie pseudotypisierte Virionen bildet, die LCMV-Glykoprotein in ihrer Hülle enthalten.

20. Pseudotypisiertes retrovirales Virion, **dadurch gekennzeichnet, daß** es die Glykoproteine GP-1 und GP-2 des LCMV umfaßt.

21. Retroviraler Pseudotyp-Vektor, **dadurch gekennzeichnet, daß** er die Glykoproteine GP-1 und GP-2 des LCMV umfaßt.

22. Verfahren zur Herstellung einer retroviralen Verpackungszelle, bei dem man eine retrovirale Verpackungszelle, die die retroviralen Gene *gag* und *pol* enthält, unter Bedingungen mit LCMV in Kontakt bringt, die die Infektion der retrovira-len Verpackungszelle mit LCMV erlauben, wobei die von der Zelle produzierten Virionen mit LCMV-Glycoproteinen pseudotypisiert sind.

23. Verfahren nach Anspruch 22, **dadurch gekennzeichnet, daß** man als retrovirale Verpackungszelle eine verpackungszelle einsetzt, die das retrovirale Gen env enthält, wobei env so modifiziert ist, daß es für ein Env-Protein kodiert, das eine spezifische Bindung an die zielzelle vermittelt und wobei LCMV eine *gp*-Variante enthält, die für ein GP-Protein kodiert, das Fusionsaktivität aufweist.

24. Verfahren nach den Ansprüchen 22 und 23, **dadurch gekennzeichnet, daß** gp für die in SEQ ID NO: 26 dargestellte Sequenz kodiert.

25. verfahren nach den Ansprüchen 22 bis 24, **dadurch gekennzeichnet, daß** *gp* die in SEQ ID NO: 25 dargestellte Sequenz aufweist.

26. Verfahren nach den Ansprüchen 22 und 23, **dadurch gekennzeichnet, daß** man als LCMV die deletierte Mutante L(ARM) verwendet.

27. Verfahren zur Herstellung einer retroviralen Verpackungszelle nach den Ansprüchen 8 bis 19, **dadurch gekennzeichnet, daß** man eine mit einem oder mehreren Fremdgenen transfizierte retrovirale Verpackungszelle mit einem Expressionsplasmid transfiziert, das das *gp*-Gen des LCMV nach Anspruch 1 bis 3 und gegebenenfalls zusätzlich ein oder mehrere Gene aus der Gruppe bestehend aus *np, l* und z des LCMV enthält.

28. Verfahren nach Anspruch 27, **dadurch gekennzeichnet, daß** man als retrovirale Verpackungszelle eine Verpackungszelle einsetzt, die das retrovirale Gen env enthält, :das so modifiziert ist, daß es für ein Env-Protein kodiert, das eine spezifische Bindung an die Zielzelle vermittelt und wobei gp eine Variante ist, die für ein GP-Protein kodiert, das Fusionsaktivität aufweist.

29. Verfahren nach den Ansprüchen 27 und 28, **dadurch gekennzeichnet, daß** *gp* für die in SEQ ID NO: 26 dargestellte Sequenz kodiert.

30. Verfahren nach den Ansprüchen 27 bis 29, **dadurch gekennzeichnet, daß** *gp* die in SEQ ID NO: 25 dargestellte Sequenz aufweist.

31. Verfahren zur Herstellung retroviraler Pseudotyp-Vektoren, **dadurch gekennzeichnet, daß** man ein Verfahren nach den Ansprüchen 22 bis 30 durchführt und anschließend die Verpackungszellen unter Bedingungen kultiviert, die zur Produktion von Virionen geeignet sind.

32. Verwendung einer retroviralen Verpackungszelle nach den Ansprüchen 8 bis 19 zur *in vitro*-Infektion, von Zellen und zur Expression des Transgens in diesen Zellen.

33. Verwendung einer retroviralen verpackungszelle nach den Ansprüchen 8 bis 19, gegebenenfalls formuliert mit pharmazeutisch verträglichen Hilfs- und/oder Trägerstoffen, zur Herstellung eines pharmazeutischen Präparates zur Gentherapie.

34. Verwendung nach Anspruch 32 und 33 zur *in vitro*-Transfektion hämatopoetischer Stammzellen.

35. Verwendung nach den Anspruch 33 und 34, wobei die Gentherapie die Therapie von infektiösen Erkrankungen, Neoplasien, Melanomen sowie anderen durch Gentherapie zugänglichen Erkrankungen umfaßt.

36. Verwendung nach Anspruch 35, **dadurch gekennzeichnet, daß** die infektiöse Erkrankung eine HIV-Infektion oder AIDS ist.

37. Verwendung nach Anspruch 35, **dadurch gekennzeichnet, daß** die Neoplasie das Mamma-Karzinom ist.

38. Pharmazeutisches Präparat zur Gentherapie, **dadurch gekennzeichnet, daß** es retrovirale Verpackungszellen nach einem der Ansprüche 8 bis 19 und gegebenenfalls pharmazeutisch verträgliche Hilfs- und/oder Trägerstoffe umfaßt.

39. Pharmazeutisches Präparat nach Anspruch 38, **dadurch gekennzeichnet, daß** die Gentherapie die Therapie von infektiösen Erkrankungen, Neoplasien, Melanomen sowie anderen durch Gentherapie zugänglichen Erkrankungen umfaßt.

40. Pharmazeutisches Präparat nach Anspruch 39, **dadurch gekennzeichnet, daß** die infektiöse Erkrankung eine HIV-Infektion oder AIDS ist.

41. Pharmazeutisches Präparat nach Anspruch 39, **dadurch gekennzeichnet, daß** die Neoplasie das Mamma-Karzinom ist.

## Claims

1. Retroviral packaging cell, **characterized in that** it contains the retroviral *gag* and *pol* genes and also the gp gene which encodes the LCMV GP-1 and GP-2 glycoproteins.

2. Retroviral packaging cell according to Claim 1, **characterized in that** the LCMV strain is wild-type LCMV, LCMV Armstrong, LCMV WE or LCMV WE-HPI.

3. Retroviral packaging cell according to Claims 1 and 2, **characterized in that** the gp nucleic acid sequence is a nucleic acid sequence which, compared to the LCMV nucleic acid sequence, has point mutations or deletions.

4. Retroviral packaging cell according to Claims 1 to 3, **characterized in that** it further contains the retroviral *env* gene and/or regulatory retroviral genes.

5. Retroviral packaging cell according to Claims 1 to 4, **characterized in that** it further contains at least one gene from the group consisting of the LCMV *np* gene coding for the nucleoprotein, the LCMV *l* gene coding for the RNA polymerase, and the LCMV z gene coding for a protein with an unknown function.

6. Retroviral packaging cell according to Claims 1 to 5, **characterized in that** the retrovirus is selected from the group consisting of MLV-related viruses and lentiviruses.

7. Retroviral packaging cell according to Claim 6, **characterized in that** the retrovirus is selected from MLV, HIV, SIV or FIV.

8. Retroviral packaging cell according to Claims 1 to 7, **characterized in that** it contains one or more transgenes selected from the group consisting of marker genes and/or therapeutically usable genes.

9. Retroviral packaging cell according to Claim 8, **characterized in that** the marker genes are selected from the group consisting of *neo, lacZ* and *EGFP.*

10. Retroviral packaging cell according to either of Claims 8 and 9, **characterized in that** the therapeutically usable genes are selected from the group consisting of the suicide genes Herpes simplex virus thymidine kinase (HSV tk) and cytosine deaminase (CD), sequences with antiviral action and genes usable in tumour therapy.

11. Retroviral packaging cell according to Claim 10, **characterized in that** the sequences with antiviral action are selected from the group consisting of ribozymes, antisense sequences, genes with transdominant-negative action and cytokine genes.

12. Retroviral packaging cell according to Claim 10, **characterized in that** the gene usable in tumour therapy is *mdr-1*.

13. Retroviral packaging cell according to Claims 1 to 12, which has been infected with lymphocytic choriomeningitis virus and contains foreign genes.

14. Retroviral packaging cell according to Claims 1 to 12, **characterized in that** it is obtainable by transfection of a packaging cell with an expression plasmid which comprises the LCMV *gp* gene according to Claims 1 to 3 and, where appropriate, additionally the LCMV *np* gene, *l* gene and/or *z* gene or parts thereof.

15. Retroviral packaging cell according to Claims 1 to 14, **characterized in that** it contains the retroviral *env* gene which encodes an Env protein imparting specific binding to the target cell, with LCMV *gp* being a variant which encodes a GP protein having functional activity.

16. Retroviral packaging cell according to Claims 1 to 15, **characterized in that** *gp* encodes the sequence depicted in SEQ ID NO:26.

17. Retroviral packaging cell according to Claims 1 to 16, **characterized in that** *gp* is the sequence depicted in SEQ ID NO:25.

18. Retroviral packaging cell according to Claims 8 to 13, **characterized in that** the retrovirus is MLV which does not express any Env protein and LCMV is the defective mutant L(ARM).

19. Retroviral packaging cell containing the retroviral *gag* and *pol* genes, **characterized in that** it forms pseudotyped virions whose envelope contains LCMV glycoprotein.

20. Pseudotyped retroviral virion, **characterized in that** it comprises the LCMV GP-1 and GP-2 glycoproteins.

21. Retroviral pseudotype vector, **characterized in that** it comprises the LCMV GP-1 and GP-2 glycoproteins.

22. Process for the preparation of a retroviral packaging cell, in which a retroviral packaging cell containing the retroviral *gag* and *pol* genes is contacted with LCMV under conditions which allow the retroviral packaging cell to be infected with LCMV, with the virions produced by the cell being pseudotyped with LCMV glycoproteins.

23. Process according to Claim 22, **characterized in that** the retroviral packaging cell employed is a packaging cell containing the retroviral *env* gene, with *env* having been modified so as to encode an Env protein and with LCMV containing a *gp* variant which encodes a GP protein having functional activity.

24. Process according to Claims 22 and 23, **characterized in that** *gp* encodes the sequence depicted in SEQ ID NO:26.

25. Process according to Claims 22 to 24, **characterized in that** *gp* is the sequence depicted in SEQ ID NO:25.

26. Process according to Claims 22 and 23, **characterized in that** the LCMV used is the deleted mutant L(ARM).

27. Process for the preparation of a retroviral packaging cell according to Claims 8 to 19, **characterized in that** a retroviral packaging cell transfected with one or more foreign genes is transfected with an expression plasmid comprising the LCMV gp gene according to Claims 1 to 3 and, where appropriate, additionally one or more genes of the group consisting of LCMV *np, l* and/or *z*.

28. Process according to Claim 27, **characterized in that** the retroviral packaging cell employed is a packaging cell containing the retroviral *env* gene which has been modified so as to encode an Env protein mediating specific binding to the target cell and with *gp* being a variant which encodes a GP protein having functional activity.

29. Process according to Claims 27 and 28, **characterized in that** *gp* encodes the sequence depicted in SEQ ID NO:26.

30. Process according to Claims 27 to 29, **characterized in that** *gp* is the sequence depicted in SEQ ID NO:25.

31. Process for the preparation of retroviral pseudotype vectors, **characterized in that** a process according to Claims 22 to 30 is carried out and the packaging cells are subsequently cultured under conditions suitable for producing virions.

32. Use of a retroviral packaging cell according to Claims 8 to 19 for infecting cells *in vitro* and expressing the transgene in these cells.

33. Use of a retroviral packaging cell according to Claims 8 to 19, formulated, where appropriate, with pharmaceutically compatible excipients and/or carriers, for the preparation of a pharmaceutical preparation for gene therapy.

34. Use according to Claims 32 and 33 for *in-vitro* transfection of haematopoietic stem cells.

35. Use according to Claims 33 and 34, in which the gene therapy comprises the therapy of infectious diseases, neoplasms, melanomas and other disorders accessible by gene therapy.

36. Use according to Claim 35, **characterized in that** the infectious disease is an HIV infection or AIDS.

37. Use according to Claim 35, **characterized in that** the neoplasm is mamma carcinoma.

38. Pharmaceutical preparation for gene therapy, **characterized in that** it comprises retroviral packaging cells according to any of Claims 8 to 19 and, where appropriate, pharmaceutically compatible excipients and/or carriers.

39. Pharmaceutical preparation according to Claim 38, **characterized in that** the gene therapy comprises the therapy of infectious diseases, neoplasms, melanomas and other disorders accessible by gene therapy.

40. Pharmaceutical preparation according to Claim 39, **characterized in that** the infectious disease is an HIV infection or AIDS.

41. Pharmaceutical preparation according to Claim 39, **characterized in that** the neoplasm is mamma carcinoma.

## Revendications

1. Cellule rétrovirale d'encapsidation, **caractérisée en ce qu'**elle contient les gènes rétroviraux *gag, pol* et de plus le gène *gp* codant pour les glycoprotéines GP-1 et GP-2 de LCMV.

2. Cellule rétrovirale d'encapsidation selon la revendication 1 **caractérisée en ce que** la souche de LCMV est LCMV du type sauvage, LCMV-Armstrong, LCMV-WE ou LCMV WE-HPI.

3. Cellule rétrovirale d'encapsidation selon la revendication 1 ou 2, **caractérisée en ce que** la séquence d'acide nucléique de gp est une séquence d'acide nucléique qui présente des mutations par points ou des délétions par rapport à la séquence d'acide nucléique de LCMV.

4. Cellule rétrovirale d'encapsidation selon les revendications 1 à 3, **caractérisée en ce qu'**elle contient de plus le gène rétroviral *env* et/ou des gènes rétroviraux régulateurs.

5. Cellule rétrovirale d'encapsidation selon les revendications 1 à 4 **caractérisée en ce qu'**elle contient de plus au moins un gène du groupe se composant du gène *np* codant pour la nucléoprotéine, du gène *1* codant pour l'ARN polymérase et du gène *z* codant pour une fonction inconnue de LCMV.

6. Cellule rétrovirale d'encapsidation selon les revendications 1 à 5, **caractérisée en ce que** le rétrovirus est choisi dans le groupe consistant en virus en rapport avec MLV et lentivirus.

7. Cellule rétrovirale d'encapsidation selon la revendication 6, **caractérisée en ce que** le rétrovirus est choisi parmi VLM, VIH, VIS ou VIF.

8. Cellule rétrovirale d'encapsidation selon les revendications 1 à 7 **caractérisée en ce qu'**elle contient un transgène ou plusieurs transgènes, qui est/sont choisis dans le groupe consistant en gènes marqueurs et/ou gènes thérapeutiquement utilisables.

9. Cellule rétrovirale d'encapsidation selon la revendication 8, **caractérisée en ce que** les gènes marqueurs sont choisis dans le groupe consistant en *neo, lacZ* ou *EGFP*.

10. Cellule rétrovirale d'encapsidation selon la revendication 8 ou 9, **caractérisée en ce que** les gènes thérapeutiquement utilisable sont choisis dans le groupe comprenant les gènes suicides thymidine kinase du virus de l'Herpès Simplex (HSV-tk) et cytosine désaminase (CD), des séquences antivirales efficaces et dans les gènes utilisables en chimiothérapie.

11. Cellule rétrovirale d'encapsidation selon la revendication 10, **caractérisée en ce que** les séquences antivirales efficaces sont choisies dans le groupe consistant en ribozymes, séquences anti-sens, gènes à effet négatif transdominant et gènes de cytokines.

12. Cellule rétrovirale d'encapsidation selon la revendication 10, **caractérisée en ce que** le gène utilisable en chimiothérapie est *mdr-1*.

13. Cellule rétrovirale d'encapsidation selon les revendications 1 à 12, qui est infectée du virus de la chorioméningite lymphocytaire et contient des gènes étrangers.

14. Cellule rétrovirale d'encapsidation selon les revendications 1 à 12, **caractérisée en ce qu'**elle peut être obtenue par transfection d'une cellule d'encapsidation avec un plasmide d'expression, qui contient le gène gp de LCMV selon les revendications 1 à 3 et le cas échéant, de plus, le gène np, le gène-1 et/ou le gène z de LCMV ou une partie de ceux-ci.

15. Cellule rétrovirale d'encapsidation selon les revendications 1 à 14, **caractérisée en ce qu'**elle contient le gène rétroviral *env* qui code pour une protéine env qui permet une liaison spécifique aux cellules cibles et où *gp* est une variante de LCMV qui code pour une protéine GP qui présente une activité de fusion.

16. Cellule rétrovirale d'encapsidation selon les revendications 1 à 15, **caractérisée en ce que** *gp* code pour la séquence représentée dans SEQ ID NO: 26.

17. Cellule rétrovirale d'encapsidation selon les revendications 1 à 16, **caractérisée en ce que** *gp* présente la séquence représentée à SEQ ID NO: 25.

18. Cellule rétrovirale d'encapsidation selon les revendications 8 à 13, **caractérisée en ce que** le rétrovirus est MLV qui n'exprime aucune protéine Env et LCMV est L(ARM) mutant défectueux.

19. Cellule rétrovirale d'encapsidation qui contient les gènes rétroviraux gag et *pol* **caractérisée en ce qu'**elle forme des virions pseudotypiques qui contiennent la glycoprotéine de LCMV dans leur enveloppe.

20. Virion rétroviral pseudotypé **caractérisé en ce qu'**il contient les glycoprotéines GP-1 et GP-2 de LCMV.

21. Vecteur pseudotype rétroviral **caractérisé en ce qu'**il contient les glycoprotéines GP-1 et GP-2 de LCMV.

22. Procédé pour la production d'une cellule rétrovirale d'encapsidation où l'on met en contact une cellule rétrovirale d'encapsidation qui contient les gènes rétroviraux *gag* et *pol*, dans certaines conditions, avec LCMV, qui permettent l'infection de la cellule rétrovirale d'encapsidation avec LCMV, et les virions produits par la cellule sont pseudotypés par les glycoprotéines de LCMV.

23. Procédé selon la revendication 22, **caractérisé en ce que** l'on utilise, en tant que cellule rétrovirale d'encapsidation, une cellule d'encapsidation qui contient le gène rétroviral env, et env est modifié de façon à coder pour une protéine env qui permet une liaison spécifique à la cellule cible et ainsi LCMV contient une variante de gp qui code pour une protéine GP qui présente une activité de fusion.

24. Procédé selon les revendications 22 et 23, **caractérisé en ce que** *gp* code pour la séquence représentée par SEQ ID NO: 26.

25. Procédé selon les revendications 22 à 24, **caractérisé en ce que** *gp* présente la séquence représentée à SEQ ID NO: 25.

26. Procédé selon les revendications 22 et 23, **caractérisé en ce qu'**on utilise, en tant que LCMV, le mutant délaissé L(ARM).

27. Procédé pour la production d'une cellule rétrovirale d'encapsidation selon les revendications 8 à 19, **caractérisé en ce que** l'on transfecte une cellules rétrovirales d'encapsidation transfectée par un ou plusieurs gènes étrangers avec un plasmide d'expression qui contient le gène gp de LCMV selon les revendications 1 à 3 et le cas échéant, de plus, un ou plusieurs gènes du groupe se composant de *np, 1* et *z* de LCMV.

28. Procédé selon la revendication 27, **caractérisé en ce que** l'on utilise, en tant que cellule rétrovirale d'encapsidation une cellule d'encapsidation qui contient le gène rétroviral *env* qui est modifié de façon à coder pour une protéine env qui permet une liaison spécifique à la cellule cible et où *gp* est une variante, qui code pour une protéine GP qui présente une activité de fusion.

29. Procédé selon les revendications 27 et 28 **caractérisé en ce que** *gp* code pour la séquence représentée dans SEQ ID NO: 26.

30. Procédé selon les revendications 27 à 29 **caractérisé en ce que** *gp* présente la séquence représentée à SEQ ID NO: 25.

31. Procédé pour la production de vecteurs pseudotypiques rétroviraux **caractérisé en ce que** l'on met en oeuvre un procédé selon les revendications 22 à 30 et ensuite, on cultive les cellules d'encapsidation dans des conditions qui sont appropriées à la production de virions.

32. Utilisation d'une cellule rétrovirale d'encapsidation selon les revendications 8 à 19 pour l'infection *in vitro* de cellules et pour l'expression du transgène dans ces cellules.

33. Utilisation d'une cellule rétrovirale d'encapsidation selon les revendications 8 à 19, formulée le cas échéant avec des auxiliaires et/ou supports pharmaceutiquement compatibles, pour la production d'une préparation pharmaceutique pour la thérapie génique.

34. Utilisation selon la revendication 32 et 33 pour la transfection *in vitro* de cellules souches hématopoïétiques.

35. Utilisation selon la revendication 33 et 34 où la thérapie génique comprend la thérapie de maladies infectieuses, néoplasie, mélanomes ainsi que d'autres maladies accessibles à la génothérapie.

36. Utilisation selon la revendication 35 **caractérisée en ce que** la maladie infectieuse est une infection de VIH ou bien le SIDA.

37. Utilisation selon la revendication 35, **caractérisée en ce que** la néoplasie est le carcinome mammaire.

38. Préparation pharmaceutique pour la thérapie génique, **caractérisée en ce qu'**elle contient des cellules rétrovirales d'encapsidation selon l'une quelconque des revendications 8 à 19 et le cas échéant un auxiliaire et/ou support phamaceutiquement acceptable.

39. Préparation pharmaceutique selon la revendication 38, **caractérisée en ce que** la thérapie génique comprend la thérapie de maladies infectieuses, néoplasies, mélanomes ainsi qu'autres maladies accessibles à la thérapie génique.

40. Préparation pharmaceutique selon la revendication 39, **caractérisée en ce que** la maladie infectieuse est une infection due à VIH ou bien le SIDA.

41. Préparation pharmaceutique selon la revendication 39, **caractérisée en ce que** la néoplasie est le carcinome mammaire.


### SEQUENZ PROTOKOLL

<110> Heinrich-Pette-Institut
<120> Retrovirale, mit LCMV pseudotypisierte Hybrid-Vektoren
<130> p050488
<140>
   <141>
<150> DE 198 56 463.5
   <151> 1998-11-26
<160> 26
<170> PatentIn Ver. 2.0
<210> 1
   <211> 3375
   <212> DNA
   <213> Lymphocytic choriomeningitis virus
<400> 1
   cgcaccgggg atcctaggct ttttggattg cgctttcctt taggacaact gggtgctgga 60
   ttctatccag taaaaggatg ggtcagattg tgacaatgtt tgaggctttg cctcacatca 120
   ttgatgaggt catcaacatt gtcattattg tgctcattat aatcacgagc atcaaagctg 180
   tgtacaattt cgccacctgt gggatattag cactggtcag cttccttttt ctggctggta 240
   ggtcctgtgg catgtacggc cttaatggtc ccgatatcta taaaggggtt taccagttca 300
   aatcagtgga gtttgatatg tctcacttaa atctgacgat gcccaatgcg tgctcagtca 360
   acaactctca tcactacatc agtatgggaa gctctggact ggagccaact ttcaccaacg 420
   actccatcct taatcacaac ttctgcaact taacctccgc tctcaacaaa aagtcttttg 480
   accatacact catgagtata gtctcgagtc tacacctcag tatcagaggg aattccaact 540
   acaaagcagt gtcttgtgat tttaacaatg gcatcaccat tcaatacaac ttgtcatctt 600
   cggacccaca gagcgccatg agccagtgta ggactttcag aggtagagtc ttggacatgt 660
   ttagaactgc ctttggagga aagtacatga gaagtggctg gggctggaca ggttcagatg 720
   gcaagaccac ttggtgcagc caaacaagct atcagtacct aatcatacaa aacaggactt 780
   gggaaaacca ctgtagatat gcaggccctt ttgggatgtc tagaatcctc tttgctcagg 840
   aaaagacaaa gtttctcact aggagacttt caggcacatt cacctggacc ctgtcagact 900
   cctcaggagt agaaaatcca ggtggttatt gcctgaccaa atggatgatc cttgctgcag 960
   agctcaaatg ttttgggaat acagctgttg caaaatgtaa tgtcaatcat gatgaagagt 1020
   tctgtgacat gctacgacta attgattaca acaaggctgc cctgagtaag ttcaagcaag 1080
   atgtagagtc tgccttgcat gtattcaaaa caacattaaa ttctctgatt tccgatcagc 1140
   tgttgatgag gaatcatcta agagatctaa tgggggtacc atactgtaat tactcaaagt 1200
   tctggtatct ggaacatgct aagactggtg agactagtgt acccaagtgt tggcttgtca 1260
   ctaatggctc ctacttgaat gagacccatt ttagtgatca aatcgaacaa gaagcagata 1320
   acatgatcac agagatgttg aggaaggact acataaaaag acaagggagt actcctttag 1380
   ccttaatgga tcttttgatg ttttcaacat cagcatactt gatcagcatc tttctgcatt 1440
   ttgtgaggat accaacacat agacacataa agggcggttc atgtccaaag ccacatcgct 1500
   tgaccaacaa ggggatctgt agttgtggtg cattcaaggt gcctggtgta aaaactatct 1560
   ggaaaagacg ctgatcagca gcgcctccct gactctccac ctcgaaagag gtggagagtc 1620
   agggaggccc agcgggtctt agagtgtcac aacattgggt cctctgaaga tcaaatcatg 1680
   tggcaggatg ttgtgaacgg tctttagatc agggagtctt gccttggaag cactctcaaa 1740
   gatgatgcag tccatgagtg cacagtgtgg ggtgatttct ttcttctttt tgtctctcac 1800
   taccccagtg tgcattttgc atagccagcc atatttgtcc cacactttat cttcatattc 1860
   tcttgaggcc tccttagtca tctcaacatc aatgagtttt atgtcccttc tattctgtga 1920
   gtccagaagc tttctgatgt catcagaacc ttgacagctc aagaccatcc cttgtgggag 1980
   agcacctata actgatgagg tcagcccagc ctgtgcattg aagaggtcag caagatccat 2040
   gccgtgtgaa tacttggagt cctgcttgaa ttgcttctgg tccgtaggtt ctctgtaaaa 2100
   atgtatgaat tgcccatttt gtggttgaaa tattgctatc tccactggat cattgaacct 2160
   gccttcaatg tcaatccatg tgggagcatt gggatcaatc cctcccatca agtctttcaa 2220
   cagcattgtt tgactgtaac tcaagcccac ctgaggtggg cctgctgctc caggcactgg 2280
   cctagatgag ttggccacaa gtttttcatt tgtgagatca attgtcgtgt tctcccatgc 2340
   tctccccaca actgacgttc tacaggctat gtatggccat ccttcacctg aaagacagac 2400
   tttataaagg atgttttcat aaggatttct atccccaact tgatctgaga caaacatgtt 2460
   gagtttcttc ttggccccaa ggactgcttt taggagatcc tcactattgc ttggtttgat 2520
   caaaatagat tccagcatgt tccctccatg tagcagagct gcccccgctt tcacagccgc 2580
   accaagactg aaattataac cagagatatt gatactagat tgctgttcag taatgacccc 2640
   cagaactggg tgtttatctt ttagcctttc taggtcactg agattcgggt atttgactgt 2700
   gtaaagtaag ccaaggtctg tgagtgcctg cacaacatca ttgagtgggg tctgtgactg 2760
   ttttgccatg caagccattg tcaggcttgg cattgtgccg aactgattgt tcagaagtga 2820
   tgagtccttc acatcccaaa cccttactac accacttgca ccctgctgag gtcttctcat 2880
   cccaaccatt tgcagtattt gggatctctg atcaagttgt tgtgctgtca aatttcccat 2940
   gtagactcca gaagcttgag gcctctcagt tctcataatt ttggccttca gcttctcaag 3000
   atcagctgca agggtcatca attcctctgc actaagtctt cccactttca gaacattttt 3060
   ctttgatgta gacttcggat caacaagaga atgcacagtc tggttaagac tcctgagtct 3120
   ctgcaagtct ttatcgtccc tcctttcctt tctcatgatc ctctgaacgt tgctgacttc 3180
   agaaaagtcc aacccattta gaagactggt tgcgtccttg atgacggcag cctttacatc 3240
   tgatgtaaaa ccctgcaact ccctcctcaa cgcctgtgtc cactgaaagc ttttgacttc 3300
   tttggacaaa gacattttgt cacacaatga atttccaaat aaaagcgcaa tcaaatgcct 3360
   aggatccact gtgcg 3375
<210> 2
   <211> 498
   <212> PRT
   <213> Lymphocytic choriomeningitis virus
<400> 2
   Met Gly Gln Ile Val Thr Met Phe Glu Ala Leu Pro His Ile Ile Asp
   1               5                   10                  15
   Glu Val Ile Asn Ile Val Ile Ile Val Leu Ile Ile Ile Thr Ser Ile
               20                  25                  30
   Lys Ala Val Tyr Asn Phe Ala Thr Cys Gly Ile Leu Ala Leu Val Ser
           35                  40                  45
   Phe Leu Phe Leu Ala Gly Arg Ser Cys Gly Met Tyr Gly Leu Asn Gly
       50                  55                  60
   Pro Asp Ile Tyr Lys Gly Val Tyr Gln Phe Lys Ser Val Glu Phe Asp
   65                  70                  75                  80
   Met Ser His Leu Asn Leu Thr Met Pro Asn Ala Cys Ser Val Asn Asn
                   85                  90                  95
   Ser His His Tyr Ile Ser Met Gly Ser Ser Gly Leu Glu Pro Thr Phe
               100                 105                 110
   Thr Asn Asp Ser Ile Leu Asn His Asn Phe Cys Asn Leu Thr Ser Ala
           115                 120                 125
   Leu Asn Lys Lys Ser Phe Asp His Thr Leu Met Ser Ile Val Ser Ser
       130                 135                 140
   Leu His Leu Ser Ile Arg Gly Asn Ser Asn Tyr Lys Ala Val Ser Cys
   145                 150                 155                 160
   Asp Phe Asn Asn Gly Ile Thr Ile Gln Tyr Asn Leu Ser Ser Ser Asp
                   165                 170                 175
   Pro Gln Ser Ala Met Ser Gln Cys Arg Thr Phe Arg Gly Arg Val Leu
               180                 185                 190
   Asp Met Phe Arg Thr Ala Phe Gly Gly Lys Tyr Met Arg Ser Gly Trp
           195                 200                 205
   Gly Trp Thr Gly Ser Asp Gly Lys Thr Thr Trp Cys Ser Gln Thr Ser
       210                 215                 220
   Tyr Gln Tyr Leu Ile Ile Gln Asn Arg Thr Trp Glu Asn His Cys Arg
   225                 230                 235                 240
   Tyr Ala Gly Pro Phe Gly Met Ser Arg Ile Leu Phe Ala Gln Glu Lys
                   245                 250                 255
   Thr Lys Phe Leu Thr Arg Arg Leu Ser Gly Thr Phe Thr Trp Thr Leu
               260                 265                 270
   Ser Asp Ser Ser Gly Val Glu Asn Pro Gly Gly Tyr Cys Leu Thr Lys
           275                 280                 285
   Trp Met Ile Leu Ala Ala Glu Leu Lys Cys Phe Gly Asn Thr Ala Val
       290                 295                 300
   Ala Lys Cys Asn Val Asn His Asp Glu Glu Phe Cys Asp Met Leu Arg
   305                 310                 315                 320
   Leu Ile Asp Tyr Asn Lys Ala Ala Leu Ser Lys Phe Lys Gln Asp Val
                   325                 330                 335
   Glu Ser Ala Leu His Val Phe Lys Thr Thr Leu Asn Ser Leu Ile Ser
               340                 345                 350
   Asp Gln Leu Leu Met Arg Asn His Leu Arg Asp Leu Met Gly Val Pro
           355                 360                 365
   Tyr Cys Asn Tyr Ser Lys Phe Trp Tyr Leu Glu His Ala Lys Thr Gly
       370                 375                 380
   Glu Thr Ser Val Pro Lys Cys Trp Leu Val Thr Asn Gly Ser Tyr Leu
   385                 390                 395                 400
   Asn Glu Thr His Phe Ser Asp Gln Ile Glu Gln Glu Ala Asp Asn Met
                   405                 410                 415
   Ile Thr Glu Met Leu Arg Lys Asp Tyr Ile Lys Arg Gln Gly Ser Thr
               420                 425                 430
   Pro Leu Ala Leu Met Asp Leu Leu Met Phe Ser Thr Ser Ala Tyr Leu
           435                 440                 445
   Ile Ser Ile Phe Leu His Phe Val Arg Ile Pro Thr His Arg His Ile
       450                 455                 460
   Lys Gly Gly Ser Cys Pro Lys Pro His Arg Leu Thr Asn Lys Gly Ile
   465                 470                 475                 480
   Cys Ser Cys Gly Ala Phe Lys Val Pro Gly Val Lys Thr Ile Trp Lys
                   485                 490                 495
   Arg Arg
<210> 3
   <211> 3376
   <212> DNA
   <213> Lymphocytic choriomeningitis virus
<400> 3
   cgcaccgggg atcctaggct ttttggattg cgctttcctc tagatcaact gggtgtcagg 60
   ccctatccta cagaaggatg ggtcagattg tgacaatgtt tgaggctctg cctcacatca 120
   tcgatgaggt gatcaacatt gtcattattg tgcttatcgt gatcacgggt atcaaggctg 180
   tctacaattt tgccacctgt gggatattcg cattgatcag tttcctactt ctggctggca 240
   ggtcctgtgg catgtacggt cttaagggac ccgacattta caaaggagtt taccaattta 300
   agtcagtgga gtttgatatg tcacatctga acctgaccat gcccaacgca tgttcagcca 360
   acaactccca ccattacatc agtatgggga cttctggact agaattgacc ttcaccaatg 420
   attccatcat cagtcacaac ttttgcaatc tgacctctgc cttcaacaaa aagacctttg 480
   accacacact catgagtata gtttcgagcc tacacctcag tatcagaggg aactccaact 540
   ataaggcagt atcctgcgac ttcaacaatg gcataaccat ccaatacaac ttgacattct 600
   cagatcgaca aagtgctcag agccagtgta gaaccttcag aggtagagtc ctagatatgt 660
   ttagaactgc cttcgggggg aaatacatga ggagtggctg gggctggaca ggctcagatg 720
   gcaagaccac ctggtgtagc cagacgagtt accaatacct gattatacaa aatagaacct 780
   gggaaaacca ctgcacatat gcaggtcctt ttgggatgtc caggattctc ctttcccaag 840
   agaagactaa gttcttcact aggagactag cgggcacatt cacctggact ttgtcagact 900
   cttcaggggt ggagaatcca ggtggttatt gcctgaccaa atggatgatt cttgctgcag 960
   agcttaagtg tttcgggaac acagcagttg cgaaatgcaa tgtaaatcat gatgccgaat 1020
   tctgtgacat gctgcgacta attgactaca acaaggctgc tttgagtaag ttcaaagagg 1080
   acgtagaatc tgccttgcac ttattcaaaa caacagtgaa ttctttgatt tcagatcaac 1140
   tactgatgag gaaccacttg agagatctga tgggggtgcc atattgcaat tactcaaagt 1200
   tttggtacct agaacatgca aagaccggcg aaactagtgt ccccaagtgc tggcttgtca 1260
   ccaatggttc ttacttaaat gagacccact tcagtgatca aatcgaacag gaagccgata 1320
   acatgattac agagatgttg aggaaggatt acataaagag gcaggggagt acccccctag 1380
   cattgatgga ccttctgatg ttttccacat ctgcatatct agtcagcatc ttcctgcacc 1440
   ttgtcaaaat accaacacac aggcacataa aaggtggctc atgtccaaag ccacaccgat 1500
   taaccaacaa aggaatttgt agttgtggtg catttaaggt gcctggtgta aaaaccgtct 1560
   ggaaaagacg ctgaagaaca gcgcctccct gactctccac ctcgaaagag gtggagagtc 1620
   agggaggccc agagggtctt agagtgtcac aacatttggg cctctaaaaa ttaggtcatg 1680
   tggcagaatg ttgtgaacag ttttcagatc tgggagcctt gctttggagg cgctttcaaa 1740
   aatgatgcag tccatgagtg cacagtgcgg ggtgatctct ttcttctttt tgtcccttac 1800
   tattccagta tgcatcttac acaaccagcc atatttgtcc cacactttgt cttcatactc 1860
   cctcgaagct tccctggtca tttcaacatc gataagctta atgtccttcc tattctgtga 1920
   gtccagaagc tttctgatgt catcggagcc ttgacagctt agaaccatcc cctgcggaag 1980
   agcacctata actgacgagg tcaacccggg ttgcgcattg aagaggtcgg caagatccat 2040
   gccgtgtgag tacttggaat cttgcttgaa ttgtttttga tcaacgggtt ccctgtaaaa 2100
   gtgtatgaac tgcccgttct gtggttggaa aattgctatt tccactggat cattaaatct 2160
   accctcaatg tcaatccatg taggagcgtt ggggtcaatt cctcccatga ggtcttttaa 2220
   aagcattgtc tggctgtagc ttaagcccac ctgaggtgga cctgctgctc caggcgctgg 2280
   cctgggtgaa ttgactgcag gtttctcgct tgtgagatca attgttgtgt tttcccatgc 2340
   tctccccaca atcgatgttc tacaagctat gtatggccat ccttcacctg aaaggcaaac 2400
   tttatagagg atgttttcat aagggttcct gtccccaact tggtctgaaa caaacatgtt 2460
   gagttttctc ttggccccga gaactgcctt caagaggtcc tcgctgttgc ttggcttgat 2520
   caaaattgac tctaacatgt tacccccatc caacagggct gcccctgcct tcacggcagc 2580
   accaagacta aagttatagc cagaaatgtt gatgctggac tgctgttcag tgatgacccc 2640
   cagaactggg tgcttgtctt tcagcctttc aagatcatta agatttggat acttgactgt 2700
   gtaaagcaag ccaaggtctg tgagcgcttg tacaacgtca ttgagcggag tctgtgactg 2760
   tttggccata caagccatag ttagacttgg cattgtgcca aattgattgt tcaaaagtga 2820
   tgagtctttc acatcccaaa ctcttaccac accacttgca ccctgctgag gctttctcat 2880
   cccaactatc tgtaggatct gagatctttg gtctagttgc tgtgttgtta agttccccat 2940
   atatacccct gaagcctggg gcctttcaga cctcatgatc ttggccttca gcttctcaag 3000
   gtcagccgca agagacatca gttcttctgc actgagcctc cccactttca aaacattctt 3060
   ctttgatgtt gactttaaat ccacaagaga atgtacagtc tggttgagac ttctgagtct 3120
   ctgtaggtct ttgtcatctc tcttttcctt cctcatgatc ctctgaacat tgctgacctc 3180
   agagaagtcc aacccattca gaaggttggt tgcatcctta atgacagcag ccttcacatc 3240
   tgatgtgaag ctctgcaatt ctcttctcaa tgcttgcgtc cattggaagc tcttaacttc 3300
   cttagacaag gacatcttgt tgctcaatgg tttctcaaga caaatgcgca atcaaatgcc 3360
   taggatccac tgtgcg 3376
<210> 4
   <211> 498
   <212> PRT
   <213> Lymphocytic choriomeningitis virus
<220>
   <223> Huell-Glycoprotein
<400> 4
   Met Gly Gln Ile Val Thr Met Phe Glu Ala Leu Pro His Ile Ile Asp
   1               5                   10                  15
   Glu Val Ile Asn Ile Val Ile Ile Val Leu Ile Val Ile Thr Gly Ile
               20                  25                  30
   Lys Ala Val Tyr Asn Phe Ala Thr Cys Gly Ile Phe Ala Leu Ile Ser
           35                  40                  45
   Phe Leu Leu Leu Ala Gly Arg Ser Cys Gly Met Tyr Gly Leu Lys Gly
       50                  55                  60
   Pro Asp Ile Tyr Lys Gly Val Tyr Gln Phe Lys Ser Val Glu Phe Asp
   65                  70                  75                  80
   Met Ser His Leu Asn Leu Thr Met Pro Asn Ala Cys Ser Ala Asn Asn
                   85                  90                  95
   Ser His His Tyr Ile Ser Met Gly Thr Ser Gly Leu Glu Leu Thr Phe
               100                 105                 110
   Thr Asn Asp Ser Ile Ile Ser His Asn Phe Cys Asn Leu Thr Ser Ala
           115                 120                 125
   Phe Asn Lys Lys Thr Phe Asp His Thr Leu Met Ser Ile Val Ser Ser
       130                 135                 140
   Leu His Leu Ser Ile Arg Gly Asn Ser Asn Tyr Lys Ala Val Ser Cys
   145                 150                 155                 160
   Asp Phe Asn Asn Gly Ile Thr Ile Gln Tyr Asn Leu Thr Phe Ser Asp
                   165                 170                 175
   Arg Gln Ser Ala Gln Ser Gln Cys Arg Thr Phe Arg Gly Arg Val Leu
               180                 185                 190
   Asp Met Phe Arg Thr Ala Phe Gly Gly Lys Tyr Met Arg Ser Gly Trp
           195                 200                 205
   Gly Trp Thr Gly Ser Asp Gly Lys Thr Thr Trp Cys Ser Gln Thr Ser
   210 215 220
   Tyr Gln Tyr Leu Ile Ile Gln Asn Arg Thr Trp Glu Asn His Cys Thr
   225 230 235 240
   Tyr Ala Gly Pro Phe Gly Met Ser Arg Ile Leu Leu Ser Gln Glu Lys
   245 250 255
   Thr Lys Phe Phe Thr Arg Arg Leu Ala Gly Thr Phe Thr Trp Thr Leu
   260 265 270
   Ser Asp Ser Ser Gly Val Glu Asn Pro Gly Gly Tyr Cys Leu Thr Lys
   275 280 285
   Trp Met Ile Leu Ala Ala Glu Leu Lys Cys Phe Gly Asn Thr Ala Val
   290 295 300
   Ala Lys Cys Asn Val Asn His Asp Ala Glu Phe Cys Asp Met Leu Arg
   305 310 315 320
   Leu Ile Asp Tyr Asn Lys Ala Ala Leu Ser Lys Phe Lys Glu Asp Val
   325 330 335
   Glu Ser Ala Leu His Leu Phe Lys Thr Thr Val Asn Ser Leu Ile Ser
   340 345 350
   Asp Gln Leu Leu Met Arg Asn His Leu Arg Asp Leu Met Gly Val Pro
   355 360 365
   Tyr Cys Asn Tyr Ser Lys Phe Trp Tyr Leu Glu His Ala Lys Thr Gly
   370 375 380
   Glu Thr Ser Val Pro Lys Cys Trp Leu Val Thr Asn Gly Ser Tyr Leu
   385 390 395 400
   Asn Glu Thr His Phe Ser Asp Gln Ile Glu Gln Glu Ala Asp Asn Met
   405 410 415
   Ile Thr Glu Met Leu Arg Lys Asp Tyr Ile Lys Arg Gln Gly Ser Thr
   420 425 430
   Pro Leu Ala Leu Met Asp Leu Leu Met Phe Ser Thr Ser Ala Tyr Leu
   435 440 445
   Val Ser Ile Phe Leu His Leu Val Lys Ile Pro Thr His Arg His Ile
   450 455 460
   Lys Gly Gly Ser Cys Pro Lys Pro His Arg Leu Thr Asn Lys Gly Ile
   465 470 475 480
   Cys Ser Cys Gly Ala Phe Lys Val Pro Gly Val Lys Thr Val Trp Lys
   485 490 495
   Arg Arg
<210> 5
   <211> 558
   <212> PRT
   <213> Lymphocytic choriomeningitis virus
<220>
   <223> Nucleoprotein
<400> 5
   Met Ser Leu Ser Lys Glu Val Lys Ser Phe Gln Trp Thr Gln Ala Leu
    1               5                  10                  15
   Arg Arg Glu Leu Gln Ser Phe Thr Ser Asp Val Lys Ala Ala Val Ile
               20                  25                  30
   Lys Asp Ala Thr Asn Leu Leu Asn Gly Leu Asp Phe Ser Glu Val Ser
           35                  40                  45
   Asn Val Gln Arg Ile Met Arg Lys Glu Lys Arg Asp Asp Lys Asp Leu
       50                  55                  60
   Gln Arg Leu Arg Ser Leu Asn Gln Thr Val His Ser Leu Val Asp Leu
   65                  70                  75                  80
   Lys Ser Thr Ser Lys Lys Asn Val Leu Lys Val Gly Arg Leu Ser Ala
                   85                  90                  95
   Glu Glu Leu Met Ser Leu Ala Ala Asp Leu Glu Lys Leu Lys Ala Lys
               100                 105                 110
   Ile Met Arg Ser Glu Arg Pro Gln Ala Ser Gly Val Tyr Met Gly Asn
           115                 120                 125
   Leu Thr Thr Gln Gln Leu Asp Gln Arg Ser Gln Ile Leu Gln Ile Val
       130                 135                 140
   Gly Met Arg Lys Pro Gln Gln Gly Ala Ser Gly Val Val Arg Val Trp
   145                 150                 155                 160
   Asp Val Lys Asp Ser Ser Leu Leu Asn Asn Gln Phe Gly Thr Met Pro
                   165                 170                 175
   Ser Leu Thr Met Ala Cys Met Ala Lys Gln Ser Gln Thr Pro Leu Asn
               180                 185                 190
   Asp Val Val Gln Ala Leu Thr Asp Leu Gly Leu Leu Tyr Thr Val Lys
           195                 200                 205
   Tyr Pro Asn Leu Asn Asp Leu Glu Arg Leu Lys Asp Lys His Pro Val
       210                 215                 220
   Leu Gly Val Ile Thr Glu Gln Gln Ser Ser Ile Asn Ile Ser Gly Tyr
   225                 230                 235                 240
   Asn Phe Ser Leu Gly Ala Ala Val Lys Ala Gly Ala Ala Leu Leu Asp
                   245                 250                 255
   Gly Gly Asn Met Leu Glu Ser Ile Leu Ile Lys Pro Ser Asn Ser Glu
               260                 265                 270
   Asp Leu Leu Lys Ala Val Leu Gly Ala Lys Arg Lys Leu Asn Met Phe
           275                 280                 285
   Val Ser Asp Gln Val Gly Asp Arg Asn Pro Tyr Glu Asn Ile Leu Tyr
       290                 295                 300
   Lys Val Cys Leu Ser Gly Glu Gly Trp Pro Tyr Ile Ala Cys Arg Thr
   305                 310                 315                 320
   Ser Ile Val Gly Arg Ala Trp Glu Asn Thr Thr Ile Asp Leu Thr Ser
                   325                 330                 335
   Glu Lys Pro Ala Val Asn Ser Pro Arg Pro Ala Pro Gly Ala Ala Gly
               340                 345                 350
   Pro Pro Gln Val Gly Leu Ser Tyr Ser Gln Thr Met Leu Leu Lys Asp
           355                 360                 365
   Leu Met Gly Gly Ile Asp Pro Asn Ala Pro Thr Trp Ile Asp Ile Glu
       370                 375                 380
   Gly Arg Phe Asn Asp Pro Val Glu Ile Ala Ile Phe Gln Pro Gln Asn
   385                 390                 395                 400
   Gly Gln Phe Ile His Phe Tyr Arg Glu Pro Val Asp Gln Lys Gln Phe
                   405                 410                 415
   Lys Gln Asp Ser Lys Tyr Ser His Gly Met Asp Leu Ala Asp Leu Phe
               420                 425                 430
   Asn Ala Gln Pro Gly Leu Thr Ser Ser Val Ile Gly Ala Leu Pro Gln
           435                 440                 445
   Gly Met Val Leu Ser Cys Gln Gly Ser Asp Asp Ile Arg Lys Leu Leu
       450                 455                 460
   Asp Ser Gln Asn Arg Lys Asp Ile Lys Leu Ile Asp Val Glu Met Thr
   465                 470                 475                 480
   Arg Glu Ala Ser Arg Glu Tyr Glu Asp Lys Val Trp Asp Lys Tyr Gly
                   485                 490                 495
   Trp Leu Cys Lys Met His Thr Gly Ile Val Arg Asp Lys Lys Lys Lys
               500                 505                 510
   Glu Ile Thr Pro His Cys Ala Leu Met Asp Cys Ile Ile Phe Glu Ser
           515                 520                 525
   Ala Ser Lys Ala Arg Leu Pro Asp Leu Lys Thr Val His Asn Ile Leu
       530                 535                 540
   Pro His Asp Leu Ile Phe Arg Gly Pro Asn Val Val Thr Leu
   545                 550                 555
<210> 6
   <211> 6680
   <212> DNA
   <213> Lymphocytic choriomeningitis virus
<400> 6
   cgcaccgagg atcctaggct ttttgatgcg caatggatga aatcatctca gaattgagag 60
   agttatgttt aaactatata gaacaggatg agaggttgtc aaggcagaaa ctcaactttc 120
   tgggacaaag ggaacccaga atggttctga ttgagggact caagttgctg tcacgctgca 180
   ttgaaataga cagtgcagac aagagtggct gcacacacaa ccacgacgat aagtctgtgg 240
   aaacaatttt ggtggagtct ggaattgtat gcccaggact accacttatc attcctgatg 300
   gttacaagct gatagacaat tctctcattc ttcttgagtg ttttgttagg agctcaccag 360
   ccagttttga gaagaaattt atagaggaca ctaacaaatt ggcatgcatc agggaagacc 420
   ttgctgttgc gggtgtcaca ttagttccaa tagtagatgg tcgttgtgat tatgataata 480
   gttttatgcc agagtgggca aacttcaaat ttagagacct tttattcaaa cttttggagt 540
   attctaacca aaatgagaaa gtctttgaag agtctgaata ttttagactc tgtgagtccc 600
   tgaagactac tatcgacaag cgctccggta tggactctat gaaaattctg aaagatgcga 660
   ggtcaactca caatgatgaa attatgagga tgtgccacga aggcatcaac cccaacatga 720
   gctgtgatga tgtggttttt ggaataaact ctcttttcag caggtttaga agagatttag 780
   aaagtgggaa attaaagaga aactttcaga aagtaaaccc tgaaggcttg atcaaggaat 840
   tctctgagct ctatgaaaac cttgctgata gtgatgatat cttaacatta agcagggagg 900
   cagtcgaatc ctgtcctttg atgagattca taactgcaga gacccatggg cacgaaaggg 960
   gaagtgagac tagcactgaa tatgagaggc tcctctctat gttaaacaaa gtcaagagtt 1020
   tgaaactgtt gaatactaga aggagacagt tgttaaatct ggatgttttg tgtctttcct 1080
   cattgataaa acagtcgaaa ttcaaagggt taaaaaatga taaacactgg gtgggttgtt 1140
   gctatagtag tgtgaatgat aggctggtaa gctttcacag cactaaagag gagttcatta 1200
   gacttttgag gaatagaaaa aagtcaaagg tgtttagaaa ggtgtctttt gaggaattgt 1260
   ttagggcgtc tattagtgag ttcattgcaa aaattcaaaa atgcctgtta gtggtgggac 1320
   tgagtttcga gcattacgga ctgtctgaac accttgagca agaatgccac ataccattca 1380
   ctgaatttga gaactttatg aaaattggag ctcacccgat aatgtattat acgaagtttg 1440
   aagattacaa tttccaaccc agcacagagc agctgaagaa catacagagc ctgagaagat 1500
   tatcatctgt ttgtctggcc ttaacaaaca gtatgaaaac tagctcagtt gctagactaa 1560
   ggcaaaatca aatagggtct gtgagatatc aagtggtaga atgcaaagaa gtgttttgcc 1620
   aagtaataaa actggactct gaagaatacc acctattata ccagaagact ggagaatctt 1680
   caaggtgcta ctccatacaa ggcccggatg gtcatttaat ttccttctat gcagatccta 1740
   aaaggttctt tttaccaatt ttttcagatg aggtcttata caatatgata gacatcatga 1800
   tttcatggat tagatcatgt cctgatttga aagactgtct caccgacatt gaggttgcac 1860
   tgaggaccct attgttgcta atgctcacca acccaacaaa gagaaatcaa aagcaggtac 1920
   agagtgtcag atatttggtg atggcaatag tgtcagattt ttcatctaca tcattaatgg 1980
   ataagttgag ggaggatctg atcacacctg ctgagaaggt ggtgtataag ctgcttagat 2040
   tcctaataaa aactattttt ggtactggtg agaaggtgtt gttgagtgca aaatttaaat 2100
   ttatgttgaa tgtgtcatac ctgtgtcatt tgatcacaaa ggagacccct gacaggctaa 2160
   cagatcagat aaaatgtttt gaaaagttct ttgagcccaa aagtcaattt ggtttttttg 2220
   tcaaccccaa ggaagcaatc actcctgagg aagaatgtgt gttctatgag caaatgaaga 2280
   gattcactag taaagaaatt gactgtcagc atacaactcc aggtgttaat ctggaagcct 2340
   ttagcctaat ggtgtcttca tttaacaacg gcactttaat tttcaaagga gagaagaagc 2400
   taaacagcct agatcccatg actaactctg gatgtgcgac agcattagat cttgctagta 2460
   acaaaagtgt ggtggttaat aagcatctaa atggagaacg acttctggaa tatgacttta 2520
   acaaattgct tgttagtgct gtgagtcaaa ttacggagag tttcgtaaga aaacaaaagt 2580
   ataagttgag ccactcagac tatgaatata aagtttccaa gttagtctct agattggtca 2640
   tcggttccaa gggagaagag acagggagat cggaagacaa cctggcagaa atatgttttg 2700
   atggagaaga agagacaagc ttcttcaaaa gtctcgaaga aaaggtcaac accacaatag 2760
   cacggtacag aagaggtagg agggccaatg acaaaggaga tggagaaaaa cttacaaata 2820
   caaaaggact acatcattta cagcttattc taacagggaa gatggctcac ttaagaaaag 2880
   ttatcttgtc agaaatatct ttccatttag tagaagactt tgacccatca tgtctaacca 2940
   atgatgacat gaaatttatc tgtgaggctg ttgagggttc cacagagctg tcacctttgt 3000
   atttcacctc agtcattaaa gatcagtgtg gcctcgatga gatggcaaaa aacctttgta 3060
   gaaagttctt ttctgagaat gattggtttt cttgcatgaa gatgattctg ttgcaaatga 3120
   atgcaaatgc gtactcaggg aaatacaggc atatgcaaag gcaaggcttg aatttcaaat 3180
   ttgactggga caaactggaa gaagacgtga gaatcagtga gagggaaagt aattctgagt 3240
   cccttagtaa agctctgtcg ttgacaaaat gtatgagtgc tgctttgaaa aatctgtgct 3300
   tctactcaga agaatcacca acatcataca cctcagtagg tcctgactct ggaaggctga 3360
   aatttgcact atcttataaa gagcaggttg gggaaatagg agaactctat attggagatt 3420
   tgaggacaaa aatgttcaca aggttaatag aagattattt tgagtctttt tcaagtttct 3480
   tttcaggctc ctgtttaaac aatgataagg aatttgaaaa tgcaatcttg tcaatgacta 3540
   tcaatgtgcg ggaagggttc ttaaactata gtatggatca cagcaaatgg ggaccaatga 3600
   tgtgcccatt tttgttctta atgtttctac aaaatctcaa actaggtgat gaccagtatg 3660
   tgcgttccgg gaaagatcat gttagcactt tgttaacttg gcacatgcat aagcttgtcg 3720
   aggtcccctt tcctgttgtg aatgcaatga tgaaatcata tgtcaagtcg aagctaaaac 3780
   ttctcagggg ttcagaaaca actgttactg agagaatttt cagacaatat tttgaaatgg 3840
   ggatagtgcc atcccatata tccagcctta ttgatatggg gcagggaatc ttgcataatg 3900
   cttctgactt ctatggtttg cttagcgaga ggttcatcaa ctactgcatt ggtgttatct 3960
   ttggcgaaag accagaggct tacacatcaa gtgatgatca gatcacttta tttgatagga 4020
   ggctgagtga cctggttgta agtgatccgg aggaagtcct tgtcctgttg gaattccaat 4080
   ctcatctgag cggcttgtta aacaaattta tcagcccaaa aagtgtggct gggaggttcg 4140
   ctgcagaatt taaatctaga ttctatgtat ggggggagga agtccctctt ctcacaaagt 4200
   ttgtatctgc agcgctacac aatgtcaagt gtaaagagcc acatcaactt tgtgaaacaa 4260
   tagatacaat tgcagatcaa gccatcgcaa atggcgtccc agtctcccta gttaatagta 4320
   tccaaaggag aacactggac ctcctaaagt atgccaattt ccctttggat ccatttctac 4380
   tgaataccaa cactgatgtg aaagattggc tggatggttc tagaggttac agaatacaaa 4440
   gactcattga ggaactgtgt cctaatgaaa caaaggttgt aagaaagctt gtaaggaaac 4500
   tgcatcataa gctcaaaaat ggtgaattta atgaagaatt tttcttagac ctatttaaca 4560
   gagataaaac ggaggccatt cttcaattgg gagacctcct cggtcttgaa gaagatctga 4620
   atcagttagc agatgttaac tggttgaatt tgaatgaaat gttcccatta aggatggttt 4680
   taagacaaaa ggtggtttat ccatcagtga tgactttcca agaggaaaga atcccatcat 4740
   tgatcaagac actccagaac aaactttgta gtaaattcac aaggggtgca cagaagctgc 4800
   tgtcagaagc aatcaacaag tcagctttcc agagttgtat ctcatctggc tttataggcc 4860
   tttgcaaaac tctaggaagc aggtgtgtga gaaacaaaaa tagggaaaat ctgtatatca 4920
   aaaagctgct tgaggatcta accacagatg atcatgtgac aagagtttgc aatcgggatg 4980
   gtataacgct gtacatttgt gacaaacagt ctcatccaga agcccaccgt gatcatatat 5040
   gccttttaag gcctcttctt tgggactaca tttgtatttc attgagcaac tcttttgagt 5100
   tgggtgtttg ggtcctagca gaaccgacca aagggaagaa taacagtgag aacctaactc 5160
   ttaagcactt aaacccatgt gattatgtag caagaaagcc tgagagctca aggctactgg 5220
   aggacaaagt gaatttgaac caagtgattc aatctgtgag gcggctatat cccaagatct 5280
   ttgaggatca gcttcttcca tttatgtctg acatgagctc aaaaaacatg aggtggagtc 5340
   ccagaattaa attccttgac ctctgtgttt taattgatat taactcagaa tccttgtcac 5400
   tcatttctca tgttgttaag tggaaaaggg atgaacatta cactgttctg ttttctgacc 5460
   ttgccaattc tcatcagcga tctgactcca gtctggttga tgaatttgtt gttagcacga 5520
   gggatgtctg caagaacttc ttaaaacagg tgtattttga atcatttgtt cgagaatttg 5580
   ttgcaacaac caggacatta ggcaattttt catggttccc tcataaagaa atgatgccat 5640
   ctgaagatgg tgctgaggca ctgggcccct ttcaatcatt tgtctcaaag gtggtgaaca 5700
   aaaatgtgga gaggcctatg tttaggaatg atttgcagtt tggttttggg tggttctctt 5760
   accgaatggg agatgttgtg tgtaatgctg ccatgttgat taggcagggc ctgacaaacc 5820
   caaaggcatt taaatcctta aaggatctgt gggactacat gctcaactac acaaaagggg 5880
   tattggagtt ttcaatttca gtggacttta cgcacaatca gaataatact gactgtttaa 5940
   ggaaattttc attgatattc ttggttaggt gccaattaca gaatccaggt gtggctgaac 6000
   ttttatcatg ctctcacctc tttaagggtg agatagatag aagaatgttg gatgaatgcc 6060
   tccacttact gaggacagac tctgtcttca aggtgaacga tggtgtcttt gatatcagat 6120
   ctgaagagtt tgaggattac atggaagatc ccttgatact tggtgattct cttgagcttg 6180
   agttgttggg ctccaaaaga atactggatg ggattagatc tattgacttt gagagagttg 6240
   gacctgagtg ggagcctgtg ccactgactg taaagatggg tgcccttttt gaaggaagaa 6300
   accttgtcca aaatatcatt gtgaagctgg agaccaagga catgaaagtc tttctagcag 6360
   gacttgaggg ctatgaaaag attagtgatg tccttgggaa cctcttcctg catcgattca 6420
   gaactggtga acatttgttg ggttcagaga taagtgtaat cctccaggaa ctatgtatag 6480
   acagatctat tctgctgatt ccactgtcgc ttttgccaga ctggttcgcc tttaaggatt 6540
   gcagactttg ttttagcaaa tctaggagca ctttgatgta tgaaatagtg gggggcaggt 6600
   ttagactcaa ggggaggtcc tgcgacgatt ggctaggcgg gtcggtggcc gaggacatcg 6660
   actgatgggc atctcctggg 6680
<210> 7
   <211> 2210
   <212> PRT
   <213> Lymphocytic choriomeningitis virus
<220>
   <223> L-Protein
<210> 8
   <211> 95
   <212> PRT
   <213> Lymphocytic choriomeningitis virus
<220>
   <223> Unbekanntes Protein
<400> 8
   Met Ser Ser Ala Thr Asp Pro Pro Ser Gln Ser Ser Gln Asp Leu Pro
   1               5                   10                  15
   Leu Ser Leu Asn Leu Pro Pro Thr Ile Ser Tyr Ile Lys Val Leu Leu
               20                  25                  30
   Asp Leu Leu Lys Gln Ser Leu Gln Ser Leu Lys Ala Asn Gln Ser Gly
           35                  40                  45
   Lys Ser Asp Ser Gly Ile Ser Arg Ile Asp Leu Ser Ile His Ser Ser
       50                  55                  60
   Trp Arg Ile Thr Leu Ile Ser Glu Pro Asn Lys Cys Ser Pro Val Leu
   65                  70                  75                  80
   Asn Arg Cys Arg Lys Arg Phe Pro Arg Thr Ser Leu Ile Phe Ser
                   85                  90                  95
<210> 9
   <211> 4695
   <212> DNA
   <213> Homo sapiens
<400> 9
   cccgggctgg gctgagaccc gcagaggaag acgctctagg gatttgtccc ggactagcga 60
   gatggcaagg ctgaggacgg gaggctgatt gagaggcgaa ggtacaccct aatctcaata 120
   caacctttgg agctaagcca gcaatggtag agggaagatt ctgcacgtcc cttccaggcg 180
   gcctccccgt caccaccccc cccaacccgc cccgaccgga gctgagagta attcatacaa 240
   aaggactcgc ccctgccttg gggaatccca gggaccgtcg ttaaactccc actaacgtag 300
   aacccagaga tcgctgcgtt cccgccccct cacccgcccg ctctcgtcat cactgaggtg 360
   gagaagagca tgcgtgaggc tccggtgccc gtcagtgggc agagcgcaca tcgcccacag 420
   tccccgagaa gttgggggga ggggtcggca attgaaccgg tgcctagaga aggtggcgcg 480
   gggtaaactg ggaaagtgat gtcgtgtact ggctccgcct ttttcccgag ggtgggggag 540
   aaccgtatat aagtgcagta gtcgccgtga acgttctttt tcgcaacggg tttgccgcca 600
   gaacacaggt aagtgccgtg tgtggttccc gcgggcctgg cctctttacg ggttatggcc 660
   cttgcgtgcc ttgaattact tccacgcccc tggctgcagt acgtgattct tgatcccgag 720
   cttcgggttg gaagtgggtg ggagagttcg aggccttgcg cttaaggagc cccttcgcct 780
   cgtgcttgag ttgaggcctg gcctgggcgc tggggccgcc gcgtgcgaat ctggtggcac 840
   cttcgcgcct gtctcgctgc tttcgataag tctctagcca tttaaaattt ttgatgacct 900
   gctgcgacgc tttttttctg gcaagatagt cttgtaaatg cgggccaaga tctgcacact 960
   ggtatttcgg tttttggggc cgcgggcggc gacggggccc gtgcgtccca gcgcacatgt 1020
   tcggcgaggc ggggcctgcg agcgcggcca ccgagaatcg gacgggggta gtctcaagct 1080
   ggccggcctg ctctggtgcc tggcctcgcg ccgccgtgta tcgccccgcc ctgggcggca 1140
   aggctggccc ggtcggcacc agttgcgtga gcggaaagat ggccgcttcc cggccctgct 1200
   gcagggagct caaaatggag gacgcggcgc tcgggagagc gggcgggtga gtcacccaca 1260
   caaaggaaaa gggcctttcc gtcctcagcc gtcgcttcat gtgactccac ggagtaccgg 1320
   gcgccgtcca ggcacctcga ttagttctcg agcttttgga gtacgtcgtc tttaggttgg 1380
   ggggaggggt tttatgcgat ggagtttccc cacactgagt gggtggagac tgaagttagg 1440
   ccagcttggc acttgatgta attctccttg gaatttgccc tttttgagtt tggatcttgg 1500
   ttcattctca agcctcagac agtggttcaa agtttttttc ttccatttca ggtgtcgtga 1560
   aaactacccc taaaagccaa aatgggaaag gaaaagactc atatcaacat tgtcgtcatt 1620
   ggacacgtag attcgggcaa gtccaccact actggccatc tgatctataa atgcggtggc 1680
   atcgacaaaa gaaccattga aaaatttgag aaggaggctg ctgaggtatg tttaatacca 1740
   gaaagggaaa gatcaactaa aatgagtttt accagcagaa tcattaggtg atttccccag 1800
   aactagtgag tggtttagat ctgaatgcta atagttaaga ccttacttat gaaataattt 1860
   tgcttttggt gacttctgta atcgtattgc tagtgagtag atttggatgt taatagttaa 1920
   gatcctactt ataaaagttt gatttttggt tgcttctgta acccaaagtg accaaaatca 1980
   ctttggactt ggagttgtaa agtggaaact gccaattaag ggctggggac aaggaaattg 2040
   aagctggagt ttgtgtttta gtaaccaagt aacgactctt aatccttaca gatgggaaag 2100
   ggctccttca agtatgcctg ggtcttggat aaactgaaag ctgagcgtga acgtggtatc 2160
   accattgata tctccttgtg gaaatttgag accagcaagt actatgtgac tatcattgat 2220
   gccccaggac acagagactt tatcaaaaac atgattacag ggacatctca ggttgggatt 2280
   aataattcta ggtttcttta tcccaaaagg cttgctttgt acactggttt tgtcatttgg 2340
   agagttgaca gggatatgtc tttgctttct ttaaaggctg actgtgctgt cctgattgtt 2400
   gctgctggtg ttggtgaatt tgaagctggt atctccaaga atgggcagac ccgagagcat 2460
   gcccttctgg cttacacact gggtgtgaaa caactaattg tcggtgttaa caaaatggat 2520
   tccactgagc caccctacag ccagaagaga tatgaggaaa ttgttaagga agtcagcact 2580
   tacattaaga aaattggcta caaccccgac acagtagcat ttgtgccaat ttctggttgg 2640
   aatggtgaca acatgctgga gccaagtgct aacgtaagtg gctttcaaga ccattgttaa 2700
   aaagctctgg gaatggcgat ttcatgctta cacaaattgg catgcttgtg tttcagatgc 2760
   cttggttcaa gggatggaaa gtcacccgta aggatggcaa tgccagtgga accacgctgc 2820
   ttgaggctct ggactgcatc ctaccaccaa ctcgtccaac tgacaagccc ttgcgcctgc 2880
   ctctccagga tgtctacaaa attggtggta agttggctgt aaacaaagtt gaatttgagt 2940
   tgatagagta ctgtctgcct tcataggtat ttagtatgct gtaaatattt ttaggtattg 3000
   gtactgttcc tgttggccga gtggagactg gtgttctcaa acccggtatg gtggtcacct 3060
   ttgctccagt caacgttaca acggaagtaa aatctgtcga aatgcaccat gaagctttga 3120
   gtgaagctct tcctggggac aatgtgggct tcaatgtcaa gaatgtgtct gtcaaggatg 3180
   ttcgtcgtgg caacgttgct ggtgacagca aaaatgaccc accaatggaa gcagctggct 3240
   tcactgctca ggtaacaatt taaagtaaca ttaacttatt gcagaggcta aagtcatttg 3300
   agactttgga tttgcactga atgcaaatct tttttccaag gtgattatcc tgaaccatcc 3360
   aggccaaata agcgccggct atgcccctgt attggattgc cacacggctc acattgcatg 3420
   caagtttgct gagctgaagg aaaagattga tcgccgttct ggtaaaaagc tggaagatgg 3480
   ccctaaattc ttgaagtctg gtgatgctgc cattgttgat atggttcctg gcaagcccat 3540
   gtgtgttgag agcttctcag actatccacc tttgggtaag gatgactact taaatgtaaa 3600
   aaagttgtgt taaagatgaa aaatacaact gaacagtact ttgggtaata attaactttt 3660
   tttttaatag gtcgctttgc tgttcgtgat atgagacaga cagttgcggt gggtgtcatc 3720
   aaagcagtgg acaagaaggc tgctggagct ggcaaggtca ccaagtctgc ccagaaagct 3780
   cagaaggcta aatgaatatt atccctaata cctgccaccc cactcttaat cagtggtgga 3840
   agaacggtct cagaactgtt tgtttcaatt ggccatttaa gtttagtagt aaaagactgg 3900
   ttaatgataa caatgcatcg taaaaccttc agaaggaaag gagaatgttt tgtggaccac 3960
   tttggttttc ttttttgcgt gtggcagttt taagttatta gtttttaaaa tcagtacttt 4020
   ttaatggaaa caacttgacc aaaaatttgt cacagaattt tgagacccat taaaaaagtt 4080
   aaatgagaaa cctgtgtgtt cctttggtca acaccgagac atttaggtga aagacatcta 4140
   attctggttt tacgaatctg gaaacttctt gaaaatgtaa ttcttgagtt aacacttctg 4200
   ggtggagaat agggttgttt tccccccaca taattggaag gggaaggaat atcatttaaa 4260
   gctatgggag ggtttctttg attacaacac tggagagaaa tgcagcatgt tgctgattgc 4320
   ctgtcactaa aacaggccaa aaactgagtc cttgggttgc atagaaagct tcatgttgct 4380
   aaaccaatgt taagtgaatc tttggaaaca aaatgtttcc aaattactgg gatgtgcatg 4440
   ttgaaacgtg ggttaaaatg actgggcagt gaaagttgac tatttgccat gacataagaa 4500
   ataagtgtag tggctagtgt acaccctatg agtggaaggg tccattttga agtcagtgga 4560
   gtaagcttta tgccattttg atggtttcac aagttctatt gagtgctatt cagaatagga 4620
   acaaggttct aatagaaaaa gatggcaatt tgaagtagct ataaaattag actaattaca 4680
   ttgcttttct ccgac                                                  4695
<210> 10
   <211> 462
   <212> PRT
   <213> Homo sapiens
<220>
   <223> Elongationsfaktor EF-1-alpha
<400> 10
   Met Gly Lys Glu Lys Thr His Ile Asn Ile Val Val Ile Gly His Val
   1               5                   10                  15
   Asp Ser Gly Lys Ser Thr Thr Thr Gly His Leu Ile Tyr Lys Cys Gly
               20                  25                  30
   Gly Ile Asp Lys Arg Thr Ile Glu Lys Phe Glu Lys Glu Ala Ala Glu
           35                  40                  45
   Met Gly Lys Gly Ser Phe Lys Tyr Ala Trp Val Leu Asp Lys Leu Lys
       50                  55                  60
   Ala Glu Arg Glu Arg Gly Ile Thr Ile Asp Ile Ser Leu Trp Lys Phe
   65                  70                  75                  80
   Glu Thr Ser Lys Tyr Tyr Val Thr Ile Ile Asp Ala Pro Gly His Arg
                   85                  90                  95
   Asp Phe Ile Lys Asn Met Ile Thr Gly Thr Ser Gln Ala Asp Cys Ala
               100                 105                 110
   Val Leu Ile Val Ala Ala Gly Val Gly Glu Phe Glu Ala Gly Ile Ser
           115                 120                 125
   Lys Asn Gly Gln Thr Arg Glu His Ala Leu Leu Ala Tyr Thr Leu Gly
       130                 135                 140
   Val Lys Gln Leu Ile Val Gly Val Asn Lys Met Asp Ser Thr Glu Pro
   145                 150                 155                 160
   Pro Tyr Ser Gln Lys Arg Tyr Glu Glu Ile Val Lys Glu Val Ser Thr
                   165                 170                 175
   Tyr Ile Lys Lys Ile Gly Tyr Asn Pro Asp Thr Val Ala Phe Val Pro
               180                 185                 190
   Ile Ser Gly Trp Asn Gly Asp Asn Met Leu Glu Pro Ser Ala Asn Met
           195                 200                 205
   Pro Trp Phe Lys Gly Trp Lys Val Thr Arg Lys Asp Gly Asn Ala Ser
       210                 215                 220
   Gly Thr Thr Leu Leu Glu Ala Leu Asp Cys Ile Leu Pro Pro Thr Arg
   225                 230                 235                 240
   Pro Thr Asp Lys Pro Leu Arg Leu Pro Leu Gln Asp Val Tyr Lys Ile
                   245                 250                 255
   Gly Gly Ile Gly Thr Val Pro Val Gly Arg Val Glu Thr Gly Val Leu
               260                 265                 270
   Lys Pro Gly Met Val Val Thr Phe Ala Pro Val Asn Val Thr Thr Glu
           275                 280                 285
   Val Lys Ser Val Glu Met His His Glu Ala Leu Ser Glu Ala Leu Pro
       290                 295                 300
   Gly Asp Asn Val Gly Phe Asn Val Lys Asn Val Ser Val Lys Asp Val
   305                 310                 315                 320
   Arg Arg Gly Asn Val Ala Gly Asp Ser Lys Asn Asp Pro Pro Met Glu
                   325                 330                 335
   Ala Ala Gly Phe Thr Ala Gln Val Ile Ile Leu Asn His Pro Gly Gln
               340                 345                 350
   Ile Ser Ala Gly Tyr Ala Pro Val Leu Asp Cys His Thr Ala His Ile
           355                 360                 365
   Ala Cys Lys Phe Ala Glu Leu Lys Glu Lys Ile Asp Arg Arg Ser Gly
       370                 375                 380
   Lys Lys Leu Glu Asp Gly Pro Lys Phe Leu Lys Ser Gly Asp Ala Ala
   385                 390                 395                 400
   Ile Val Asp Met Val Pro Gly Lys Pro Met Cys Val Glu Ser Phe Ser
                   405                 410                 415
   Asp Tyr Pro Pro Leu Gly Arg Phe Ala Val Arg Asp Met Arg Gln Thr
               420                 425                 430
   Val Ala Val Gly Val Ile Lys Ala Val Asp Lys Lys Ala Ala Gly Ala
           435                 440                 445
   Gly Lys Val Thr Lys Ser Ala Gln Lys Ala Gln Lys Ala Lys
       450                 455                 460
<210> 11
   <211> 8332
   <212> DNA
   <213> Moloney murine leukemia virus
<400> 11
   tatgcgcctg cgtcggtact agttagctaa ctagctctgt atctggcgga cccgtggtgg 60
   aactgacgag ttcggaacac ccggccgcaa ccctgggaga cgtcccaggg acttcggggg 120
   ccgtttttgt ggcccgacct gagtccaaaa atcccgatcg ttttggactc tttggtgcac 180
   cccccttaga ggagggatat gtggttctgg taggagacga gaacctaaaa cagttcccgc 240
   ctccgtctga atttttgctt tcggtttggg accgaagccg cgccgcgcgt cttgtctgct 300
   gcagcatcgt tctgtgttgt ctctgtctga ctgtgtttct gtatttgtct gagaatatgg 360
   gccagactgt taccactccc ttaagtttga ccttaggtca ctggaaagat gtcgagcgga 420
   tcgctcacaa ccagtcggta gatgtcaaga agagacgttg ggttaccttc tgctctgcag 480
   aatggccaac ctttaacgtc ggatggccgc gagacggcac ctttaaccga gacctcatca 540
   cccaggttaa gatcaaggtc ttttcacctg gcccgcatgg acacccagac caggtcccct 600
   acatcgtgac ctgggaagcc ttggcttttg acccccctcc ctgggtcaag ccctttgtac 660
   accctaagcc tccgcctcct cttcctccat ccgccccgtc tctccccctt gaacctcctc 720
   gttcgacccc gcctcgatcc tccctttatc cagccctcac tccttctcta ggcgccaaac 780
   ctaaacctca agttctttct gacagtgggg ggccgctcat cgacctactt acagaagacc 840
   ccccgcctta tagggaccca agaccacccc cttccgacag ggacggaaat ggtggagaag 900
   cgacccctgc gggagaggca ccggacccct ccccaatggc atctcgccta cgtgggagac 960
   gggagccccc tgtggccgac tccactacct cgcaggcatt ccccctccgc gcaggaggaa 1020
   acggacagct tcaatactgg ccgttctcct cttctgacct ttacaactgg aaaaataata 1080
   acccttcttt ttctgaagat ccaggtaaac tgacagctct gatcgagtct gttctcatca 1140
   cccatcagcc cacctgggac gactgtcagc agctgttggg gactctgctg accggagaag 1200
   aaaaacaacg ggtgctctta gaggctagaa aggcggtgcg gggcgatgat gggcgcccca 1260
   ctcaactgcc caatgaagtc gatgccgctt ttcccctcga gcgcccagac tgggattaca 1320
   ccacccaggc aggtaggaac cacctagtcc actatcgcca gttgctccta gcgggtctcc 1380
   aaaacgcggg cagaagcccc accaatttgg ccaaggtaaa aggaataaca caagggccca 1440
   atgagtctcc ctcggccttc ctagagagac ttaaggaagc ctatcgcagg tacactcctt 1500
   atgaccctga ggacccaggg caagaaacta atgtgtctat gtctttcatt tggcagtctg 1560
   ccccagacat tgggagaaag ttagagaggt tagaagattt aaaaaacaag acgcttggag 1620
   atttggttag agaggcagaa aagatcttta ataaacgaga aaccccggaa gaaagagagg 1680
   aacgtatcag gagagaaaca gaggaaaaag aagaacgccg taggacagag gatgagcaga 1740
   aagagaaaga aagagatcgt aggagacata gagagatgag caagctattg gccactgtcg 1800
   ttagtggaca gaaacaggat agacagggag gagaacgaag gaggtcccaa ctcgatcgcg 1860
   accagtgtgc ctactgcaaa gaaaaggggc actgggctaa agattgtccc aagaaaccac 1920
   gaggacctcg gggaccaaga ccccagacct ccctcctgac cctagatgac tagggaggtc 1980
   agggtcagga gcccccccct gaacccagga taaccctcaa agtcgggggg caacccgtca 2040
   ccttcctggt agatactggg gcccaacact ccgtgctgac ccaaaatcct ggacccctaa 2100
   gtgataagtc tgcctgggtc caaggggcta ctggaggaaa gcggtatcgc tggaccacgg 2160
   atcgcaaagt acatctagct accggtaagg tcacccactc tttcctccat gtaccagact 2220
   gtccctatcc tctgttagga agagatttgc tgactaaact aaaagcccaa atccactttg 2280
   agggatcagg agctcaggtt atgggaccaa tggggcagcc cctgcaagtg ttgaccctaa 2340
   atatagaaga tgagcatcgg ctacatgaga cctcaaaaga gccagatgtt tctctagggt 2400
   ccacatggct gtctgatttt cctcaggcct gggcggaaac cgggggcatg ggactggcag 2460
   ttcgccaagc tcctctgatc atacctctga aagcaacctc tacccccgtg tccataaaac 2520
   aataccccat gtcacaagaa gccagactgg ggatcaagcc ccacatacag agactgttgg 2580
   accagggaat actggtaccc tgccagtccc cctggaacac gcccctgcta cccgttaaga 2640
   aaccagggac taatgattat aggcctgtcc aggatctgag agaagtcaac aagcgggtgg 2700
   aagacatcca ccccaccgtg cccaaccctt acaacctctt gagcgggctc ccaccgtccc 2760
   accagtggta cactgtgctt gatttaaagg atgccttttt ctgcctgaga ctccacccca 2820
   ccagtcagcc tctcttcgcc tttgagtgga gagatccaga gatgggaatc tcaggacaat 2880
   tgacctggac cagactccca cagggtttca aaaacagtcc caccctgttt gatgaggcac 2940
   tgcacagaga cctagcagac ttccggatcc agcacccaga cttgatcctg ctacagtacg 3000
   tggatgactt actgctggcc gccacttctg agctagactg ccaacaaggt actcgggccc 3060
   tgttacaaac cctagggaac ctcgggtatc gggcctcggc caagaaagcc caaatttgcc 3120
   agaaacaggt caagtatctg gggtatcttc taaaagaggg tcagagatgg ctgactgagg 3180
   ccagaaaaga gactgtgatg gggcagccta ctccgaagac ccctcgacaa ctaagggagt 3240
   tcctagggac ggcaggcttc tgtcgcctct ggatccctgg gtttgcagaa atggcagccc 3300
   ccttgtaccc tctcaccaaa acggggactc tgtttaattg gggcccagac caacaaaagg 3360
   cctatcaaga aatcaagcaa gctcttctaa ctgccccagc cctggggttg ccagatttga 3420
   ctaagccctt tgaactcttt gtcgacgaga agcagggcta cgccaaaggt gtcctaacgc 3480
   aaaaactggg accttggcgt cggccggtgg cctacctgtc caaaaagcta gacccagtag 3540
   cagctgggtg gcccccttgc ctacggatgg tagcagccat tgccgtactg acaaaggatg 3600
   caggcaagct aaccatggga cagccactag tcattctggc cccccatgca gtagaggcac 3660
   tagtcaaaca accccccgac cgctggcttt ccaacgcccg gatgactcac tatcaggcct 3720
   tgcttttgga cacggaccgg gtccagttcg gaccggtggt agccctgaac ccggctacgc 3780
   tgctcccact gcctgaggaa gggctgcaac acaactgcct tgatatcctg gccgaagccc 3840
   acggaacccg acccgaccta acggaccagc cgctcccaga cgccgaccac acctggtaca 3900
   cggatggaag cagtctctta caagagggac agcgtaaggc gggagctgcg gtgaccaccg 3960
   agaccgaggt aatctgggct aaagccctgc cagccgggac atccgctcag cgggctgaac 4020
   tgatagcact cacccaggcc ctaaagatgg cagaaggtaa gaagctaaat gtttatactg 4080
   atagccgtta tgcttttgct actgcccata tccatggaga aatatacaga aggcgtgggt 4140
   tgctcacatc agaaggcaaa gagatcaaaa ataaagacga gatcttggcc ctactaaaag 4200
   ccctctttct gcccaaaaga cttagcataa tccattgtcc aggacatcaa aagggacaca 4260
   gcgccgaggc tagaggcaac cggatggctg accaagcggc ccgaaaggca gccatcacag 4320
   agactccaga cacctctacc ctcctcatag aaaattcatc accctacacc tcagaacatt 4380
   ttcattacac agtgactgat ataaaggacc taaccaagtt gggggccatt tatgataaaa 4440
   caaagaagta ttgggtctac caaggaaaac ctgtgatgcc tgaccagttt acttttgaat 4500
   tattagactt tcttcatcag ctgactcacc tcagcttctc aaaaatgaag gctctcctag 4560
   agagaagcca cagtccctac tacatgctga accgggatcg aacactcaaa aatatcactg 4620
   agacctgcaa agcttgtgca caagtcaacg ccagcaagtc tgccgttaaa cagggaacta 4680
   gggtccgcgg gcatcggccc ggcactcatt gggagatcga tttcaccgag ataaagcccg 4740
   gattgtatgg ctataaatat cttctagttt ttatagatac cttttctggc tggatagaag 4800
   ccttcccaac caagaaagaa accgccaagg tcgtaaccaa gaagctacta gaggagatct 4860
   tccccaggtt cggcatgcct caggtattgg gaactgacaa tgggcctgcc ttcgtctcca 4920
   aggtgagtca gacagtggcc gatctgttgg ggattgattg gaaattacat tgtgcataca 4980
   gaccccaaag ctcaggccag gtagaaagaa tgaatagaac catcaaggag actttaacta 5040
   aattaacgct tgcaactggc tctagagact gggtgctcct actcccctta gccctgtacc 5100
   gagcccgcaa cacgccgggc ccccatggcc tcaccccata tgagatctta tatggggcac 5160
   ccccgcccct tgtaaacttc cctgaccctg acatgacaag agttactaac agcccctctc 5220
   tccaagctca cttacaggct ctctacttag tccagcacga agtctggaga cctctggcgg 5280
   cagcctacca agaacaactg gaccgaccgg tggtacctca cccttaccga gtcggcgaca 5340
   cagtgtgggt ccgccgacac cagactaaga acctagaacc tcgctggaaa ggaccttaca 5400
   cagtcctgct gaccaccccc accgccctca aagtagacgg catcgcagct tggatacacg 5460
   ccgcccacgt gaaggctgcc gaccccgggg gtggaccatc ctctagactg acatggcgcg 5520
   ttcaacgctc tcaaaacccc ttaaaaataa ggttaacccg cgaggccccc taatcccctt 5580
   aattcttctg atgctcagag gggtcagtac tgcttcgccc ggctccagtc ctcatcaagt 5640
   ctataatatc acctgggagg taaccaatgg agatcgggag acggtatggg caacttctgg 5700
   caaccaccct ctgtggacct ggtggcctga ccttacccca gatttatgta tgttagccca 5760
   ccatggacca tcttattggg ggctagaata tcaatcccct ttttcttctc ccccggggcc 5820
   cccttgttgc tcagggggca gcagcccagg ctgttccaga gactgcgaag aacctttaac 5880
   ctccctcacc cctcggtgca acactgcctg gaacagactc aagctagacc agacaactca 5940
   taaatcaaat gagggatttt atgtttgccc cgggccccac cgcccccgag aatccaagtc 6000
   atgtgggggt ccagactcct tctactgtgc ctattggggc tgtgagacaa ccggtagagc 6060
   ttactggaag ccctcctcat catgggattt catcacagta aacaacaatc tcacctctga 6120
   ccaggctgtc caggtatgca aagataataa gtggtgcaac cccttagtta ttcggtttac 6180
   agacgccggg agacgggtta cttcctggac cacaggacat tactggggct tacgtttgta 6240
   tgtctccgga caagatccag ggcttacatt tgggatccga ctcagatacc aaaatctagg 6300
   accccgcgtc ccaatagggc caaaccccgt tctggcagac caacagccac tctccaagcc 6360
   caaacctgtt aagtcgcctt cagtcaccaa accacccagt gggactcctc tctcccctac 6420
   ccaacttcca ccggcgggaa cggaaaatag gctgctaaac ttagtagacg gagcctacca 6480
   agccctcaac ctcaccagtc ctgacaaaac ccaagagtgc tggttgtgtc tagtagcggg 6540
   acccccctac tacgaagggg ttgccgtcct gggtacctac tccaaccata cctctgctcc 6600
   agccaactgc tccgtggcct cccaacacaa gttgaccctg tccgaagtga ccggacaggg 6660
   actctgcata ggagcagttc ccaaaacaca tcaggcccta tgtaatacca cccagacaag 6720
   cagtcgaggg tcctattatc tagttgcccc tacaggtacc atgtgggctt gtagtaccgg 6780
   gcttactcca tgcatctcca ccaccatact gaaccttacc actgattatt gtgttcttgt 6840
   cgaactctgg ccaagagtca cctatcattc ccccagctat gtttacggcc tgtttgagag 6900
   atccaaccga cacaaaagag aaccggtgtc gttaaccctg gccctattat tgggtggact 6960
   aaccatgggg ggaattgccg ctggaatagg aacagggact actgctctaa tggccactca 7020
   gcaattccag cagctccaag ccgcagtaca ggatgatctc agggaggttg aaaaatcaat 7080
   ctctaaccta gaaaagtctc tcacttccct gtctgaagtt gtcctacaga atcgaagggg 7140
   cctagacttg ttatttctaa aagaaggagg gctgtgtgct gctctaaaag aagaatgttg 7200
   cttctatgcg gaccacacag gactagtgag agacagcatg gccaaattga gagagaggct 7260
   taatcagaga cagaaactgt ttgagtcaac tcaaggatgg tttgagggac tgtttaacag 7320
   atccccttgg tttaccacct tgatatctac cattatggga cccctcattg tactcctaat 7380
   gattttgctc ttcggaccct gcattcttaa tcgattagtc caatttgtta aagacaggat 7440
   atcagtggtc caggctctag ttttgactca acaatatcac cagctgaagc ctatagagta 7500
   cgagccatag ataaaataaa agattttatt tagtctccag aaaaaggggg gaatgaaaga 7560
   ccccacctgt aggtttggca agctagctta agtaacgcca ttttgcaagg catggaaaaa 7620
   tacataactg agaatagaga agttcagatc aaggtcagga acagatggaa cagctgaata 7680
   tgggccaaac aggatatctg tggtaagcag ttcctgcccc ggctcagggc caagaacaga 7740
   tggaacagct gaatatgggc caaacaggat atctgtggta agcagttcct gccccggctc 7800
   agggccaaga acagatggtc cccagatgcg gtccagccct cagcagtttc tagagaacca 7860
   tcagatgttt ccagggtgcc ccaaggacct gaaatgaccc tgtgccttat ttgaactaac 7920
   caatcagttc gcttctcgct tctgttcgcg cgcttctgct ccccgagctc aataaaagag 7980
   cccacaaccc ctcactcggg gcgccagtcc tccgattgac tgagtcgccc gggtacccgt 8040
   gtatccaata aaccctcttg cagttgcagc gccagtcctc cgattgactg agtcgcccgg 8100
   gtacccgtgt atccaataaa ccctcttgca gttgcatccg acttgtggtc tcgctgttcc 8160
   ttgggagggt ctcctctgag tgattgacta cccgtcagcg ggggtctttc atttgggggc 8220
   tcgtccggga tcgggagacc cctgcccagg gaccaccgac ccaccaccgg gaggtaagct 8280
   ggccagcaac ttatctgtgt ctgtccgatt gtctagtgtc tatgactgat tt 8332
<210> 12
   <211> 538
   <212> PRT
   <213> Moloney murine leukemia virus
<220>
   <223> gag-Protein
<400> 12
   Met Gly Gln Thr Val Thr Thr Pro Leu Ser Leu Thr Leu Gly His Trp
   1 5 10 15
   Lys Asp Val Glu Arg Ile Ala His Asn Gln Ser Val Asp Val Lys Lys
   20 25 30
   Arg Arg Trp Val Thr Phe Cys Ser Ala Glu Trp Pro Thr Phe Asn Val
   35 40 45
   Gly Trp Pro Arg Asp Gly Thr Phe Asn Arg Asp Leu Ile Thr Gln Val
   50 55 60
   Lys Ile Lys Val Phe Ser Pro Gly Pro His Gly His Pro Asp Gln Val
   65 70 75 80
   Pro Tyr Ile Val Thr Trp Glu Ala Leu Ala Phe Asp Pro Pro Pro Trp
   85 90 95
   Val Lys Pro Phe Val His Pro Lys Pro Pro Pro Pro Leu Pro Pro Ser
   100 105 110
   Ala Pro Ser Leu Pro Leu Glu Pro Pro Arg Ser Thr Pro Pro Arg Ser
   115 120 125
   Ser Leu Tyr Pro Ala Leu Thr Pro Ser Leu Gly Ala Lys Pro Lys Pro
   130 135 140
   Gln Val Leu Ser Asp Ser Gly Gly Pro Leu Ile Asp Leu Leu Thr Glu
   145 150 155 160
   Asp Pro Pro Pro Tyr Arg Asp Pro Arg Pro Pro Pro Ser Asp Arg Asp
   165 170 175
   Gly Asn Gly Gly Glu Ala Thr Pro Ala Gly Glu Ala Pro Asp Pro Ser
               180                 185                 190
   Pro Met Ala Ser Arg Leu Arg Gly Arg Arg Glu Pro Pro Val Ala Asp
           195                 200                 205
   Ser Thr Thr Ser Gln Ala Phe Pro Leu Arg Ala Gly Gly Asn Gly Gln
       210                 215                 220
   Leu Gln Tyr Trp Pro Phe Ser Ser Ser Asp Leu Tyr Asn Trp Lys Asn
   225                 230                 235                 240
   Asn Asn Pro Ser Phe Ser Glu Asp Pro Gly Lys Leu Thr Ala Leu Ile
                   245                 250                 255
   Glu Ser Val Leu Ile Thr His Gln Pro Thr Trp Asp Asp Cys Gln Gln
               260                 265                 270
   Leu Leu Gly Thr Leu Leu Thr Gly Glu Glu Lys Gln Arg Val Leu Leu
           275                 280                 285
   Glu Ala Arg Lys Ala Val Arg Gly Asp Asp Gly Arg Pro Thr Gln Leu
       290                 295                 300
   Pro Asn Glu Val Asp Ala Ala Phe Pro Leu Glu Arg Pro Asp Trp Asp
   305                 310                 315                 320
   Tyr Thr Thr Gln Ala Gly Arg Asn His Leu Val His Tyr Arg Gln Leu
                   325                 330                 335
   Leu Leu Ala Gly Leu Gln Asn Ala Gly Arg Ser Pro Thr Asn Leu Ala
               340                 345                 350
   Lys Val Lys Gly Ile Thr Gln Gly Pro Asn Glu Ser Pro Ser Ala Phe
           355                 360                 365
   Leu Glu Arg Leu Lys Glu Ala Tyr Arg Arg Tyr Thr Pro Tyr Asp Pro
       370                 375                 380
   Glu Asp Pro Gly Gln Glu Thr Asn Val Ser Met Ser Phe Ile Trp Gln
   385                 390                 395                 400
   Ser Ala Pro Asp Ile Gly Arg Lys Leu Glu Arg Leu Glu Asp Leu Lys
                   405                 410                 415
   Asn Lys Thr Leu Gly Asp Leu Val Arg Glu Ala Glu Lys Ile Phe Asn
               420                 425                 430
   Lys Arg Glu Thr Pro Glu Glu Arg Glu Glu Arg Ile Arg Arg Glu Thr
           435                 440                 445
   Glu Glu Lys Glu Glu Arg Arg Arg Thr Glu Asp Glu Gln Lys Glu Lys
       450                 455                 460
   Glu Arg Asp Arg Arg Arg His Arg Glu Met Ser Lys Leu Leu Ala Thr
   465                 470                 475                 480
   Val Val Ser Gly Gln Lys Gln Asp Arg Gln Gly Gly Glu Arg Arg Arg
                   485                 490                 495
   Ser Gln Leu Asp Arg Asp Gln Cys Ala Tyr Cys Lys Glu Lys Gly His
               500                 505                 510
   Trp Ala Lys Asp Cys Pro Lys Lys Pro Arg Gly Pro Arg Gly Pro Arg
           515                 520                 525
   Pro Gln Thr Ser Leu Leu Thr Leu Asp Asp
       530                 535
<210> 13
   <211> 1737
   <212> PRT
   <213> Moloney murine leukemia virus
<220>
   <223> gag-pol-Protein
<400> 13
   Met Gly Gln Thr Val Thr Thr Pro Leu Ser Leu Thr Leu Gly His Trp
   1               5                   10                  15
   Lys Asp Val Glu Arg Ile Ala His Asn Gln Ser Val Asp Val Lys Lys
               20                  25                  30
   Arg Arg Trp Val Thr Phe Cys Ser Ala Glu Trp Pro Thr Phe Asn Val
           35                  40                  45
   Gly Trp Pro Arg Asp Gly Thr Phe Asn Arg Asp Leu Ile Thr Gln Val
   50 55 60
   Lys Ile Lys Val Phe Ser Pro Gly Pro His Gly His Pro Asp Gln Val
   65 70 75 80
   Pro Tyr Ile Val Thr Trp Glu Ala Leu Ala Phe Asp Pro Pro Pro Trp
   85 90 95
   Val Lys Pro Phe Val His Pro Lys Pro Pro Pro Pro Leu Pro Pro Ser
   100 105 110
   Ala Pro Ser Leu Pro Leu Glu Pro Pro Arg Ser Thr Pro Pro Arg Ser
   115 120 125
   Ser Leu Tyr Pro Ala Leu Thr Pro Ser Leu Gly Ala Lys Pro Lys Pro
   130 135 140
   Gln Val Leu Ser Asp Ser Gly Gly Pro Leu Ile Asp Leu Leu Thr Glu
   145 150 155 160
   Asp Pro Pro Pro Tyr Arg Asp Pro Arg Pro Pro Pro Ser Asp Arg Asp
   165 170 175
   Gly Asn Gly Gly Glu Ala Thr Pro Ala Gly Glu Ala Pro Asp Pro Ser
   180 185 190
   Pro Met Ala Ser Arg Leu Arg Gly Arg Arg Glu Pro Pro Val Ala Asp
   195 200 205
   Ser Thr Thr Ser Gln Ala Phe Pro Leu Arg Ala Gly Gly Asn Gly Gln
   210 215 220
   Leu Gln Tyr Trp Pro Phe Ser Ser Ser Asp Leu Tyr Asn Trp Lys Asn
   225 230 235 240
   Asn Asn Pro Ser Phe Ser Glu Asp Pro Gly Lys Leu Thr Ala Leu Ile
   245 250 255
   Glu Ser Val Leu Ile Thr His Gln Pro Thr Trp Asp Asp Cys Gln Gln
   260 265 270
   Leu Leu Gly Thr Leu Leu Thr Gly Glu Glu Lys Gln Arg Val Leu Leu
   275 280 285
   Glu Ala Arg Lys Ala Val Arg Gly Asp Asp Gly Arg Pro Thr Gln Leu
   290 295 300
   Pro Asn Glu Val Asp Ala Ala Phe Pro Leu Glu Arg Pro Asp Trp Asp
   305 310 315 320
   Tyr Thr Thr Gln Ala Gly Arg Asn His Leu Val His Tyr Arg Gln Leu
   325 330 335
   Leu Leu Ala Gly Leu Gln Asn Ala Gly Arg Ser Pro Thr Asn Leu Ala
   340 345 350
   Lys Val Lys Gly Ile Thr Gln Gly Pro Asn Glu Ser Pro Ser Ala Phe
   355 360 365
   Leu Glu Arg Leu Lys Glu Ala Tyr Arg Arg Tyr Thr Pro Tyr Asp Pro
   370 375 380
   Glu Asp Pro Gly Gln Glu Thr Asn Val Ser Met Ser Phe Ile Trp Gln
   385 390 395 400
   Ser Ala Pro Asp Ile Gly Arg Lys Leu Glu Arg Leu Glu Asp Leu Lys
   405 410 415
   Asn Lys Thr Leu Gly Asp Leu Val Arg Glu Ala Glu Lys Ile Phe Asn
   420 425 430
   Lys Arg Glu Thr Pro Glu Glu Arg Glu Glu Arg Ile Arg Arg Glu Thr
   435 440 445
   Glu Glu Lys Glu Glu Arg Arg Arg Thr Glu Asp Glu Gln Lys Glu Lys
   450 455 460
   Glu Arg Asp Arg Arg Arg His Arg Glu Met Ser Lys Leu Leu Ala Thr
   465 470 475 480
   Val Val Ser Gly Gln Lys Gln Asp Arg Gln Gly Gly Glu Arg Arg Arg
   485 490 495
   Ser Gln Leu Asp Arg Asp Gln Cys Ala Tyr Cys Lys Glu Lys Gly His
   500 505 510
   Trp Ala Lys Asp Cys Pro Lys Lys Pro Arg Gly Pro Arg Gly Pro Arg
   515 520 525
   Pro Gln Thr Ser Leu Leu Thr Leu Asp Asp Gly Gly Gln Gly Gln Glu
   530 535 540
   Pro Pro Pro Glu Pro Arg Ile Thr Leu Lys Val Gly Gly Gln Pro Val
   545 550 555 560
   Thr Phe Leu Val Asp Thr Gly Ala Gln His Ser Val Leu Thr Gln Asn
   565 570 575
   Pro Gly Pro Leu Ser Asp Lys Ser Ala Trp Val Gln Gly Ala Thr Gly
   580 585 590
   Gly Lys Arg Tyr Arg Trp Thr Thr Asp Arg Lys Val His Leu Ala Thr
   595 600 605
   Gly Lys Val Thr His Ser Phe Leu His Val Pro Asp Cys Pro Tyr Pro
   610 615 620
   Leu Leu Gly Arg Asp Leu Leu Thr Lys Leu Lys Ala Gln Ile His Phe
   625 630 635 640
   Glu Gly Ser Gly Ala Gln Val Met Gly Pro Met Gly Gln Pro Leu Gln
   645 650 655
   Val Leu Thr Leu Asn Ile Glu Asp Glu His Arg Leu His Glu Thr Ser
   660 665 670
   Lys Glu Pro Asp Val Ser Leu Gly Ser Thr Trp Leu Ser Asp Phe Pro
   675 680 685
   Gln Ala Trp Ala Glu Thr Gly Gly Met Gly Leu Ala Val Arg Gln Ala
   690 695 700
   Pro Leu Ile Ile Pro Leu Lys Ala Thr Ser Thr Pro Val Ser Ile Lys
   705 710 715 720
   Gln Tyr Pro Met Ser Gln Glu Ala Arg Leu Gly Ile Lys Pro His Ile
   725 730 735
   Gln Arg Leu Leu Asp Gln Gly Ile Leu Val Pro Cys Gln Ser Pro Trp
   740 745 750
   Asn Thr Pro Leu Leu Pro Val Lys Lys Pro Gly Thr Asn Asp Tyr Arg
   755 760 765
   Pro Val Gln Asp Leu Arg Glu Val Asn Lys Arg Val Glu Asp Ile His
   770 775 780
   Pro Thr Val Pro Asn Pro Tyr Asn Leu Leu Ser Gly Leu Pro Pro Ser
   785 790 795 800
   His Gln Trp Tyr Thr Val Leu Asp Leu Lys Asp Ala Phe Phe Cys Leu
   805 810 815
   Arg Leu His Pro Thr Ser Gln Pro Leu Phe Ala Phe Glu Trp Arg Asp
   820 825 830
   Pro Glu Met Gly Ile Ser Gly Gln Leu Thr Trp Thr Arg Leu Pro Gln
   835 840 845
   Gly Phe Lys Asn Ser Pro Thr Leu Phe Asp Glu Ala Leu His Arg Asp
   850 855 860
   Leu Ala Asp Phe Arg Ile Gln His Pro Asp Leu Ile Leu Leu Gln Tyr
   865 870 875 880
   Val Asp Asp Leu Leu Leu Ala Ala Thr Ser Glu Leu Asp Cys Gln Gln
   885 890 895
   Gly Thr Arg Ala Leu Leu Gln Thr Leu Gly Asn Leu Gly Tyr Arg Ala
   900 905 910
   Ser Ala Lys Lys Ala Gln Ile Cys Gln Lys Gln Val Lys Tyr Leu Gly
   915 920 925
   Tyr Leu Leu Lys Glu Gly Gln Arg Trp Leu Thr Glu Ala Arg Lys Glu
   930 935 940
   Thr Val Met Gly Gln Pro Thr Pro Lys Thr Pro Arg Gln Leu Arg Glu
   945 950 955 960
   Phe Leu Gly Thr Ala Gly Phe Cys Arg Leu Trp Ile Pro Gly Phe Ala
   965 970 975
   Glu Met Ala Ala Pro Leu Tyr Pro Leu Thr Lys Thr Gly Thr Leu Phe
   980 985 990
   Asn Trp Gly Pro Asp Gln Gln Lys Ala Tyr Gln Glu Ile Lys Gln Ala
   995 1000 1005
   Leu Leu Thr Ala Pro Ala Leu Gly Leu Pro Asp Leu Thr Lys Pro Phe
   1010 1015 1020
   Glu Leu Phe Val Asp Glu Lys Gln Gly Tyr Ala Lys Gly Val Leu Thr
   1025 1030 1035 1040
   Gln Lys Leu Gly Pro Trp Arg Arg Pro Val Ala Tyr Leu Ser Lys Lys
   1045 1050 1055
   Leu Asp Pro Val Ala Ala Gly Trp Pro Pro Cys Leu Arg Met Val Ala
   1060 1065 1070
   Ala Ile Ala Val Leu Thr Lys Asp Ala Gly Lys Leu Thr Met Gly Gln
           1075                1080                1085
   Pro Leu Val Ile Leu Ala Pro His Ala Val Glu Ala Leu Val Lys Gln
       1090                1095                1100
   Pro Pro Asp Arg Trp Leu Ser Asn Ala Arg Met Thr His Tyr Gln Ala
   1105                1110                1115                1120
   Leu Leu Leu Asp Thr Asp Arg Val Gln Phe Gly Pro Val Val Ala Leu
                   1125                1130                1135
   Asn Pro Ala Thr Leu Leu Pro Leu Pro Glu Glu Gly Leu Gln His Asn
               1140                1145                1150
   Cys Leu Asp Ile Leu Ala Glu Ala His Gly Thr Arg Pro Asp Leu Thr
           1155                1160                1165
   Asp Gln Pro Leu Pro Asp Ala Asp His Thr Trp Tyr Thr Asp Gly Ser
       1170                1175                1180
   Ser Leu Leu Gln Glu Gly Gln Arg Lys Ala Gly Ala Ala Val Thr Thr
   1185                1190                1195                1200
   Glu Thr Glu Val Ile Trp Ala Lys Ala Leu Pro Ala Gly Thr Ser Ala
                   1205                1210                1215
   Gln Arg Ala Glu Leu Ile Ala Leu Thr Gln Ala Leu Lys Met Ala Glu
               1220                1225                1230
   Gly Lys Lys Leu Asn Val Tyr Thr Asp Ser Arg Tyr Ala Phe Ala Thr
           1235                1240                1245
   Ala His Ile His Gly Glu Ile Tyr Arg Arg Arg Gly Leu Leu Thr Ser
       1250                1255                1260
   Glu Gly Lys Glu Ile Lys Asn Lys Asp Glu Ile Leu Ala Leu Leu Lys
   1265                1270                1275                1280
   Ala Leu Phe Leu Pro Lys Arg Leu Ser Ile Ile His Cys Pro Gly His
                   1285                1290                1295
   Gln Lys Gly His Ser Ala Glu Ala Arg Gly Asn Arg Met Ala Asp Gln
               1300                1305                1310
   Ala Ala Arg Lys Ala Ala Ile Thr Glu Thr Pro Asp Thr Ser Thr Leu
           1315                1320                1325
   Leu Ile Glu Asn Ser Ser Pro Tyr Thr Ser Glu His Phe His Tyr Thr
       1330                1335                1340
   Val Thr Asp Ile Lys Asp Leu Thr Lys Leu Gly Ala Ile Tyr Asp Lys
   1345                1350                1355                1360
   Thr Lys Lys Tyr Trp Val Tyr Gln Gly Lys Pro Val Met Pro Asp Gln
                   1365                1370                1375
   Phe Thr Phe Glu Leu Leu Asp Phe Leu His Gln Leu Thr His Leu Ser
               1380                1385                1390
   Phe Ser Lys Met Lys Ala Leu Leu Glu Arg Ser His Ser Pro Tyr Tyr
           1395                1400                1405
   Met Leu Asn Arg Asp Arg Thr Leu Lys Asn Ile Thr Glu Thr Cys Lys
       1410                1415                1420
   Ala Cys Ala Gln Val Asn Ala Ser Lys Ser Ala Val Lys Gln Gly Thr
   1425                1430                1435                1440
   Arg Val Arg Gly His Arg Pro Gly Thr His Trp Glu Ile Asp Phe Thr
                   1445                1450                1455
   Glu Ile Lys Pro Gly Leu Tyr Gly Tyr Lys Tyr Leu Leu Val Phe Ile
               1460                1465                1470
   Asp Thr Phe Ser Gly Trp Ile Glu Ala Phe Pro Thr Lys Lys Glu Thr
           1475                1480                1485
   Ala Lys Val Val Thr Lys Lys Leu Leu Glu Glu Ile Phe Pro Arg Phe
       1490                1495                1500
   Gly Met Pro Gln Val Leu Gly Thr Asp Asn Gly Pro Ala Phe Val Ser
   1505                1510                1515                1520
   Lys Val Ser Gln Thr Val Ala Asp Leu Leu Gly Ile Asp Trp Lys Leu
                   1525                1530                1535
   His Cys Ala Tyr Arg Pro Gln Ser Ser Gly Gln Val Glu Arg Met Asn
               1540                1545                1550
   Arg Thr Ile Lys Glu Thr Leu Thr Lys Leu Thr Leu Ala Thr Gly Ser
           1555                1560                1565
   Arg Asp Trp Val Leu Leu Leu Pro Leu Ala Leu Tyr Arg Ala Arg Asn
       1570                1575                1580
   Thr Pro Gly Pro His Gly Leu Thr Pro Tyr Glu Ile Leu Tyr Gly Ala
   1585                1590                1595                1600
   Pro Pro Pro Leu Val Asn Phe Pro Asp Pro Asp Met Thr Arg Val Thr
                   1605                1610                1615
   Asn Ser Pro Ser Leu Gln Ala His Leu Gln Ala Leu Tyr Leu Val Gln
               1620                1625                1630
   His Glu Val Trp Arg Pro Leu Ala Ala Ala Tyr Gln Glu Gln Leu Asp
           1635                1640                1645
   Arg Pro Val Val Pro His Pro Tyr Arg Val Gly Asp Thr Val Trp Val
       1650                1655                1660
   Arg Arg His Gln Thr Lys Asn Leu Glu Pro Arg Trp Lys Gly Pro Tyr
   1665                1670                1675                1680
   Thr Val Leu Leu Thr Thr Pro Thr Ala Leu Lys Val Asp Gly Ile Ala
                   1685                1690                1695
   Ala Trp Ile His Ala Ala His Val Lys Ala Ala Asp Pro Gly Gly Gly
               1700                1705                1710
   Pro Ser Ser Arg Leu Thr Trp Arg Val Gln Arg Ser Gln Asn Pro Leu
           1715                1720                1725
   Lys Ile Arg Leu Thr Arg Glu Ala Pro
       1730                1735
<210> 14
   <211> 665
   <212> PRT
   <213> Moloney murine leukemia virus
<220>
   <223> env-Protein
<400> 14
   Met Ala Arg Ser Thr Leu Ser Lys Pro Leu Lys Asn Lys Val Asn Pro
   1               5                   10                  15
   Arg Gly Pro Leu Ile Pro Leu Ile Leu Leu Met Leu Arg Gly Val Ser
               20                  25                  30
   Thr Ala Ser Pro Gly Ser Ser Pro His Gln Val Tyr Asn Ile Thr Trp
           35                  40                  45
   Glu Val Thr Asn Gly Asp Arg Glu Thr Val Trp Ala Thr Ser Gly Asn
       50                  55                  60
   His Pro Leu Trp Thr Trp Trp Pro Asp Leu Thr Pro Asp Leu Cys Met
   65                  70                  75                  80
   Leu Ala His His Gly Pro Ser Tyr Trp Gly Leu Glu Tyr Gln Ser Pro
                   85                  90                  95
   Phe Ser Ser Pro Pro Gly Pro Pro Cys Cys Ser Gly Gly Ser Ser Pro
               100                 105                 110
   Gly Cys Ser Arg Asp Cys Glu Glu Pro Leu Thr Ser Leu Thr Pro Arg
           115                 120                 125
   Cys Asn Thr Ala Trp Asn Arg Leu Lys Leu Asp Gln Thr Thr His Lys
       130                 135                 140
   Ser Asn Glu Gly Phe Tyr Val Cys Pro Gly Pro His Arg Pro Arg Glu
   145                 150                 155                 160
   Ser Lys Ser Cys Gly Gly Pro Asp Ser Phe Tyr Cys Ala Tyr Trp Gly
                   165                 170                 175
   Cys Glu Thr Thr Gly Arg Ala Tyr Trp Lys Pro Ser Ser Ser Trp Asp
               180                 185                 190
   Phe Ile Thr Val Asn Asn Asn Leu Thr Ser Asp Gln Ala Val Gln Val
           195                 200                 205
   Cys Lys Asp Asn Lys Trp Cys Asn Pro Leu Val Ile Arg Phe Thr Asp
       210                 215                 220
   Ala Gly Arg Arg Val Thr Ser Trp Thr Thr Gly His Tyr Trp Gly Leu
   225                 230                 235                 240
   Arg Leu Tyr Val Ser Gly Gln Asp Pro Gly Leu Thr Phe Gly Ile Arg
                   245                 250                 255
   Leu Arg Tyr Gln Asn Leu Gly Pro Arg Val Pro Ile Gly Pro Asn Pro
               260                 265                 270
   Val Leu Ala Asp Gln Gln Pro Leu Ser Lys Pro Lys Pro Val Lys Ser
           275                 280                 285
   Pro Ser Val Thr Lys Pro Pro Ser Gly Thr Pro Leu Ser Pro Thr Gln
       290                 295                 300
   Leu Pro Pro Ala Gly Thr Glu Asn Arg Leu Leu Asn Leu Val Asp Gly
   305                 310                 315                     320
   Ala Tyr Gln Ala Leu Asn Leu Thr Ser Pro Asp Lys Thr Gln Glu Cys
                   325                 330                 335
   Trp Leu Cys Leu Val Ala Gly Pro Pro Tyr Tyr Glu Gly Val Ala Val
               340                 345                 350
   Leu Gly Thr Tyr Ser Asn His Thr Ser Ala Pro Ala Asn Cys Ser Val
           355                 360                 365
   Ala Ser Gln His Lys Leu Thr Leu Ser Glu Val Thr Gly Gln Gly Leu
       370                 375                 380
   Cys Ile Gly Ala Val Pro Lys Thr His Gln Ala Leu Cys Asn Thr Thr
   385                 390                 395                     400
   Gln Thr Ser Ser Arg Gly Ser Tyr Tyr Leu Val Ala Pro Thr Gly Thr
                   405                 410                 415
   Met Trp Ala Cys Ser Thr Gly Leu Thr Pro Cys Ile Ser Thr Thr Ile
               420                 425                 430
   Leu Asn Leu Thr Thr Asp Tyr Cys Val Leu Val Glu Leu Trp Pro Arg
           435                 440                 445
   Val Thr Tyr His Ser Pro Ser Tyr Val Tyr Gly Leu Phe Glu Arg Ser
       450                 455                 460
   Asn Arg His Lys Arg Glu Pro Val Ser Leu Thr Leu Ala Leu Leu Leu
   465                 470                 475                     480
   Gly Gly Leu Thr Met Gly Gly Ile Ala Ala Gly Ile Gly Thr Gly Thr
                   485                 490                 495
   Thr Ala Leu Met Ala Thr Gln Gln Phe Gln Gln Leu Gln Ala Ala Val
               500                 505                 510
   Gln Asp Asp Leu Arg Glu Val Glu Lys Ser Ile Ser Asn Leu Glu Lys
           515                 520                 525
   Ser Leu Thr Ser Leu Ser Glu Val Val Leu Gln Asn Arg Arg Gly Leu
       530                 535                 540
   Asp Leu Leu Phe Leu Lys Glu Gly Gly Leu Cys Ala Ala Leu Lys Glu
   545                 550                 555                     560
   Glu Cys Cys Phe Tyr Ala Asp His Thr Gly Leu Val Arg Asp Ser Met
                   565                 570                 575
   Ala Lys Leu Arg Glu Arg Leu Asn Gln Arg Gln Lys Leu Phe Glu Ser
               580                 585                 590
   Thr Gln Gly Trp Phe Glu Gly Leu Phe Asn Arg Ser Pro Trp Phe Thr
           595                 600                 605
   Thr Leu Ile Ser Thr Ile Met Gly Pro Leu Ile Val Leu Leu Met Ile
       610                 615                 620
   Leu Leu Phe Gly Pro Cys Ile Leu Asn Arg Leu Val Gln Phe Val Lys
   625                 630                 635                     640
   Asp Arg Ile Ser Val Val Gln Ala Leu Val Leu Thr Gln Gln Tyr His
                   645                 650                 655
   Gln Leu Lys Pro Ile Glu Tyr Glu Pro
               660                 665
<210> 15
   <211> 9709
   <212> DNA
   <213> Human immunodeficiency virus type 1
<400> 15
   tggaagggct aatttggtcc caaaaaagac aagagatcct tgatctgtgg atctaccaca 60
   cacaaggcta cttccctgat tggcagaact acacaccagg gccagggatc agatatccac 120
   tgacctttgg atggtgcttc aagttagtac cagttgaacc agagcaagta gaagaggcca 180
   aataaggaga gaagaacagc ttgttacacc ctatgagcca gcatgggatg gaggacccgg 240
   agggagaagt attagtgtgg aagtttgaca gcctcctagc atttcgtcac atggcccgag 300
   agctgcatcc ggagtactac aaagactgct gacatcgagc tttctacaag ggactttccg 360
   ctggggactt tccagggagg tgtggcctgg gcgggactgg ggagtggcga gccctcagat 420
   gctacatata agcagctgct ttttgcctgt actgggtctc tctggttaga ccagatctga 480
   gcctgggagc tctctggcta actagggaac ccactgctta agcctcaata aagcttgcct 540
   tgagtgctca aagtagtgtg tgcccgtctg ttgtgtgact ctggtaacta gagatccctc 600
   agaccctttt agtcagtgtg gaaaatctct agcagtggcg cccgaacagg gacttgaaag 660
   cgaaagtaaa gccagaggag atctctcgac gcaggactcg gcttgctgaa gcgcgcacgg 720
   caagaggcga ggggcggcga ctggtgagta cgccaaaaat tttgactagc ggaggctaga 780
   aggagagaga tgggtgcgag agcgtcggta ttaagcgggg gagaattaga taaatgggaa 840
   aaaattcggt taaggccagg gggaaagaaa caatataaac taaaacatat agtatgggca 900
   agcagggagc tagaacgatt cgcagttaat cctggccttt tagagacatc agaaggctgt 960
   agacaaatac tgggacagct acaaccatcc cttcagacag gatcagaaga acttagatca 1020
   ttatataata caatagcagt cctctattgt gtgcatcaaa ggatagatgt aaaagacacc 1080
   aaggaagcct tagataagat agaggaagag caaaacaaaa gtaagaaaaa ggcacagcaa 1140
   gcagcagctg acacaggaaa caacagccag gtcagccaaa attaccctat agtgcagaac 1200
   ctccaggggc aaatggtaca tcaggccata tcacctagaa ctttaaatgc atgggtaaaa 1260
   gtagtagaag agaaggcttt cagcccagaa gtaataccca tgttttcagc attatcagaa 1320
   ggagccaccc cacaagattt aaataccatg ctaaacacag tggggggaca tcaagcagcc 1380
   atgcaaatgt taaaagagac catcaatgag gaagctgcag aatgggatag attgcatcca 1440
   gtgcatgcag ggcctattgc accaggccag atgagagaac caaggggaag tgacatagca 1500
   ggaactacta gtacccttca ggaacaaata ggatggatga cacataatcc acctatccca 1560
   gtaggagaaa tctataaaag atggataatc ctgggattaa ataaaatagt aagaatgtat 1620
   agccctacca gcattctgga cataagacaa ggaccaaagg aaccctttag agactatgta 1680
   gaccgattct ataaaactct aagagccgag caagcttcac aagaggtaaa aaattggatg 1740
   acagaaacct tgttggtcca aaatgcgaac ccagattgta agactatttt aaaagcattg 1800
   ggaccaggag cgacactaga agaaatgatg acagcatgtc agggagtggg gggacccggc 1860
   cataaagcaa gagttttggc tgaagcaatg agccaagtaa caaatccagc taccataatg 1920
   atacagaaag gcaattttag gaaccaaaga aagactgtta agtgtttcaa ttgtggcaaa 1980
   gaagggcaca tagccaaaaa ttgcagggcc cctaggaaaa agggctgttg gaaatgtgga 2040
   aaggaaggac accaaatgaa agattgtact gagagacagg ctaatttttt agggaagatc 2100
   tggccttccc acaagggaag gccagggaat tttcttcaga gcagaccaga gccaacagcc 2160
   ccaccagaag agagcttcag gtttggggaa gagacaacaa ctccctctca gaagcaggag 2220
   ccgatagaca aggaactgta tcctttagct tccctcagat cactctttgg cagcgacccc 2280
   tcgtcacaat aaagataggg gggcaattaa aggaagctct attagataca ggagcagatg 2340
   atacagtatt agaagaaatg aatttgccag gaagatggaa accaaaaatg atagggggaa 2400
   ttggaggttt tatcaaagta ggacagtatg atcagatact catagaaatc tgcggacata 2460
   aagctatagg tacagtatta gtaggaccta cacctgtcaa cataattgga agaaatctgt 2520
   tgactcagat tggctgcact ttaaattttc ccattagtcc tattgagact gtaccagtaa 2580
   aattaaagcc aggaatggat ggcccaaaag ttaaacaatg gccattgaca gaagaaaaaa 2640
   taaaagcatt agtagaaatt tgtacagaaa tggaaaagga aggaaaaatt tcaaaaattg 2700
   ggcctgaaaa tccatacaat actccagtat ttgccataaa gaaaaaagac agtactaaat 2760
   ggagaaaatt agtagatttc agagaactta ataagagaac tcaagatttc tgggaagttc 2820
   aattaggaat accacatcct gcagggttaa aacagaaaaa atcagtaaca gtactggatg 2880
   tgggcgatgc atatttttca gttcccttag ataaagactt caggaagtat actgcattta 2940
   ccatacctag tataaacaat gagacaccag ggattagata tcagtacaat gtgcttccac 3000
   agggatggaa aggatcacca gcaatattcc agtgtagcat gacaaaaatc ttagagcctt 3060
   ttagaaaaca aaatccagac atagtcatct atcaatacat ggatgatttg tatgtaggat 3120
   ctgacttaga aatagggcag catagaacaa aaatagagga actgagacaa catctgttga 3180
   ggtggggatt taccacacca gacaaaaaac atcagaaaga acctccattc ctttggatgg 3240
   gttatgaact ccatcctgat aaatggacag tacagcctat agtgctgcca gaaaaggaca 3300
   gctggactgt caatgacata cagaaattag tgggaaaatt gaattgggca agtcagattt 3360
   atgcagggat taaagtaagg caattatgta aacttcttag gggaaccaaa gcactaacag 3420
   aagtagtacc actaacagaa gaagcagagc tagaactggc agaaaacagg gagattctaa 3480
   aagaaccggt acatggagtg tattatgacc catcaaaaga cttaatagca gaaatacaga 3540
   agcaggggca aggccaatgg acatatcaaa tttatcaaga gccatttaaa aatctgaaaa 3600
   caggaaaata tgcaagaatg aagggtgccc acactaatga tgtgaaacaa ttaacagagg 3660
   cagtacaaaa aatagccaca gaaagcatag taatatgggg aaagactcct aaatttaaat 3720
   tacccataca aaaggaaaca tgggaagcat ggtggacaga gtattggcaa gccacctgga 3780
   ttcctgagtg ggagtttgtc aatacccctc ccttagtgaa gttatggtac cagttagaga 3840
   aagaacccat aataggagca gaaactttct atgtagatgg ggcagccaat agggaaacta 3900
   aattaggaaa agcaggatat gtaactgaca gaggaagaca aaaagttgtc cccctaacgg 3960
   acacaacaaa tcagaagact gagttacaag caattcatct agctttgcag gattcgggat 4020
   tagaagtaaa catagtgaca gactcacaat atgcattggg aatcattcaa gcacaaccag 4080
   ataagagtga atcagagtta gtcagtcaaa taatagagca gttaataaaa aaggaaaaag 4140
   tctacctggc atgggtacca gcacacaaag gaattggagg aaatgaacaa gtagatgggt 4200
   tggtcagtgc tggaatcagg aaagtactat ttttagatgg aatagataag gcccaagaag 4260
   aacatgagaa atatcacagt aattggagag caatggctag tgattttaac ctaccacctg 4320
   tagtagcaaa agaaatagta gccagctgtg ataaatgtca gctaaaaggg gaagccatgc 4380
   atggacaagt agactgtagc ccaggaatat ggcagctaga ttgtacacat ttagaaggaa 4440
   aagttatctt ggtagcagtt catgtagcca gtggatatat agaagcagaa gtaattccag 4500
   cagagacagg gcaagaaaca gcatacttcc tcttaaaatt agcaggaaga tggccagtaa 4560
   aaacagtaca tacagacaat ggcagcaatt tcaccagtac tacagttaag gccgcctgtt 4620
   ggtgggcggg gatcaagcag gaatttggca ttccctacaa tccccaaagt caaggagtaa 4680
   tagaatctat gaataaagaa ttaaagaaaa ttataggaca ggtaagagat caggctgaac 4740
   atcttaagac agcagtacaa atggcagtat tcatccacaa ttttaaaaga aaagggggga 4800
   ttggggggta cagtgcaggg gaaagaatag tagacataat agcaacagac atacaaacta 4860
   aagaattaca aaaacaaatt acaaaaattc aaaattttcg ggtttattac agggacagca 4920
   gagatccagt ttggaaagga ccagcaaagc tcctctggaa aggtgaaggg gcagtagtaa 4980
   tacaagataa tagtgacata aaagtagtgc caagaagaaa agcaaagatc atcagggatt 5040
   atggaaaaca gatggcaggt gatgattgtg tggcaagtag acaggatgag gattaacaca 5100
   tggaaaagat tagtaaaaca ccatatgtat atttcaagga aagctaagga ctggttttat 5160
   agacatcact atgaaagtac taatccaaaa ataagttcag aagtacacat cccactaggg 5220
   gatgctaaat tagtaataac aacatattgg ggtctgcata caggagaaag agactggcat 5280
   ttgggtcagg gagtctccat agaatggagg aaaaagagat atagcacaca agtagaccct 5340
   gacctagcag accaactaat tcatctgcac tattttgatt gtttttcaga atctgctata 5400
   agaaatacca tattaggacg tatagttagt cctaggtgtg aatatcaagc aggacataac 5460
   aaggtaggat ctctacagta cttggcacta gcagcattaa taaaaccaaa acagataaag 5520
   ccacctttgc ctagtgttag gaaactgaca gaggacagat ggaacaagcc ccagaagacc 5580
   aagggccaca gagggagcca tacaatgaat ggacactaga gcttttagag gaacttaaga 5640
   gtgaagctgt tagacatttt cctaggatat ggctccataa cttaggacaa catatctatg 5700
   aaacttacgg ggatacttgg gcaggagtgg aagccataat aagaattctg caacaactgc 5760
   tgtttatcca tttcagaatt gggtgtcgac atagcagaat aggcgttact cgacagagga 5820
   gagcaagaaa tggagccagt agatcctaga ctagagccct ggaagcatcc aggaagtcag 5880
   cctaaaactg cttgtaccaa ttgctattgt aaaaagtgtt gctttcattg ccaagtttgt 5940
   ttcatgacaa aagccttagg catctcctat ggcaggaaga agcggagaca gcgacgaaga 6000
   gctcatcaga acagtcagac tcatcaagct tctctatcaa agcagtaagt agtacatgta 6060
   atgcaaccta taatagtagc aatagtagca ttagtagtag caataataat agcaatagtt 6120
   gtgtggtcca tagtaatcat agaatatagg aaaatattaa gacaaagaaa aatagacagg 6180
   ttaattgata gactaataga aagagcagaa gacagtggca atgagagtga aggagaagta 6240
   tcagcacttg tggagatggg ggtggaaatg gggcaccatg ctccttggga tattgatgat 6300
   ctgtagtgct acagaaaaat tgtgggtcac agtctattat ggggtacctg tgtggaagga 6360
   agcaaccacc actctatttt gtgcatcaga tgctaaagca tatgatacag aggtacataa 6420
   tgtttgggcc acacatgcct gtgtacccac agaccccaac ccacaagaag tagtattggt 6480
   aaatgtgaca gaaaatttta acatgtggaa aaatgacatg gtagaacaga tgcatgagga 6540
   tataatcagt ttatgggatc aaagcctaaa gccatgtgta aaattaaccc cactctgtgt 6600
   tagtttaaag tgcactgatt tgaagaatga tactaatacc aatagtagta gcgggagaat 6660
   gataatggag aaaggagaga taaaaaactg ctctttcaat atcagcacaa gcataagaga 6720
   taaggtgcag aaagaatatg cattctttta taaacttgat atagtaccaa tagataatac 6780
   cagctatagg ttgataagtt gtaacacctc agtcattaca caggcctgtc caaaggtatc 6840
   ctttgagcca attcccatac attattgtgc cccggctggt tttgcgattc taaaatgtaa 6900
   taataagacg ttcaatggaa caggaccatg tacaaatgtc agcacagtac aatgtacaca 6960
   tggaatcagg ccagtagtat caactcaact gctgttaaat ggcagtctag cagaagaaga 7020
   tgtagtaatt agatctgcca atttcacaga caatgctaaa accataatag tacagctgaa 7080
   cacatctgta gaaattaatt gtacaagacc caacaacaat acaagaaaaa gtatccgtat 7140
   ccagagggga ccagggagag catttgttac aataggaaaa ataggaaata tgagacaagc 7200
   acattgtaac attagtagag caaaatggaa tgccacttta aaacagatag ctagcaaatt 7260
   aagagaacaa tttggaaata ataaaacaat aatctttaag caatcctcag gaggggaccc 7320
   agaaattgta acgcacagtt ttaattgtgg aggggaattt ttctactgta attcaacaca 7380
   actgtttaat agtacttggt ttaatagtac ttggagtact gaagggtcaa ataacactga 7440
   aggaagtgac acaatcacac tcccatgcag aataaaacaa tttataaaca tgtggcagga 7500
   agtaggaaaa gcaatgtatg cccctcccat cagtggacaa attagatgtt catcaaatat 7560
   tactgggctg ctattaacaa gagatggtgg taataacaac aatgggtccg agatcttcag 7620
   acctggagga ggcgatatga gggacaattg gagaagtgaa ttatataaat ataaagtagt 7680
   aaaaattgaa ccattaggag tagcacccac caaggcaaag agaagagtgg tgcagagaga 7740
   aaaaagagca gtgggaatag gagctttgtt ccttgggttc ttgggagcag caggaagcac 7800
   tatgggctgc acgtcaatga cgctgacggt acaggccaga caattattgt ctgatatagt 7860
   gcagcagcag aacaatttgc tgagggctat tgaggcgcaa cagcatctgt tgcaactcac 7920
   agtctggggc atcaaacagc tccaggcaag aatcctggct gtggaaagat acctaaagga 7980
   tcaacagctc ctggggattt ggggttgctc tggaaaactc atttgcacca ctgctgtgcc 8040
   ttggaatgct agttggagta ataaatctct ggaacagatt tggaataaca tgacctggat 8100
   ggagtgggac agagaaatta acaattacac aagcttaata cactccttaa ttgaagaatc 8160
   gcaaaaccag caagaaaaga atgaacaaga attattggaa ttagataaat gggcaagttt 8220
   gtggaattgg tttaacataa caaattggct gtggtatata aaattattca taatgatagt 8280
   aggaggcttg gtaggtttaa gaatagtttt tgctgtactt tctatagtga atagagttag 8340
   gcagggatat tcaccattat cgtttcagac ccacctccca atcccgaggg gacccgacag 8400
   gcccgaagga atagaagaag aaggtggaga gagagacaga gacagatcca ttcgattagt 8460
   gaacggatcc ttagcactta tctgggacga tctgcggagc ctgtgcctct tcagctacca 8520
   ccgcttgaga gacttactct tgattgtaac gaggattgtg gaacttctgg gacgcagggg 8580
   gtgggaagcc ctcaaatatt ggtggaatct cctacagtat tggagtcagg aactaaagaa 8640
   tagtgctgtt aacttgctca atgccacagc catagcagta gctgagggga cagatagggt 8700
   tatagaagta ttacaagcag cttatagagc tattcgccac atacctagaa gaataagaca 8760
   gggcttggaa aggattttgc tataagatgg gtggcaagtg gtcaaaaagt agtgtgattg 8820
   gatggcctgc tgtaagggaa agaatgagac gagctgagcc agcagcagat ggggtgggag 8880
   cagtatctcg agacctagaa aaacatggag caatcacaag tagcaataca gcagctaaca 8940
   atgctgcttg tgcctggcta gaagcacaag aggaggaaga ggtgggtttt ccagtcacac 9000
   ctcaggtacc tttaagacca atgacttaca aggcagctgt agatcttagc cactttttaa 9060
   aagaaaaggg gggactggaa gggctaattc actcccaaag aagacaagat atccttgatc 9120
   tgtggatcta ccacacacaa ggctacttcc ctgattggca gaactacaca ccagggccag 9180
   gggtcagata tccactgacc tttggatggt gctacaagct agtaccagtt gagccagata 9240
   aggtagaaga ggccaataaa ggagagaaca ccagcttgtt acaccctgtg agcctgcatg 9300
   gaatggatga ccctgagaga gaagtgttag agtggaggtt tgacagccgc ctagcatttc 9360
   atcacgtggc ccgagagctg catccggagt acttcaagaa ctgctgacat cgagcttgct 9420
   acaagggact ttccgctggg gactttccag ggaggcgtgg cctgggcggg actggggagt 9480
   ggcgagccct cagatgctgc atataagcag ctgctttttg cctgtactgg gtctctctgg 9540
   ttagaccaga tctgagcctg ggagctctct ggctaactag ggaacccact gcttaagcct 9600
   caataaagct tgccttgagt gcttcaagta gtgtgtgccc gtctgttgtg tgactctggt 9660
   aactagagat ccctcagacc cttttagtca gtgtggaaaa tctctagca            9709
<210> 16
   <211> 500
   <212> PRT
   <213> Human immunodeficiency virus type 1
<220>
   <223> gag-Polyprotein
<400> 16
   Met Gly Ala Arg Ala Ser Val Leu Ser Gly Gly Glu Leu Asp Lys Trp
   1               5                   10                  15
   Glu Lys Ile Arg Leu Arg Pro Gly Gly Lys Lys Gln Tyr Lys Leu Lys
               20                  25                  30
   His Ile Val Trp Ala Ser Arg Glu Leu Glu Arg Phe Ala Val Asn Pro
           35                  40                  45
   Gly Leu Leu Glu Thr Ser Glu Gly Cys Arg Gln Ile Leu Gly Gln Leu
       50                  55                  60
   Gln Pro Ser Leu Gln Thr Gly Ser Glu Glu Leu Arg Ser Leu Tyr Asn
   65                  70                  75                  80
   Thr Ile Ala Val Leu Tyr Cys Val His Gln Arg Ile Asp Val Lys Asp
                   85                  90                  95
   Thr Lys Glu Ala Leu Asp Lys Ile Glu Glu Glu Gln Asn Lys Ser Lys
               100                 105                 110
   Lys Lys Ala Gln Gln Ala Ala Ala Asp Thr Gly Asn Asn Ser Gln Val
           115                 120                 125
   Ser Gln Asn Tyr Pro Ile Val Gln Asn Leu Gln Gly Gln Met Val His
       130                 135                 140
   Gln Ala Ile Ser Pro Arg Thr Leu Asn Ala Trp Val Lys Val Val Glu
   145                 150                 155                 160
   Glu Lys Ala Phe Ser Pro Glu Val Ile Pro Met Phe Ser Ala Leu Ser
                   165                 170                 175
   Glu Gly Ala Thr Pro Gln Asp Leu Asn Thr Met Leu Asn Thr Val Gly
   180 185 190
   Gly His Gln Ala Ala Met Gln Met Leu Lys Glu Thr Ile Asn Glu Glu
   195 200 205
   Ala Ala Glu Trp Asp Arg Leu His Pro Val His Ala Gly Pro Ile Ala
   210 215 220
   Pro Gly Gln Met Arg Glu Pro Arg Gly Ser Asp Ile Ala Gly Thr Thr
   225 230 235 240
   Ser Thr Leu Gln Glu Gln Ile Gly Trp Met Thr His Asn Pro Pro Ile
   245 250 255
   Pro Val Gly Glu Ile Tyr Lys Arg Trp Ile Ile Leu Gly Leu Asn Lys
   260 265 270
   Ile Val Arg Met Tyr Ser Pro Thr Ser Ile Leu Asp Ile Arg Gln Gly
   275 280 285
   Pro Lys Glu Pro Phe Arg Asp Tyr Val Asp Arg Phe Tyr Lys Thr Leu
   290 295 300
   Arg Ala Glu Gln Ala Ser Gln Glu Val Lys Asn Trp Met Thr Glu Thr
   305 310 315 320
   Leu Leu Val Gln Asn Ala Asn Pro Asp Cys Lys Thr Ile Leu Lys Ala
   325 330 335
   Leu Gly Pro Gly Ala Thr Leu Glu Glu Met Met Thr Ala Cys Gln Gly
   340 345 350
   Val Gly Gly Pro Gly His Lys Ala Arg Val Leu Ala Glu Ala Met Ser
   355 360 365
   Gln Val Thr Asn Pro Ala Thr Ile Met Ile Gln Lys Gly Asn Phe Arg
   370 375 380
   Asn Gln Arg Lys Thr Val Lys Cys Phe Asn Cys Gly Lys Glu Gly His
   385 390 395 400
   Ile Ala Lys Asn Cys Arg Ala Pro Arg Lys Lys Gly Cys Trp Lys Cys
   405 410 415
   Gly Lys Glu Gly His Gln Met Lys Asp Cys Thr Glu Arg Gln Ala Asn
   420 425 430
   Phe Leu Gly Lys Ile Trp Pro Ser His Lys Gly Arg Pro Gly Asn Phe
   435 440 445
   Leu Gln Ser Arg Pro Glu Pro Thr Ala Pro Pro Glu Glu Ser Phe Arg
   450 455 460
   Phe Gly Glu Glu Thr Thr Thr Pro Ser Gln Lys Gln Glu Pro Ile Asp
   465 470 475 480
   Lys Glu Leu Tyr Pro Leu Ala Ser Leu Arg Ser Leu Phe Gly Ser Asp
   485 490 495
   Pro Ser Ser Gln
   500
<210> 17
   <211> 1003
   <212> PRT
   <213> Human immunodeficiency virus type 1
<220>
   <223> pol-Polyprotein
<400> 17
   Phe Phe Arg Glu Asp Leu Ala Phe Pro Gln Gly Lys Ala Arg Glu Phe
    1               5                  10                  15
   Ser Ser Glu Gln Thr Arg Ala Asn Ser Pro Thr Arg Arg Glu Leu Gln
               20                  25                  30
   Val Trp Gly Arg Asp Asn Asn Ser Leu Ser Glu Ala Gly Ala Asp Arg
           35                  40                  45
   Gln Gly Thr Val Ser Phe Ser Phe Pro Gln Ile Thr Leu Trp Gln Arg
       50                  55                  60
   Pro Leu Val Thr Ile Lys Ile Gly Gly Gln Leu Lys Glu Ala Leu Leu
   65                  70                  75                  80
   Asp Thr Gly Ala Asp Asp Thr Val Leu Glu Glu Met Asn Leu Pro Gly
                   85                  90                  95
   Arg Trp Lys Pro Lys Met Ile Gly Gly Ile Gly Gly Phe Ile Lys Val
               100                 105                 110
   Gly Gln Tyr Asp Gln Ile Leu Ile Glu Ile Cys Gly His Lys Ala Ile
           115                 120                 125
   Gly Thr Val Leu Val Gly Pro Thr Pro Val Asn Ile Ile Gly Arg Asn
       130                 135                 140
   Leu Leu Thr Gln Ile Gly Cys Thr Leu Asn Phe Pro Ile Ser Pro Ile
   145                 150                 155                 160
   Glu Thr Val Pro Val Lys Leu Lys Pro Gly Met Asp Gly Pro Lys Val
                   165                 170                 175
   Lys Gln Trp Pro Leu Thr Glu Glu Lys Ile Lys Ala Leu Val Glu Ile
               180                 185                 190
   Cys Thr Glu Met Glu Lys Glu Gly Lys Ile Ser Lys Ile Gly Pro Glu
           195                 200                 205
   Asn Pro Tyr Asn Thr Pro Val Phe Ala Ile Lys Lys Lys Asp Ser Thr
       210                 215                 220
   Lys Trp Arg Lys Leu Val Asp Phe Arg Glu Leu Asn Lys Arg Thr Gln
   225                 230                 235                 240
   Asp Phe Trp Glu Val Gln Leu Gly Ile Pro His Pro Ala Gly Leu Lys
                   245                 250                 255
   Gln Lys Lys Ser Val Thr Val Leu Asp Val Gly Asp Ala Tyr Phe Ser
               260                 265                 270
   Val Pro Leu Asp Lys Asp Phe Arg Lys Tyr Thr Ala Phe Thr Ile Pro
           275                 280                 285
   Ser Ile Asn Asn Glu Thr Pro Gly Ile Arg Tyr Gln Tyr Asn Val Leu
       290                 295                 300
   Pro Gln Gly Trp Lys Gly Ser Pro Ala Ile Phe Gln Cys Ser Met Thr
   305                 310                 315                 320
   Lys Ile Leu Glu Pro Phe Arg Lys Gln Asn Pro Asp Ile Val Ile Tyr
                   325                 330                 335
   Gln Tyr Met Asp Asp Leu Tyr Val Gly Ser Asp Leu Glu Ile Gly Gln
               340                 345                 350
   His Arg Thr Lys Ile Glu Glu Leu Arg Gln His Leu Leu Arg Trp Gly
           355                 360                 365
   Phe Thr Thr Pro Asp Lys Lys His Gln Lys Glu Pro Pro Phe Leu Trp
       370                 375                 380
   Met Gly Tyr Glu Leu His Pro Asp Lys Trp Thr Val Gln Pro Ile Val
   385                 390                 395                 400
   Leu Pro Glu Lys Asp Ser Trp Thr Val Asn Asp Ile Gln Lys Leu Val
                   405                 410                 415
   Gly Lys Leu Asn Trp Ala Ser Gln Ile Tyr Ala Gly Ile Lys Val Arg
               420                 425                 430
   Gln Leu Cys Lys Leu Leu Arg Gly Thr Lys Ala Leu Thr Glu Val Val
           435                 440                 445
   Pro Leu Thr Glu Glu Ala Glu Leu Glu Leu Ala Glu Asn Arg Glu Ile
       450                 455                 460
   Leu Lys Glu Pro Val His Gly Val Tyr Tyr Asp Pro Ser Lys Asp Leu
   465                 470                 475                 480
   Ile Ala Glu Ile Gln Lys Gln Gly Gln Gly Gln Trp Thr Tyr Gln Ile
                   485                 490                 495
   Tyr Gln Glu Pro Phe Lys Asn Leu Lys Thr Gly Lys Tyr Ala Arg Met
               500                 505                 510
   Lys Gly Ala His Thr Asn Asp Val Lys Gln Leu Thr Glu Ala Val Gln
           515                 520                 525
   Lys Ile Ala Thr Glu Ser Ile Val Ile Trp Gly Lys Thr Pro Lys Phe
       530                 535                 540
   Lys Leu Pro Ile Gln Lys Glu Thr Trp Glu Ala Trp Trp Thr Glu Tyr
   545                 550                 555                 560
   Trp Gln Ala Thr Trp Ile Pro Glu Trp Glu Phe Val Asn Thr Pro Pro
                   565                 570                 575
   Leu Val Lys Leu Trp Tyr Gln Leu Glu Lys Glu Pro Ile Ile Gly Ala
               580                 585                 590
   Glu Thr Phe Tyr Val Asp Gly Ala Ala Asn Arg Glu Thr Lys Leu Gly
   595 600 605
   Lys Ala Gly Tyr Val Thr Asp Arg Gly Arg Gln Lys Val Val Pro Leu
   610 615 620
   Thr Asp Thr Thr Asn Gln Lys Thr Glu Leu Gln Ala Ile His Leu Ala
   625 630 635 640
   Leu Gln Asp Ser Gly Leu Glu Val Asn Ile Val Thr Asp Ser Gln Tyr
   645 650 655
   Ala Leu Gly Ile Ile Gln Ala Gln Pro Asp Lys Ser Glu Ser Glu Leu
   660 665 670
   Val Ser Gln Ile Ile Glu Gln Leu Ile Lys Lys Glu Lys Val Tyr Leu
   675 680 685
   Ala Trp Val Pro Ala His Lys Gly Ile Gly Gly Asn Glu Gln Val Asp
   690 695 700
   Gly Leu Val Ser Ala Gly Ile Arg Lys Val Leu Phe Leu Asp Gly Ile
   705 710 715 720
   Asp Lys Ala Gln Glu Glu His Glu Lys Tyr His Ser Asn Trp Arg Ala
   725 730 735
   Met Ala Ser Asp Phe Asn Leu Pro Pro Val Val Ala Lys Glu Ile Val
   740 745 750
   Ala Ser Cys Asp Lys Cys Gln Leu Lys Gly Glu Ala Met His Gly Gln
   755 760 765
   Val Asp Cys Ser Pro Gly Ile Trp Gln Leu Asp Cys Thr His Leu Glu
   770 775 780
   Gly Lys Val Ile Leu Val Ala Val His Val Ala Ser Gly Tyr Ile Glu
   785 790 795 800
   Ala Glu Val Ile Pro Ala Glu Thr Gly Gln Glu Thr Ala Tyr Phe Leu
   805 810 815
   Leu Lys Leu Ala Gly Arg Trp Pro Val Lys Thr Val His Thr Asp Asn
   820 825 830
   Gly Ser Asn Phe Thr Ser Thr Thr Val Lys Ala Ala Cys Trp Trp Ala
   835 840 845
   Gly Ile Lys Gln Glu Phe Gly Ile Pro Tyr Asn Pro Gln Ser Gln Gly
   850 855 860
   Val Ile Glu Ser Met Asn Lys Glu Leu Lys Lys Ile Ile Gly Gln Val
   865 870 875 880
   Arg Asp Gln Ala Glu His Leu Lys Thr Ala Val Gln Met Ala Val Phe
   885 890 895
   Ile His Asn Phe Lys Arg Lys Gly Gly Ile Gly Gly Tyr Ser Ala Gly
   900 905 910
   Glu Arg Ile Val Asp Ile Ile Ala Thr Asp Ile Gln Thr Lys Glu Leu
   915 920 925
   Gln Lys Gln Ile Thr Lys Ile Gln Asn Phe Arg Val Tyr Tyr Arg Asp
   930 935 940
   Ser Arg Asp Pro Val Trp Lys Gly Pro Ala Lys Leu Leu Trp Lys Gly
   945 950 955 960
   Glu Gly Ala Val Val Ile Gln Asp Asn Ser Asp Ile Lys Val Val Pro
   965 970 975
   Arg Arg Lys Ala Lys Ile Ile Arg Asp Tyr Gly Lys Gln Met Ala Gly
   980 985 990
   Asp Asp Cys Val Ala Ser Arg Gln Asp Glu Asp
   995 1000
<210> 18
   <211> 192
   <212> PRT
   <213> Human immunodeficiency virus type 1
<220>
   <223> viv-Protein
<210> 19
   <211> 96
   <212> PRT
   <213> Human immunodeficiency virus type 1
<220>
   <223> vpr-Protein
<400> 19
   Met Glu Gln Ala Pro Glu Asp Gln Gly Pro Gln Arg Glu Pro Tyr Asn
    1               5                  10                  15
   Glu Trp Thr Leu Glu Leu Leu Glu Glu Leu Lys Ser Glu Ala Val Arg
               20                  25                  30
   His Phe Pro Arg Ile Trp Leu His Asn Leu Gly Gln His Ile Tyr Glu
           35                  40                  45
   Thr Tyr Gly Asp Thr Trp Ala Gly Val Glu Ala Ile Ile Arg Ile Leu
       50                  55                  60
   Gln Gln Leu Leu Phe Ile His Phe Arg Ile Gly Cys Arg His Ser Arg
   65                  70                  75                  80
   Ile Gly Val Thr Arg Gln Arg Arg Ala Arg Asn Gly Ala Ser Arg Ser
                   85                  90                  95
<210> 20
   <211> 86
   <212> PRT
   <213> Human immunodeficiency virus type 1
<220>
   <223> tat-Protein
<400> 20
<210> 21
   <211> 116
   <212> PRT
   <213> Human immunodeficiency virus type 1
<220>
   <223> rev-Protein
<400> 21
<210> 22
   <211> 81
   <212> PRT
   <213> Human immunodeficiency virus type 1
<220>
   <223> vpu-Protein
<400> 22
   Met Gln Pro Ile Ile Val Ala Ile Val Ala Leu Val Val Ala Ile Ile
    1               5                  10                  15
   Ile Ala Ile Val Val Trp Ser Ile Val Ile Ile Glu Tyr Arg Lys Ile
               20                  25                  30
   Leu Arg Gln Arg Lys Ile Asp Arg Leu Ile Asp Arg Leu Ile Glu Arg
           35                  40                  45
   Ala Glu Asp Ser Gly Asn Glu Ser Glu Gly Glu Val Ser Ala Leu Val
       50                  55                  60
   Glu Met Gly Val Glu Met Gly His His Ala Pro Trp Asp Ile Asp Asp
   65                  70                  75                  80
   Leu
<210> 23
   <211> 854
   <212> PRT
   <213> Human immunodeficiency virus type 1
<220>
   <223> Huell-Protein
<400> 23
   Met Arg Val Lys Glu Lys Tyr Gln His Leu Trp Arg Trp Gly Trp Lys
   1 5 10 15
   Trp Gly Thr Met Leu Leu Gly Ile Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Lys Asn Asp Thr Asn Thr Asn Ser Ser Ser
   130 135 140
   Gly Arg Met Ile Met Glu Lys Gly Glu Ile Lys Asn Cys Ser Phe Asn
   145 150 155 160
   Ile Ser Thr Ser Ile Arg Asp Lys Val Gln Lys Glu Tyr Ala Phe Phe
   165 170 175
   Tyr Lys Leu Asp Ile Val Pro Ile Asp Asn Thr Ser Tyr Arg Leu Ile
   180 185 190
   Ser Cys Asn Thr Ser Val Ile Thr Gln Ala Cys Pro Lys Val Ser Phe
   195 200 205
   Glu Pro Ile Pro Ile His Tyr Cys Ala Pro Ala Gly Phe Ala Ile Leu
   210 215 220
   Lys Cys Asn Asn Lys Thr Phe Asn Gly Thr Gly Pro Cys Thr Asn Val
   225 230 235 240
   Ser Thr Val Gln Cys Thr His Gly Ile Arg Pro Val Val Ser Thr Gln
   245 250 255
   Leu Leu Leu Asn Gly Ser Leu Ala Glu Glu Asp Val Val Ile Arg Ser
   260 265 270
   Ala Asn Phe Thr Asp Asn Ala Lys Thr Ile Ile Val Gln Leu Asn Thr
   275 280 285
   Ser Val Glu Ile Asn Cys Thr Arg Pro Asn Asn Asn Thr Arg Lys Ser
   290 295 300
   Ile Arg Ile Gln Arg Gly Pro Gly Arg Ala Phe Val Thr Ile Gly Lys
   305 310 315 320
   Ile Gly Asn Met Arg Gln Ala His Cys Asn Ile Ser Arg Ala Lys Trp
   325 330 335
   Asn Ala Thr Leu Lys Gln Ile Ala Ser Lys Leu Arg Glu Gln Phe Gly
   340 345 350
   Asn Asn Lys Thr Ile Ile Phe Lys Gln Ser Ser Gly Gly Asp Pro Glu
   355 360 365
   Ile Val Thr His Ser Phe Asn Cys Gly Gly Glu Phe Phe Tyr Cys Asn
   370 375 380
   Ser Thr Gln Leu Phe Asn Ser Thr Trp Phe Asn Ser Thr Trp Ser Thr
   385 390 395 400
   Glu Gly Ser Asn Asn Thr Glu Gly Ser Asp Thr Ile Thr Leu Pro Cys
   405 410 415
   Arg Ile Lys Gln Phe Ile Asn Met Trp Gln Glu Val Gly Lys Ala Met
   420 425 430
   Tyr Ala Pro Pro Ile Ser Gly Gln Ile Arg Cys Ser Ser Asn Ile Thr
           435                 440                 445
   Gly Leu Leu Leu Thr Arg Asp Gly Gly Asn Asn Asn Asn Gly Ser Glu
       450                 455                 460
   Ile Phe Arg Pro Gly Gly Gly Asp Met Arg Asp Asn Trp Arg Ser Glu
   465                 470                 475                 480
   Leu Tyr Lys Tyr Lys Val Val Lys Ile Glu Pro Leu Gly Val Ala Pro
                   485                 490                 495
   Thr Lys Ala Lys Arg Arg Val Val Gln Arg Glu Lys Arg Ala Val Gly
               500                 505                 510
   Ile Gly Ala Leu Phe Leu Gly Phe Leu Gly Ala Ala Gly Ser Thr Met
           515                 520                 525
   Gly Cys Thr Ser Met Thr Leu Thr Val Gln Ala Arg Gln Leu Leu Ser
       530                 535                 540
   Asp Ile Val Gln Gln Gln Asn Asn Leu Leu Arg Ala Ile Glu Ala Gln
   545                 550                 555                 560
   Gln His Leu Leu Gln Leu Thr Val Trp Gly Ile Lys Gln Leu Gln Ala
                   565                 570                 575
   Arg Ile Leu Ala Val Glu Arg Tyr Leu Lys Asp Gln Gln Leu Leu Gly
               580                 585                 590
   Ile Trp Gly Cys Ser Gly Lys Leu Ile Cys Thr Thr Ala Val Pro Trp
           595                 600                 605
   Asn Ala Ser Trp Ser Asn Lys Ser Leu Glu Gln Ile Trp Asn Asn Met
       610                 615                 620
   Thr Trp Met Glu Trp Asp Arg Glu Ile Asn Asn Tyr Thr Ser Leu Ile
   625                 630                 635                 640
   His Ser Leu Ile Glu Glu Ser Gln Asn Gln Gln Glu Lys Asn Glu Gln
                   645                 650                 655
   Glu Leu Leu Glu Leu Asp Lys Trp Ala Ser Leu Trp Asn Trp Phe Asn
               660                 665                 670
   Ile Thr Asn Trp Leu Trp Tyr Ile Lys Leu Phe Ile Met Ile Val Gly
           675                 680                 685
   Gly Leu Val Gly Leu Arg Ile Val Phe Ala Val Leu Ser Ile Val Asn
       690                 695                 700
   Arg Val Arg Gln Gly Tyr Ser Pro Leu Ser Phe Gln Thr His Leu Pro
   705                 710                 715                 720
   Ile Pro Arg Gly Pro Asp Arg Pro Glu Gly Ile Glu Glu Glu Gly Gly
                   725                 730                 735
   Glu Arg Asp Arg Asp Arg Ser Ile Arg Leu Val Asn Gly Ser Leu Ala
               740                 745                 750
   Leu Ile Trp Asp Asp Leu Arg Ser Leu Cys Leu Phe Ser Tyr His Arg
           755                 760                 765
   Leu Arg Asp Leu Leu Leu Ile Val Thr Arg Ile Val Glu Leu Leu Gly
       770                 775                 780
   Arg Arg Gly Trp Glu Ala Leu Lys Tyr Trp Trp Asn Leu Leu Gln Tyr
   785                 790                 795                 800
   Trp Ser Gln Glu Leu Lys Asn Ser Ala Val Asn Leu Leu Asn Ala Thr
                   805                 810                 815
   Ala Ile Ala Val Ala Glu Gly Thr Asp Arg Val Ile Glu Val Leu Gln
               820                 825                 830
   Ala Ala Tyr Arg Ala Ile Arg His Ile Pro Arg Arg Ile Arg Gln Gly
           835                 840                 845
   Leu Glu Arg Ile Leu Leu
       850
<210> 24
   <211> 206
   <212> PRT
   <213> Human immunodeficiency virus type 1
<220>
   <223> nef-Protein
<400> 24
   Met Gly Gly Lys Trp Ser Lys Ser Ser Val Ile Gly Trp Pro Ala Val
   1               5                   10                  15
   Arg Glu Arg Met Arg Arg Ala Glu Pro Ala Ala Asp Gly Val Gly Ala
               20                  25                  30
   Val Ser Arg Asp Leu Glu Lys His Gly Ala Ile Thr Ser Ser Asn Thr
           35                  40                  45
   Ala Ala Asn Asn Ala Ala Cys Ala Trp Leu Glu Ala Gln Glu Glu Glu
       50                  55                  60
   Glu Val Gly Phe Pro Val Thr Pro Gln Val Pro Leu Arg Pro Met Thr
   65                  70                  75                  80
   Tyr Lys Ala Ala Val Asp Leu Ser His Phe Leu Lys Glu Lys Gly Gly
                   85                  90                  95
   Leu Glu Gly Leu Ile His Ser Gln Arg Arg Gln Asp Ile Leu Asp Leu
               100                 105                 110
   Trp Ile Tyr His Thr Gln Gly Tyr Phe Pro Asp Trp Gln Asn Tyr Thr
           115                 120                 125
   Pro Gly Pro Gly Val Arg Tyr Pro Leu Thr Phe Gly Trp Cys Tyr Lys
       130                 135                 140
   Leu Val Pro Val Glu Pro Asp Lys Val Glu Glu Ala Asn Lys Gly Glu
   145                 150                 155                 160
   Asn Thr Ser Leu Leu His Pro Val Ser Leu His Gly Met Asp Asp Pro
                   165                 170                 175
   Glu Arg Glu Val Leu Glu Trp Arg Phe Asp Ser Arg Leu Ala Phe His
               180                 185                 190
   His Val Ala Arg Glu Leu His Pro Glu Tyr Phe Lys Asn Cys
           195                 200                 205
<210> 25
   <211> 1497
   <212> DNA
   <213> Kuenstliche Sequenz
<220>
   <223> Beschreibung der kuenstlichen Sequenz: LCMV-Variante WE-HPI
<220>
   <221> CDS
   <222> ()..(1497)
   <223> LCMV GP-Variante (Open reading frame)
<220>
<223> Mutationen gegenueber <400> 1 (2) an Positionen 281 (94), 329 (110), 385 (129), 397 (133), 463 (155), 521 (174), 543 (181), 631 (211), 793 (265), 1039 (347), 1363 (455) und 1370 (457).
<400> 25
   atg ggt cag att gtg aca atg ttt gag gct ttg cct cac atc att gat    48
   Met Gly Gln Ile Val Thr Met Phe Glu Ala Leu Pro His Ile Ile Asp
   1               5                   10                  15
   gag gtc atc aac att gtc att att gtg ctc att ata atc acg agc atc    96
   Glu Val Ile Asn Ile Val Ile Ile Val Leu Ile Ile Ile Thr Ser Ile
               20                  25                  30
   aaa gct gtg tac aat ttc gcc acc tgt ggg ata tta gca ctg gtc agc   144
   Lys Ala Val Tyr Asn Phe Ala Thr Cys Gly Ile Leu Ala Leu Val Ser
           35                  40                  45
   ttc ctt ttt ttg gct ggt agg tcc tgt ggc atg tac ggc ctt aat ggt   192
   Phe Leu Phe Leu Ala Gly Arg Ser Cys Gly Met Tyr Gly Leu Asn Gly
       50                  55                  60
   ccc gac atc tat aaa ggg gtt tac cag ttc aaa tca gtg gag ttt gat   240
   Pro Asp Ile Tyr Lys Gly Val Tyr Gln Phe Lys Ser Val Glu Phe Asp
   65                  70                  75                  80
   atg tct cac tta aat ctg acg atg ccc aat gcg tgc tca gcc aac aac   288
   Met Ser His Leu Asn Leu Thr Met Pro Asn Ala Cys Ser Ala Asn Asn
                   85                  90                  95
   tct cat cac tac atc agt atg gga agc tct gga ctg gag cta act ttc   336
   Ser His His Tyr Ile Ser Met Gly Ser Ser Gly Leu Glu Leu Thr Phe
               100                 105                 110
   act aac gac tcc atc ctt aat cac aat ttt tgc aac tta acc tcc gct   384
   Thr Asn Asp Ser Ile Leu Asn His Asn Phe Cys Asn Leu Thr Ser Ala
           115                 120                 125
   ttc aac aaa aag act ttt gac cat aca ctc atg agt ata gtc tcg agt   432
   Phe Asn Lys Lys Thr Phe Asp His Thr Leu Met Ser Ile Val Ser Ser
       130                 135                 140
   ctg cac ctc agt att aga ggg aat tcc aac cac aaa gca gtg tct tgt   480
   Leu His Leu Ser Ile Arg Gly Asn Ser Asn His Lys Ala Val Ser Cys
   145                 150                 155                 160
   gat ttt aac aat ggc atc acc att caa tac aac ttg tca ttt tcg gac   528
   Asp Phe Asn Asn Gly Ile Thr Ile Gln Tyr Asn Leu Ser Phe Ser Asp
                   165                 170                 175
   cca cag agc gct ata agc cag tgt agg act ttc aga ggt aga gtc ttg   576
   Pro Gln Ser Ala Ile Ser Gln Cys Arg Thr Phe Arg Gly Arg Val Leu
               180                 185                 190
   gac atg ttt aga act gcc ttt gga gga aaa tac atg aga agt ggc tgg   624
   Asp Met Phe Arg Thr Ala Phe Gly Gly Lys Tyr Met Arg Ser Gly Trp
           195                 200                 205
   ggc tgg gca ggt tca gat ggc aag acc act tgg tgc agc caa aca agc   672
   Gly Trp Ala Gly Ser Asp Gly Lys Thr Thr Trp Cys Ser Gln Thr Ser
       210                 215                 220
   tat cag tac cta atc ata caa aac agg act tgg gaa aac cac tgt aga   720
   Tyr Gln Tyr Leu Ile Ile Gln Asn Arg Thr Trp Glu Asn His Cys Arg
   225                 230                 235                 240
   tat gca ggc cct ttt ggg atg tct aga atc ctc ttt gct cag gaa aag   768
   Tyr Ala Gly Pro Phe Gly Met Ser Arg Ile Leu Phe Ala Gln Glu Lys
                   245                 250                 255
   aca aag ttt ctc act agg aga ctt gca ggc aca ttc acc tgg acc ctg   816
   Thr Lys Phe Leu Thr Arg Arg Leu Ala Gly Thr Phe Thr Trp Thr Leu
               260                 265                 270
   tca gac tcc tca gga gta gaa aat cca ggt ggt tat tgc ctg acc aaa    864
   Ser Asp Ser Ser Gly Val Glu Asn Pro Gly Gly Tyr Cys Leu Thr Lys
           275                 280                 285
   tgg atg atc ctt gct gca gag ctc aaa tgt ttt ggg aat aca gct gtt    912
   Trp Met Ile Leu Ala Ala Glu Leu Lys Cys Phe Gly Asn Thr Ala Val
       290                 295                 300
   gca aaa tgt aat gtc aat cat gat gaa gag ttc tgt gac atg cta cga    960
   Ala Lys Cys Asn Val Asn His Asp Glu Glu Phe Cys Asp Met Leu Arg
   305                 310                 315                 320
   cta att gat tac aac aag gcc gcc ctg agt aag ttc aag caa gat gta    1008
   Leu Ile Asp Tyr Asn Lys Ala Ala Leu Ser Lys Phe Lys Gln Asp Val
                   325                 330                 335
   gag tct gcc ttg cat gta ttc aaa aca aca gta aat tct ctg att tcc    1056
   Glu Ser Ala Leu His Val Phe Lys Thr Thr Val Asn Ser Leu Ile Ser
               340                 345                 350
   gat cag ctg ttg atg agg aat cat cta aga gat cta atg ggg gta cca    1104
   Asp Gln Leu Leu Met Arg Asn His Leu Arg Asp Leu Met Gly Val Pro
           355                 360                 365
   tac tgt aat tac tca aag ttc tgg tat ctg gaa cat gct aag act ggt    1152
   Tyr Cys Asn Tyr Ser Lys Phe Trp Tyr Leu Glu His Ala Lys Thr Gly
       370                 375                 380
   gag act agt gta ccc aag tgc tgg ctt gtc act aat ggc tcc tac ttg    1200
   Glu Thr Ser Val Pro Lys Cys Trp Leu Val Thr Asn Gly Ser Tyr Leu
   385                 390                 395                 400
   aat gag acc cac ttt agt gat caa atc gaa caa gaa gca gat aac atg    1248
   Asn Glu Thr His Phe Ser Asp Gln Ile Glu Gln Glu Ala Asp Asn Met
                   405                 410                 415
   atc aca gag atg ttg agg aag gac tac ata aaa aga caa ggg agt act    1296
   Ile Thr Glu Met Leu Arg Lys Asp Tyr Ile Lys Arg Gln Gly Ser Thr
               420                 425                 430
   cct tta gcc tta atg gat ctt ttg atg ttt tca aca tca gca tat cta    1344
   Pro Leu Ala Leu Met Asp Leu Leu Met Phe Ser Thr Ser Ala Tyr Leu
           435                 440                 445
   atc agc atc ttt ctg cat ctt gtg aag ata cca aca cat aga cac ata    1392
   Ile Ser Ile Phe Leu His Leu Val Lys Ile Pro Thr His Arg His Ile
       450                 455                 460
   aag ggc ggt tca tgt cca aag cca cac cgc ttg acc aac aag ggg atc    1440
   Lys Gly Gly Ser Cys Pro Lys Pro His Arg Leu Thr Asn Lys Gly Ile
   465                 470                 475                 480
   tgt agt tgt ggt gca ttc aag gtg cct ggt gta aaa act atc tgg aaa    1488
   Cys Ser Cys Gly Ala Phe Lys Val Pro Gly Val Lys Thr Ile Trp Lys
                   485                 490                 495
   aga cgc tga                                                        1497
   Arg Arg
<210> 26
   <211> 498
   <212> PRT
   <213> Kuenstliche Sequenz
<400> 26
   Met Gly Gln Ile Val Thr Met Phe Glu Ala Leu Pro His Ile Ile Asp
   1               5                   10                  15
   Glu Val Ile Asn Ile Val Ile Ile Val Leu Ile Ile Ile Thr Ser Ile
               20                  25                  30
   Lys Ala Val Tyr Asn Phe Ala Thr Cys Gly Ile Leu Ala Leu Val Ser
           35                  40                  45
   Phe Leu Phe Leu Ala Gly Arg Ser Cys Gly Met Tyr Gly Leu Asn Gly
       50                  55                  60
   Pro Asp Ile Tyr Lys Gly Val Tyr Gln Phe Lys Ser Val Glu Phe Asp
   65                  70                  75                  80
   Met Ser His Leu Asn Leu Thr Met Pro Asn Ala Cys Ser Ala Asn Asn
                   85                  90                  95
   Ser His His Tyr Ile Ser Met Gly Ser Ser Gly Leu Glu Leu Thr Phe
               100                 105                 110
   Thr Asn Asp Ser Ile Leu Asn His Asn Phe Cys Asn Leu Thr Ser Ala
           115                 120                 125
   Phe Asn Lys Lys Thr Phe Asp His Thr Leu Met Ser Ile Val Ser Ser
       130                 135                 140
   Leu His Leu Ser Ile Arg Gly Asn Ser Asn His Lys Ala Val Ser Cys
   145                 150                 155                 160
   Asp Phe Asn Asn Gly Ile Thr Ile Gln Tyr Asn Leu Ser Phe Ser Asp
                   165                 170                 175
   Pro Gln Ser Ala Ile Ser Gln Cys Arg Thr Phe Arg Gly Arg Val Leu
               180                 185                 190
   Asp Met Phe Arg Thr Ala Phe Gly Gly Lys Tyr Met Arg Ser Gly Trp
           195                 200                 205
   Gly Trp Ala Gly Ser Asp Gly Lys Thr Thr Trp Cys Ser Gln Thr Ser
       210                 215                 220
   Tyr Gln Tyr Leu Ile Ile Gln Asn Arg Thr Trp Glu Asn His Cys Arg
   225                 230                 235                 240
   Tyr Ala Gly Pro Phe Gly Met Ser Arg Ile Leu Phe Ala Gln Glu Lys
                   245                 250                 255
   Thr Lys Phe Leu Thr Arg Arg Leu Ala Gly Thr Phe Thr Trp Thr Leu
               260                 265                 270
   Ser Asp Ser Ser Gly Val Glu Asn Pro Gly Gly Tyr Cys Leu Thr Lys
           275                 280                 285
   Trp Met Ile Leu Ala Ala Glu Leu Lys Cys Phe Gly Asn Thr Ala Val
       290                 295                 300
   Ala Lys Cys Asn Val Asn His Asp Glu Glu Phe Cys Asp Met Leu Arg
   305                 310                 315                 320
   Leu Ile Asp Tyr Asn Lys Ala Ala Leu Ser Lys Phe Lys Gln Asp Val
                   325                 330                 335
   Glu Ser Ala Leu His Val Phe Lys Thr Thr Val Asn Ser Leu Ile Ser
               340                 345                 350
   Asp Gln Leu Leu Met Arg Asn His Leu Arg Asp Leu Met Gly Val Pro
           355                 360                 365
   Tyr Cys Asn Tyr Ser Lys Phe Trp Tyr Leu Glu His Ala Lys Thr Gly
       370                 375                 380
   Glu Thr Ser Val Pro Lys Cys Trp Leu Val Thr Asn Gly Ser Tyr Leu
   385                 390                 395                 400
   Asn Glu Thr His Phe Ser Asp Gln Ile Glu Gln Glu Ala Asp Asn Met
                   405                 410                 415
   Ile Thr Glu Met Leu Arg Lys Asp Tyr Ile Lys Arg Gln Gly Ser Thr
               420                 425                 430
   Pro Leu Ala Leu Met Asp Leu Leu Met Phe Ser Thr Ser Ala Tyr Leu
           435                 440                 445
   Ile Ser Ile Phe Leu His Leu Val Lys Ile Pro Thr His Arg His Ile
       450                 455                 460
   Lys Gly Gly Ser Cys Pro Lys Pro His Arg Leu Thr Asn Lys Gly Ile
   465                 470                 475                 480
   Cys Ser Cys Gly Ala Phe Lys Val Pro Gly Val Lys Thr Ile Trp Lys
                   485                 490                 495
   Arg Arg